# EUROPEAN PATENT SPECIFICATION

(11) **EP 2 640 727 B1**
(45) Date of publication and mention of the grant of the patent: **13.05.2015**
(21) Application number: 11793586.6
(22) Date of filing: 16.11.2011
(51) Int. Cl.: C07D 491/12, A61K 31/535, A61K 39/00

(54) **ALANINYL MAYTANSINOL ANTIBODY CONJUGATES**
ALANINYLMAYTANSINOL-ANTIKÖRPERKONJUGATE
CONJUGUÉS D'ANTICORPS ALANINYL-MAYTANSINOL

(30) Priority: 17.11.2010 US 414535 P
(43) Date of publication of application: 25.09.2013
(73) Proprietor: Genentech, Inc., South San Francisco, CA 94080 (US)
(72) Inventor: FLYGARE, John, South San Francisco, CA 94080 (US); JUNUTULA, Jagath Reddy, South San Francisco, CA 94080 (US); PILLOW, Thomas, South San Francisco, CA 94080 (US)
(74) Representative: Kiddle, Simon John
(86) International application number: PCT/US2011/061031
(87) International publication number: WO 2012/074757

(56) References cited:
- WO-A1-2010/126551
- WO-A2-2005/037992
- WO-A2-2005/117986
- US-A1- 2010 158 928

## Description

### RELATED APPLICATIONS

This application claims the benefit under U.S. Provisional Application Serial No. 61/414,535 filed on 17 November 2010.

### FIELD OF THE INVENTION

The invention relates generally to antibodies conjugated to maytansinoid drug moieties to form antibody-drug conjugates with therapeutic or diagnostic applications. The antibodies may be engineered with free cysteine amino acids, reactive for conjugation with alaninyl maytansinoid drug-linker reagents. The invention also relates to methods of using the alaninyl maytansinoid antibody-drug conjugate compounds for *in vitro*, *in situ*, and *in vivo* diagnosis or treatment of mammalian cells, or associated pathological conditions.

### BACKGROUND OF THE INVENTION

Antibody drug conjugates (ADC) are targeted chemotherapeutic molecules combining the ideal properties of both antibodies and cytotoxic drugs by targeting potent cytotoxic drugs to the antigen-expressing tumor cells, internalization, and release of drug, thereby enhancing their anti-tumor activity. The successful ADC development for a given target antigen depends on optimization of antibody selection, linker design and stability, cytotoxic drug potency and mode of drug and linker conjugation to the antibody. Linker properties of pH and redox sensitivities and protease susceptibility influence internalization and release of the cytotoxic drug moiety. The intracellular cleavage of disulfide containing linkers of an ADC is limited by the oxidizing potential of endosomes and lysosomes and are probably not released by reductive cleavage within the endocytic pathway (Austin et al (2005) Proc. Natl. Acad. Sci. USA 102(50):17987-17992). Reductive cleavage may occur at the cell membrane and impart a bystander killing effect of tumor and susceptible normal cells by free drug. Inappropriate release of drug likely contributes to toxicity. Once internalized, ADC efficacy is dependent on proteolytic digestion for drug activity. Linker stability plays an important role in both the efficacy and toxicity of ADC (Alley et al (2008) Bioconjugate Chem. 19:759-765). Stable linkers such as mcc are more efficacious and safer than unstable, disulfide linkers, widening the therapeutic window.

Antibodies with cysteine substitutions (ThioMabs and ThioFabs) can be engineered at sites where the cysteines are available for conjugation but do not perturb immunoglobulin folding and assembly or alter antigen binding and effector functions (Junutula, et al., 2008b Nature Biotech., 26(8):925-932; Dornan et al (2009) Blood 114(13):2721-2729; US 7521541; US 7723485; WO2009/052249). These ThioMabs can then be conjugated to cytotoxic drugs through the engineered cysteine thiol groups to obtain ThioMab drug conjugates (TDC) with uniform stoichiometry (about 2 drugs per antibody). Studies with multiple antibodies against different antigens have shown that TDC are as efficacious as conventional ADC in xenograft models and are tolerated at higher doses in relevant preclinical models. ThioMab drug conjugates have been engineered with drug attachment at different parts of the antibody (light chain-Fab, heavy chain-Fab and heavy chain-Fc). The *in vitro* and *in vivo* stability, efficacy and PK properties of TDC provide a unique advantage over conventional ADC due to their homogeneity and site-specific conjugation to cytotoxic drugs.

Antibody-drug conjugates (ADCs) composed of the maytansinoid, DM1, linked to trastuzumab show potent anti-tumor activity in HER2-overexpressing trastuzumab-sensitive and -resistant tumor cell lines and xenograft models of human cancer. Trastuzumab-mcc-DM1 (T-DM1) is currently undergoing evaluation in phase II clinical trials in patients whose disease is refractory to HER2-directed therapies (Beeram et al (2007) "A phase I study of trastuzumab-mcc-DM1 (T-DM1), a first-in-class HER2 antibody-drug conjugate (ADC), in patients (pts) with HER2+ metastatic breast cancer (BC)", American Society of Clinical Oncology 43rd:June 02 (Abs 1042; Krop et al, European Cancer Conference ECCO, Poster 2118, September 23-27, 2007, Barcelona; US 7097840; US 2005/0276812; US 2005/0166993).

Maytansinoids, derivatives of the anti-mitotic drug maytansine, bind to microtubules in a manner similar to vinca alkaloid drugs (Issell BF et al (1978) Cancer Treat. Rev. 5:199-207; Cabanillas F et al. (1979) Cancer Treat Rep, 63:507-9. Antibody-drug conjugates (ADCs) composed of the maytansinoid DM1 linked to trastuzumab show potent anti-tumor activity in HER2-overexpressing trastuzumab-sensitive and trastuzumab-resistant tumor cell lines, and xenograft models of human breast cancer. A conjugate of maytansinoids linked to the anti-HER2 murine breast cancer antibody TA.1 via the mcc linker was 200-fold less potent than the corresponding conjugate with a disulfide linker (Chari et al (1992) Cancer Res. 127-133). Antibody-drug conjugates (ADCs) composed of the maytansinoid, DM1, linked to trastuzumab show potent anti-tumor activity in HER2-overexpressing trastuzumab-sensitive and -resistant tumor cell lines and xenograft models of human cancer.

Trastuzumab-mcc-DM1 (trastuzumab emtansine, trastuzumab-DM1; T-DM1; PRO132365), a novel antibody-drug conjugate (ADC) specifically designed for the treatment of HER2-positive breast cancer, is composed of the cytotoxic agent DM1 (a thiol-containing maytansinoid anti-microtubule agent) conjugated to trastuzumab (US 6407213) via lysine side chains, with an average drug to antibody ratio of about 3.4:1. T-DM1 is in development for the treatment of HER2+ metastatic breast cancer (Beeram M, Burris H, Modi S et al. (2008) J Clin Oncol 26: May 20 suppl; abstr 1028). T-DM1 binds to HER2 with affinity similar to that of trastuzumab. Such binding is required for T-DM1 anti-tumor activity (HERCEPTIN® Investigator Brochure, Genentech, Inc., South San Francisco, CA, July 2007). It is hypothesized that after binding to HER2, T-DM1 undergoes receptor-mediated internalization, resulting in intracellular release of DM1 and subsequent cell death (Austin CD, De Mazière AM, Pisacane PI, et al. (2004) Mol Biol Cell 15(12):5268-5282).

Trastuzumab-maytansinoid ADC with various linkers was tested for *in vitro* and *in vivo* efficacy, pharmacokinetics and toxicity in pre-clinical studies (Phillips et al (2008) Cancer Res. 68(22):9280-9290). Trastuzumab linked to DM1 through a non-reducible thioether linkage (mcc), displayed superior activity compared with unconjugated trastuzumab or trastuzumab linked to other maytansinoids through disulfide linkers. Because trastuzumab linked to DM1 through a non-reducible linker offers improved efficacy and pharmacokinetics and reduced toxicity over the reducible disulfide linkers evaluated, trastuzumab-mcc-DM1 was selected for clinical development.

DM1 is a thiol-containing maytansinoid derived from the naturally occurring ester ansamitocin P3 (Remillard S, Rebhun LI, Howie GA, et al. (1975) Science 189(4207):1002-1005.3; Cassady JM, Chan KK, Floss HG. (2004) Chem Pharm Bull 52(1):1-26.4). The related plant ester, maytansine, has been studied as a chemotherapeutic agent in approximately 800 patients, administered at a dose of 2.0 mg/m² every 3 weeks either as a single dose or for 3 consecutive days (Issell BF, Crooke ST. (1978) Maytansine. Cancer Treat Rev 5:199-207). Despite nonclinical activity, the activity of maytansine in the clinic was modest at doses that could be safely delivered. The dose-limiting toxicity (DLT) was gastrointestinal, consisting of nausea, vomiting, and diarrhea (often followed by constipation). These toxicities were dose dependent but not schedule dependent. Peripheral neuropathy (predominantly sensory) was reported and was most apparent in patients with preexisting neuropathy. Subclinical transient elevations of hepatic transaminase, alkaline phosphatase, and total bilirubin were reported. Constitutional toxicities, including weakness, lethargy, dysphoria, and insomnia, were common. Less common toxicities included infusion-site phlebitis and mild myelosuppression. Further development of the drug was abandoned in the 1980s because of the narrow therapeutic window.

Clinical results to date suggest that T-DM1 may benefit patients with HER2-positive MBC who have progressed while receiving HER2-directed therapy. Trastuzumab-mcc-DM1 (T-DM1) is currently undergoing evaluation in phase II clinical trials in patients whose disease is refractory to HER2-directed therapies (Beeram et al (2007) "A phase I study of trastuzumab-MCC-DM1 (T-DM1), a first-in-class HER2 antibody-drug conjugate (ADC), in patients (pts) with HER2+ metastatic breast cancer (BC)", American Society of Clinical Oncology 43rd:June 02 (Abs 1042; Krop et al, European Cancer Conference ECCO, Poster 2118, September 23-27, 2007, Barcelona; US 7097840; US 2005/0276812; US 2005/0166993)

The optimal linker moiety of antibody-drug conjugates affects is stable in systemic circulation, yet allows for efficient drug release at the target site (Alley et al (2008) Bioconjugate Chem. 19:759-765; Christie et al (2010) Bioconjugate Chem. 21:1779-1787; US 2008/0299668). Maleimido linked ADC may undergo retro-Michael addition of thiol to release drug prior to target receptor binding (Alley et al (2008) Bioconjugate Chem. 19:759-765). Both TMAb-mcc-DM1 and Thio-TMAb-mpeo-DM1 antibody conjugates have a maleimide in the linker attaching the DM1 thiol group to mcc-maleimide or mpeo-maleimide (US 7097840; US 2005/0276812; US 2005/0166993). Incubation of antibody-drug conjugates where a cysteine thiol of the antibody is linked through a maleimide group with rat and mice plasma formed albumin-drug conjugates, consistent with retro-Michael addition of thiol to release maleimide drug conjugate and addition with albumin cysteine thiol (Alley et al (2008) Bioconjugate Chem. 19:759-765). In analogous manner, retro-Michael addition of the thiol of drug moiety DM1 in ADC can result in the loss of drug from antibody and formation of albumin-antibody, cysteine-antibody or glutathione-antibody adducts. This cleavage instability of thio-maleimide linkages decreases the potency of administered ADC. New linkers without a maleimide group attached to maytansine may prevent non-specific maytansine drug loss by retro-Michael addition or other mechanisms in the plasma prior to targeted binding.

### SUMMARY

An aspect of the present invention is to provide new linker-drug compounds of Formula I for conjugation to antibodies to form antibody-drug conjugates. 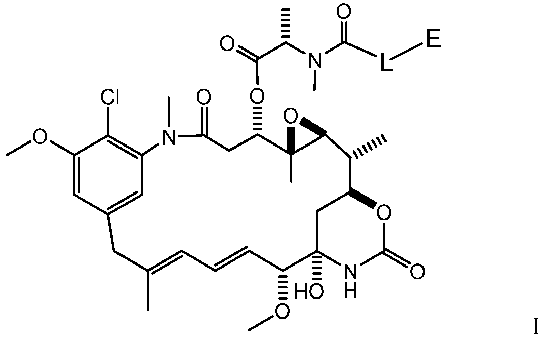 wherein:
L is 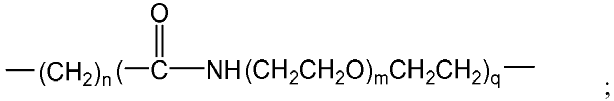
E is 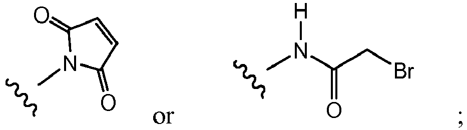
n is 2, 3, 4, 5, or 6; m is 2, 3 or 4; and q is 0 or 1.

An aspect of the present invention is to provide new antibody-drug conjugates of Formula Ia and Ib prepared from linker-drug compounds of Formula I. 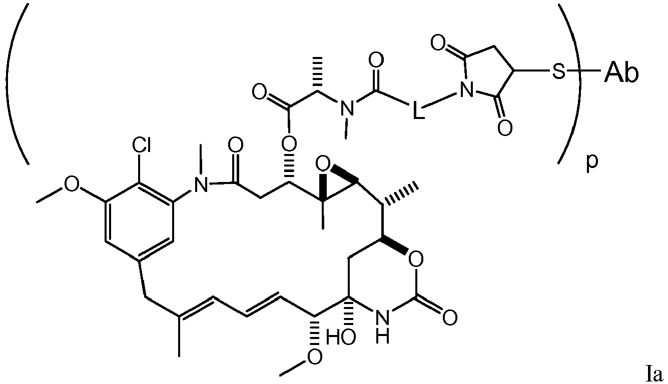 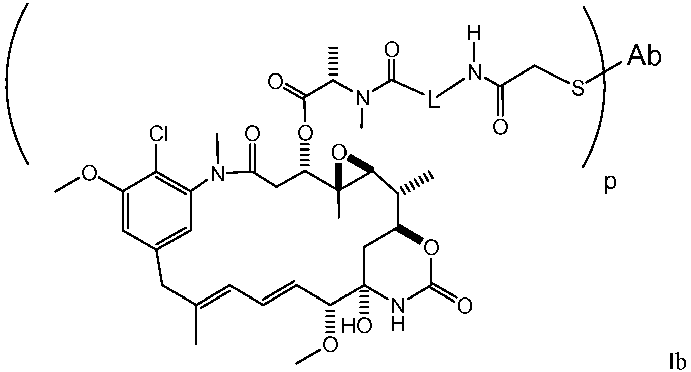 wherein:
L is 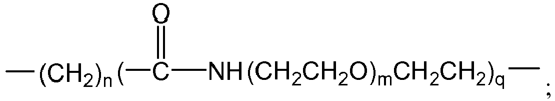
n is 2, 3, 4, 5, or 6; m is 2, 3 or 4; q is 0 or 1; p is 1 to 4; and Ab is an antibody.

The antibody may be a cysteine engineered antibody (Ab) conjugated through a free cysteine amino acid to a linker L.

An aspect of the invention is a pharmaceutical composition comprising an antibody-drug conjugate of Formula Ia or Ib and a pharmaceutically acceptable diluent, carrier or excipient.

Also disclosed is a method of treating cancer comprising to a patient a pharmaceutical composition comprising an antibody-drug conjugate of Formula Ia or Ib.

An aspect of the invention is the use of an antibody-drug conjugate of Formula Ia or Ib in the manufacture of a medicament for the treatment of cancer in a mammal.

An aspect of the invention is an article of manufacture comprising an antibody-drug conjugate of Formula Ia or Ib; a container; and a package insert or label indicating that the compound can be used to treat cancer.

### BRIEF DESCRIPTION OF THE DRAWINGS

Figure 1a shows the synthesis of drug-linker intermediate, mal-hex-ala-May **5**
Figure 1b shows the synthesis of drug intermediate, 3-(*S*-(N-methylalaninyl)maytansinol **4a**
Figure 2 shows the synthesis of drug-linker intermediate, bra-hex-ala-May **8**
Figure 3 shows the synthesis of drug-linker intermediate, mal-PEG3-ala-May **14**
Figure 4 shows the synthesis of drug-linker intermediate, bra-PEG3-ala-May **18**

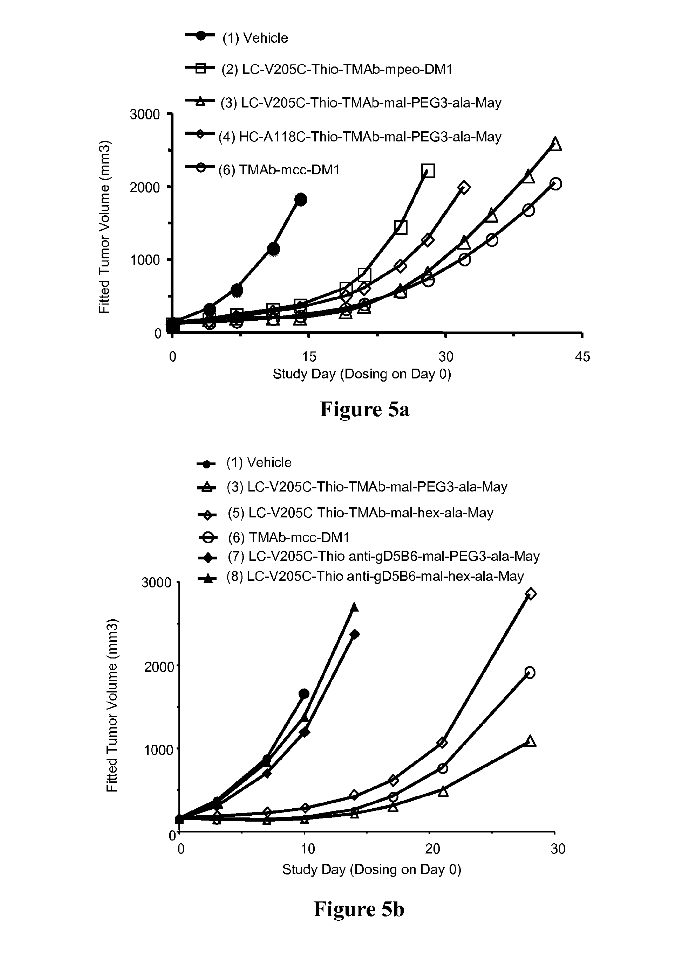

Figures 5a and 5b show plots of the in vivo fitted tumor volume change over time in MMTV-HER2 Fo5 transgenic mammary tumors inoculated into the mammary fat pad of CRL nu/nu mice after dosing with: (1) Vehicle (ADC buffer), (2) LC-V205C-Thio-TMAb-mpeo-DM1, (3) LC-V205C-Thio-TMAb-mal-PEG3-ala-May, (4) HC-A118C-Thio-TMAb-mal-PEG3-ala-May, (5) LC-V205C Thio-TMAb-mal-hex-ala-May, (6) TMAb-mcc-DM1 (trastuzumab-mcc-DM1, T-DM1), (7) LC-V205C-Thio anti-gD5B6-mal-PEG3-ala-May, (8) LC-V205C-Thio anti-gD5B6-mal-hex-ala-May (Examples 6, 8). All antibody drug conjugates (single doses) were dosed intravenously at 10 mg/kg. Anti-gD5B6 is a control antibody and its corresponding antigen does not express in Fo5 tumor tissues.

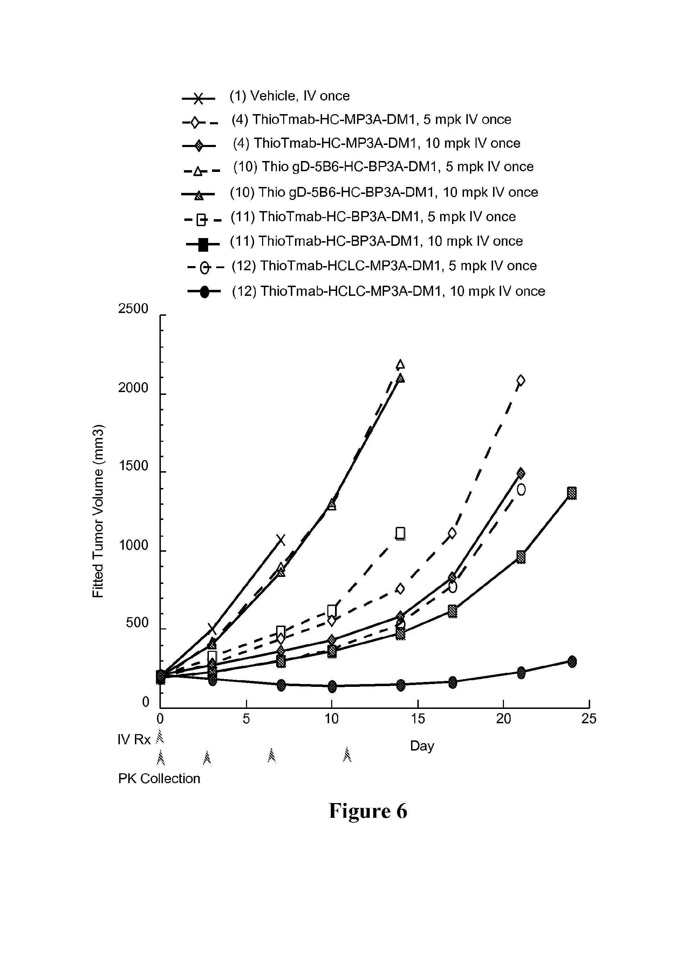

Figure 6 shows a plot of the in vivo fitted tumor volume change over time in MMTV-HER2 Fo5 transgenic mammary tumors inoculated into the mammary fat pad of CRL nu/nu mice after dosing with: (1) Vehicle: Histidine Buffer #8: 20mM Histidine Acetate, pH 5.5, 240mM Sucrose, 0.02% PS 20, (4) HC-A118C-Thio-TMAb-mal-PEG3-ala-May, 5 mg/kg, (4) HC-A118C-Thio-TMAb-mal-PEG3-ala-May, 10 mg/kg, (10) HC-A118C Thio anti-gD5B6-bra-PEG3-ala-May, 5 mg/kg, (10) HC-A118C Thio anti-gD5B6-bra-PEG3-ala-May, 10 mg/kg, (11) HC-A118C Thio TMAb-bra-PEG3-ala-May, 5 mg/kg, (11) HC-A118C Thio TMAb-bra-PEG3-ala-May, 10 mg/kg, (12) HC-A118C, LC-V205C Thio-TMAb-mal-PEG3-ala-May, 5 gm/kg, (12) HC-A118C, LC-V205C Thio-TMAb-mal-PEG3-ala-May, 10 gm/kg. All antibody drug conjugates (single doses) were dosed once intravenously at the start of the study.

### DETAILED DESCRIPTION OF EXEMPLARY EMBODIMENTS

Reference will now be made in detail to certain embodiments of the invention, examples of which are illustrated in the accompanying structures and formulas. While the invention will be described in conjunction with the illustrated embodiments, it will be understood that they are not intended to limit the invention to those embodiments. On the contrary, the invention is intended to cover all alternatives, modifications, and equivalents, which may be included within the scope of the present invention as defined by the claims.

One skilled in the art will recognize many methods and materials similar or equivalent to those described herein, which could be used in the practice of the present invention. The present invention is in no way limited to the methods and materials described.

Unless defined otherwise, technical and scientific terms used herein have the same meaning as commonly understood by one of ordinary skill in the art to which this invention belongs, and are consistent with: Singleton et al (1994) Dictionary of Microbiology and Molecular Biology, 2nd Ed., J. Wiley & Sons, New York, NY; and Janeway, C., Travers, P., Walport, M., Shlomchik (2001) Immunobiology, 5th Ed., Garland Publishing, New York.

### DEFINITIONS

Unless stated otherwise, the following terms and phrases as used herein are intended to have the following meanings:
When trade names are used herein, applicants intend to independently include the trade name product formulation, the generic drug, and the active pharmaceutical ingredient(s) of the trade name product.

The term "antibody" herein is used in the broadest sense and specifically covers monoclonal antibodies, polyclonal antibodies, dimers, multimers, multispecific antibodies (e.g., bispecific antibodies), and antibody fragments, so long as they exhibit the desired biological activity (Miller et al (2003) Jour. of Immunology 170:4854-4861). Antibodies may be murine, human, humanized, chimeric, or derived from other species. An antibody is a protein generated by the immune system that is capable of recognizing and binding to a specific antigen. (Janeway, C., Travers, P., Walport, M., Shlomchik (2001) Immuno Biology, 5th Ed., Garland Publishing, New York). A target antigen generally has numerous binding sites, also called epitopes, recognized by CDRs on multiple antibodies. Each antibody that specifically binds to a different epitope has a different structure. Thus, one antigen may have more than one corresponding antibody. An antibody includes a full-length immunoglobulin molecule or an immunologically active portion of a full-length immunoglobulin molecule, i.e., a molecule that contains an antigen binding site that immunospecifically binds an antigen of a target of interest or part thereof, such targets including but not limited to, cancer cell or cells that produce autoimmune antibodies associated with an autoimmune disease. The immunoglobulin disclosed herein can be of any type (e.g., IgG, IgE, IgM, IgD, and IgA), class (e.g., IgG1, IgG2, IgG3, IgG4, IgA1 and IgA2) or subclass of immunoglobulin molecule. The immunoglobulins can be derived from any species. In one aspect, however, the immunoglobulin is of human, murine, or rabbit origin.

"Antibody fragments" comprise a portion of a full length antibody, generally the antigen binding or variable region thereof. Examples of antibody fragments include Fab, Fab', F(ab')₂, and Fv fragments; diabodies; linear antibodies; minibodies (Olafsen et al (2004) Protein Eng. Design & Sel. 17(4):315-323), fragments produced by a Fab expression library, anti-idiotypic (anti-Id) antibodies, CDR (complementary determining region), and epitope-binding fragments of any of the above which immunospecifically bind to cancer cell antigens, viral antigens or microbial antigens, single-chain antibody molecules; and multispecific antibodies formed from antibody fragments.

The term "monoclonal antibody" as used herein refers to an antibody obtained from a population of substantially homogeneous antibodies, *i.e.*, the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al (1975) Nature, 256:495, or may be made by recombinant DNA methods (see for example: US 4816567; US 5807715). The monoclonal antibodies may also be isolated from phage antibody libraries using the techniques described in Clackson et al (1991) Nature, 352:624-628; Marks et al (1991) J. Mol. Biol., 222:581-597; for example.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (US 4816567; and Morrison et al (1984) Proc. Natl. Acad. Sci. USA, 81:6851-6855). Chimeric antibodies of interest herein include "primatized" antibodies comprising variable domain antigen-binding sequences derived from a non-human primate (*e.g.*, Old World Monkey, Ape etc) and human constant region sequences.

An "intact antibody" herein is one comprising a VL and VH domains, as well as a light chain constant domain (CL) and heavy chain constant domains, CH1, CH2 and CH3. The constant domains may be native sequence constant domains (*e.g.*, human native sequence constant domains) or amino acid sequence variant thereof. The intact antibody may have one or more "effector functions" which refer to those biological activities attributable to the Fc constant region (a native sequence Fc region or amino acid sequence variant Fc region) of an antibody. Examples of antibody effector functions include C1q binding; complement dependent cytotoxicity; Fc receptor binding; antibody-dependent cell-mediated cytotoxicity (ADCC); phagocytosis; and down regulation of cell surface receptors such as B cell receptor and BCR.

Depending on the amino acid sequence of the constant domain of their heavy chains, intact antibodies can be assigned to different "classes." There are five major classes of intact immunoglobulin antibodies: IgA, IgD, IgE, IgG, and IgM, and several of these may be further divided into "subclasses" (isotypes), *e.g.*, IgG1, IgG2, IgG3, IgG4, IgA, and IgA2. The heavy-chain constant domains that correspond to the different classes of antibodies are called α, δ, ε, y, and µ, respectively. The subunit structures and three-dimensional configurations of different classes of immunoglobulins are well known. Ig forms include hinge-modifications or hingeless forms (Roux et al (1998) J. Immunol. 161:4083-4090; Lund et al (2000) Eur. J. Biochem. 267:7246-7256; US 2005/0048572; US 2004/0229310).

A "free cysteine amino acid" refers to a cysteine amino acid residue which has been engineered into a parent antibody, has a thiol functional group (-SH), and is not paired as an intramolecular or intermolecular disulfide bridge.

The term "thiol reactivity value" is a quantitative characterization of the reactivity of free cysteine amino acids. The thiol reactivity value is the percentage of a free cysteine amino acid in a cysteine engineered antibody which reacts with a thiol-reactive reagent, and converted to a maximum value of 1. For example, a free cysteine amino acid on a cysteine engineered antibody which reacts in 100% yield with a thiol-reactive reagent, such as a biotin-maleimide reagent, to form a biotin-labelled antibody has a thiol reactivity value of 1.0. Another cysteine amino acid engineered into the same or different parent antibody which reacts in 80% yield with a thiol-reactive reagent has a thiol reactivity value of 0.8. Another cysteine amino acid engineered into the same or different parent antibody which fails totally to react with a thiol-reactive reagent has a thiol reactivity value of 0. Determination of the thiol reactivity value of a particular cysteine may be conducted by ELISA assay, mass spectroscopy, liquid chromatography, autoradiography, or other quantitative analytical tests.

A "parent antibody" is an antibody comprising an amino acid sequence from which one or more amino acid residues are replaced by one or more cysteine residues. The parent antibody may comprise a native or wild type sequence. The parent antibody may have preexisting amino acid sequence modifications (such as additions, deletions and/or substitutions) relative to other native, wild type, or modified forms of an antibody. A parent antibody may be directed against a target antigen of interest, e.g. a biologically important polypeptide. Antibodies directed against nonpolypeptide antigens (such as tumor-associated glycolipid antigens; see US 5091178) are also contemplated.

Exemplary parent antibodies include antibodies having affinity and selectivity for cell surface and transmembrane receptors and tumor-associated antigens (TAA).

"Phage display" is a technique by which variant polypeptides are displayed as fusion proteins to a coat protein on the surface of phage, e.g., filamentous phage, particles. One utility of phage display lies in the fact that large libraries of randomized protein variants can be rapidly and efficiently sorted for those sequences that bind to a target molecule with high affinity. Display of peptide and protein libraries on phage has been used for screening millions of polypeptides for ones with specific binding properties. Polyvalent phage display methods have been used for displaying small random peptides and small proteins, typically through fusions to either pIII or pVIII of filamentous phage (Wells and Lowman, (1992) Curr. Opin. Struct. Biol., 3:355-362, and references cited therein). In monovalent phage display, a protein or peptide library is fused to a phage coat protein or a portion thereof, and expressed at low levels in the presence of wild type protein. Avidity effects are reduced relative to polyvalent phage so that sorting is on the basis of intrinsic ligand affinity, and phagemid vectors are used, which simplify DNA manipulations. Lowman and Wells, Methods: A companion to Methods in Enzymology, 3:205-0216 (1991). Phage display includes techniques for producing antibody-like molecules (Janeway, C., Travers, P., Walport, M., Shlomchik (2001) Immunobiology, 5th Ed., Garland Publishing, New York, p627-628; Lee et al).

A "phagemid" is a plasmid vector having a bacterial origin of replication, e.g., Co1E1, and a copy of an intergenic region of a bacteriophage. The phagemid may be used on any known bacteriophage, including filamentous bacteriophage and lambdoid bacteriophage. The plasmid will also generally contain a selectable marker for antibiotic resistance. Segments of DNA cloned into these vectors can be propagated as plasmids. When cells harboring these vectors are provided with all genes necessary for the production of phage particles, the mode of replication of the plasmid changes to rolling circle replication to generate copies of one strand of the plasmid DNA and package phage particles. The phagemid may form infectious or non-infectious phage particles. This term includes phagemids which contain a phage coat protein gene or fragment thereof linked to a heterologous polypeptide gene as a gene fusion such that the heterologous polypeptide is displayed on the surface of the phage particle.

"Linker", "Linker Unit", or "link" means a chemical moiety comprising a chain of atoms that covalently attaches an antibody to a drug moiety. In various embodiments, a linker is a divalent radical, specified as L.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., Stereochemistry of Organic Compounds (1994) John Wiley & Sons, Inc., New York. Many organic compounds exist in optically active forms, i.e., they have the ability to rotate the plane of plane-polarized light. In describing an optically active compound, the prefixes D and L, or *R* and *S*, are used to denote the absolute configuration of the molecule about its chiral center(s). The prefixes d and 1 or (+) and (-) are employed to designate the sign of rotation of plane-polarized light by the compound, with (-) or 1 meaning that the compound is levorotatory. A compound prefixed with (+) or d is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process. The terms "racemic mixture" and "racemate" refer to an equimolar mixture of two enantiomeric species, devoid of optical activity.

The phrase "pharmaceutically acceptable salt," as used herein, refers to pharmaceutically acceptable organic or inorganic salts of an antibody-drug conjugate (ADC). Exemplary salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, *p*-toluenesulfonate, and pamoate (*i.e.*, 1,1'-methylene-bis -(2-hydroxy-3- naphthoate)) salts. A pharmaceutically acceptable salt may involve the inclusion of another molecule such as an acetate ion, a succinate ion or other counterion. The counterion may be any organic or inorganic moiety that stabilizes the charge on the parent compound. Furthermore, a pharmaceutically acceptable salt may have more than one charged atom in its structure. Instances where multiple charged atoms are part of the pharmaceutically acceptable salt can have multiple counter ions. Hence, a pharmaceutically acceptable salt can have one or more charged atoms and/or one or more counterion.

The following abbreviations are used herein and have the indicated definitions: BME is beta-mercaptoethanol, Boc is *N-*(*t*-butoxycarbonyl), cit is citrulline (2-amino-5-ureido pentanoic acid), DCC is 1,3-dicyclohexylcarbodiimide, DCM is dichloromethane, DEA is diethylamine, DEAD is diethylazodicarboxylate, DEPC is diethylphosphorylcyanidate, DIAD is diisopropylazodicarboxylate, DIEA is *N,N-*diisopropylethylamine, DMA is dimethylacetamide, DMAP is 4-dimethylaminopyridine, DME is ethyleneglycol dimethyl ether (or 1,2-dimethoxyethane), DMF is *N,N-*dimethylformamide, DMSO is dimethylsulfoxide, DTT is dithiothreitol, EDCI is 1-(3-dimethylaminopropyl)-3-ethylcarbodiimide hydrochloride, EEDQ is 2-ethoxy-1-ethoxycarbonyl-1,2-dihydroquinoline, ES-MS is electrospray mass spectrometry, EtOAc is ethyl acetate, Fmoc is *N-*(9-fluorenylmethoxycarbonyl), gly is glycine, HATU is O-(7-azabenzotriazol-1-yl)-*N,N,N',N*'-tetramethyluronium hexafluorophosphate, HOBt is 1-hydroxybenzotriazole, HPLC is high pressure liquid chromatography, ile is isoleucine, lys is lysine, MeCN (CH₃CN) is acetonitrile, MeOH is methanol, Mtr is 4-anisyldiphenylmethyl (or 4-methoxytrityl), NHS is N-hydroxysuccinimide, PBS is phosphate-buffered saline (pH 7), PEG is polyethylene glycol or a unit of ethylene glycol (-OCH₂CH₂-), Ph is phenyl, Pnp is p-nitrophenyl, MC is 6-maleimidocaproyl, phe is L-phenylalanine, PyBrop is bromo *tris-*pyrrolidino phosphonium hexafluorophosphate, SEC is size-exclusion chromatography, Su is succinimide, TFA is trifluoroacetic acid, TLC is thin layer chromatography, UV is ultraviolet, and val is valine.

### CYSTEINE ENGINEERED ANTIBODIES

The compounds of the invention include cysteine engineered antibodies where one or more amino acids of a wild-type or parent antibody are replaced with a cysteine amino acid. Any form of antibody may be so engineered, i.e. mutated. For example, a parent Fab antibody fragment may be engineered to form a cysteine engineered Fab, referred to herein as "ThioFab." Similarly, a parent monoclonal antibody may be engineered to form a "ThioMab." It should be noted that a single site mutation yields a single engineered cysteine residue in a ThioFab, while a single site mutation yields two engineered cysteine residues in a ThioMab, due to the dimeric nature of the IgG antibody. Mutants with replaced ("engineered") cysteine (Cys) residues are evaluated for the reactivity of the newly introduced, engineered cysteine thiol groups. The thiol reactivity value is a relative, numerical term in the range of 0 to 1.0 and can be measured for any cysteine engineered antibody. Thiol reactivity values of cysteine engineered antibodies of the invention are in the ranges of 0.6 to 1.0; 0.7 to 1.0; or 0.8 to 1.0.

The design, selection, and preparation methods of the invention enable cysteine engineered antibodies which are reactive with electrophilic functionality. These methods further enable antibody conjugate compounds such as antibody-zirconium conjugate (AZC) compounds with zirconium atoms at designated, designed, selective sites. Reactive cysteine residues on an antibody surface allow specifically conjugating a zirconium moiety through a thiol reactive group such as maleimide or haloacetyl. The nucleophilic reactivity of the thiol functionality of a Cys residue to a maleimide group is about 1000 times higher compared to any other amino acid functionality in a protein, such as amino group of lysine residues or the N-terminal amino group. Thiol specific functionality in iodoacetyl and maleimide reagents may react with amine groups, but higher pH (>9.0) and longer reaction times are required (Garman, 1997, Non-Radioactive Labelling: A Practical Approach, Academic Press, London).

Cysteine engineered antibodies of the invention preferably retain the antigen binding capability of their wild type, parent antibody counterparts. Thus, cysteine engineered antibodies are capable of binding, preferably specifically, to antigens. Such antigens include, for example, tumor-associated antigens (TAA), cell surface receptor proteins and other cell surface molecules, transmembrane proteins, signaling proteins, cell survival regulatory factors, cell proliferation regulatory factors, molecules associated with (for e.g., known or suspected to contribute functionally to) tissue development or differentiation, lymphokines, cytokines, molecules involved in cell cycle regulation, molecules involved in vasculogenesis and molecules associated with (for e.g., known or suspected to contribute functionally to) angiogenesis. The tumor-associated antigen may be a cluster differentiation factor (i.e., a CD protein). An antigen to which a cysteine engineered antibody is capable of binding may be a member of a subset of one of the above-mentioned categories, wherein the other subset(s) of said category comprise other molecules/antigens that have a distinct characteristic (with respect to the antigen of interest).

The parent antibody may also be a humanized antibody selected from huMAb4D5-1, huMAb4D5-2, huMAb4D5-3, huMAb4D5-4, huMAb4D5-5, huMAb4D5-6, huMAb4D5-7 and huMAb4D5-8 (Trastuzumab, HERCEPTIN®) as described in Table 3 of US 5821337; humanized 520C9 (WO 93/21319) and humanized 2C4 antibodies as described herein.

Cysteine engineered antibodies of the invention may be site-specifically and efficiently coupled with a thiol-reactive reagent. The thiol-reactive reagent may be a multifunctional linker reagent, a capture, i.e. affinity, label reagent (e.g. a biotin-linker reagent), a detection label (e.g. a fluorophore reagent), a solid phase immobilization reagent (e.g. SEPHAROSE™, polystyrene, or glass), or a zirconium-linker intermediate. One example of a thiol-reactive reagent is N-ethyl maleimide (NEM). In an exemplary embodiment, reaction of a ThioFab with a biotin-linker reagent provides a biotinylated ThioFab by which the presence and reactivity of the engineered cysteine residue may be detected and measured. Reaction of a ThioFab with a multifunctional linker reagent provides a ThioFab with a functionalized linker which may be further reacted with a zirconium moiety reagent or other label. Reaction of a ThioFab with a zirconium-linker intermediate provides a ThioFab zirconium conjugate.

The exemplary methods described here may be applied generally to the identification and production of antibodies, and more generally, to other proteins through application of the design and screening steps described herein.

Such an approach may be applied to the conjugation of other thiol-reactive agents in which the reactive group is, for example, a maleimide, an iodoacetamide, a pyridyl disulfide, or other thiol-reactive conjugation partner (Haugland, 2003, Molecular Probes Handbook of Fluorescent Probes and Research Chemicals, Molecular Probes, Inc.; Brinkley, 1992, Bioconjugate Chem. 3:2; Garman, 1997, Non-Radioactive Labelling: A Practical Approach, Academic Press, London; Means (1990) Bioconjugate Chem. 1:2; Hermanson, G. in Bioconjugate Techniques (1996) Academic Press, San Diego, pp. 40-55, 643-671). The partner may be a cytotoxic agent (e.g. a toxin such as doxorubicin or pertussis toxin), a fluorophore such as a fluorescent dye like fluorescein or rhodamine, a chelating agent for an imaging or radiotherapeutic metal, a peptidyl or non-peptidyl label or detection tag, or a clearance-modifying agent such as various isomers of polyethylene glycol, a peptide that binds to a third component, or another carbohydrate or lipophilic agent.

The sites identified on the exemplary antibody fragment, hu4D5Fabv8, herein are primarily in the constant domain of an antibody which is well conserved across all species of antibodies. These sites should be broadly applicable to other antibodies, without further need of structural design or knowledge of specific antibody structures, and without interference in the antigen binding properties inherent to the variable domains of the antibody.

Cysteine engineered antibodies which may be useful in the treatment of cancer include, but are not limited to, antibodies against cell surface receptors and tumor-associated antigens (TAA). Such antibodies may be used as naked antibodies (unconjugated to a label moiety) or as Formula I antibody-drug conjugates (ADC). Tumor-associated antigens are known in the art, and can prepared for use in generating antibodies using methods and information which are well known in the art. In attempts to discover effective cellular targets for cancer diagnosis and therapy, researchers have sought to identify transmembrane or otherwise tumor-associated polypeptides that are specifically expressed on the surface of one or more particular type(s) of cancer cell as compared to on one or more normal non-cancerous cell(s). Often, such tumor-associated polypeptides are more abundantly expressed on the surface of the cancer cells as compared to on the surface of the non-cancerous cells. The identification of such tumor-associated cell surface antigen polypeptides has given rise to the ability to specifically target cancer cells for destruction via antibody-based therapies.

Examples of tumor-associated antigens TAA include, but are not limited to, TAA (1)-(51) listed below. For convenience, information relating to these antigens, all of which are known in the art, is listed below and includes names, alternative names, Genbank accession numbers and primary reference(s), following nucleic acid and protein sequence identification conventions of the National Center for Biotechnology Information (NCBI). Nucleic acid and protein sequences corresponding to TAA (1)-(51) are available in public databases such as GenBank. Tumor-associated antigens targeted by antibodies include all amino acid sequence variants and isoforms possessing at least about 70%, 80%, 85%, 90%, or 95% sequence identity relative to the sequences identified in the cited references, or which exhibit substantially the same biological properties or characteristics as a TAA having a sequence found in the cited references. For example, a TAA having a variant sequence generally is able to bind specifically to an antibody that binds specifically to the TAA with the corresponding sequence listed.

### TUMOR-ASSOCIATED ANTIGENS (1)-(51):

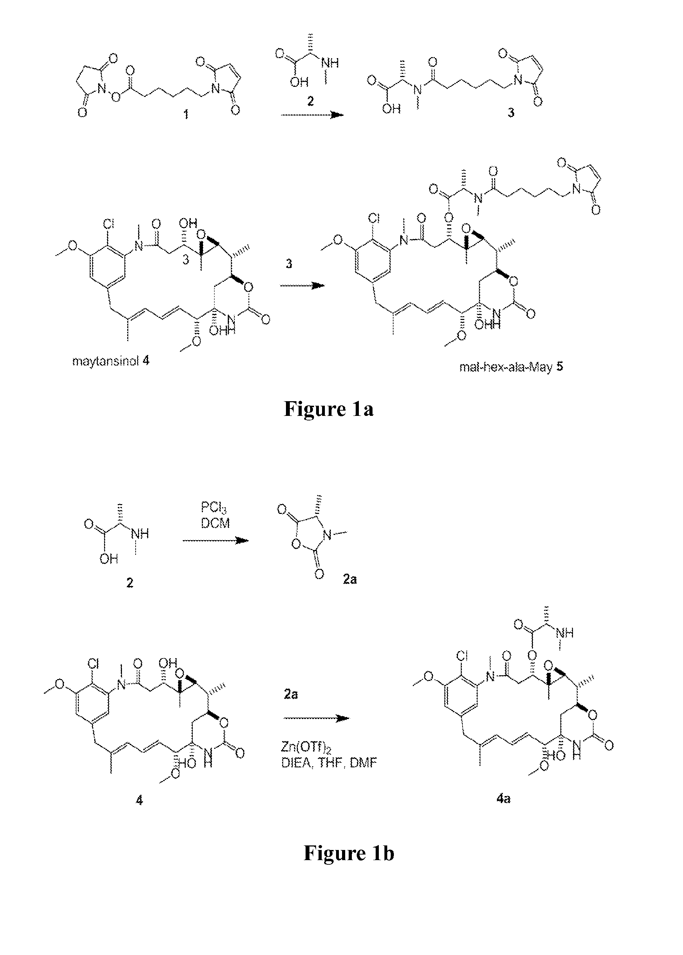

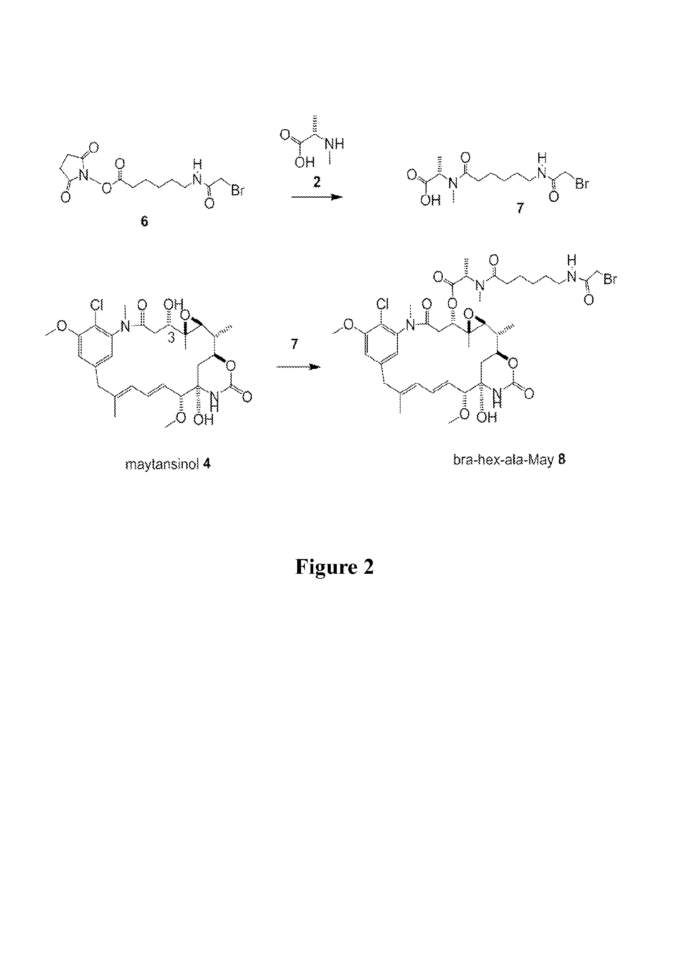

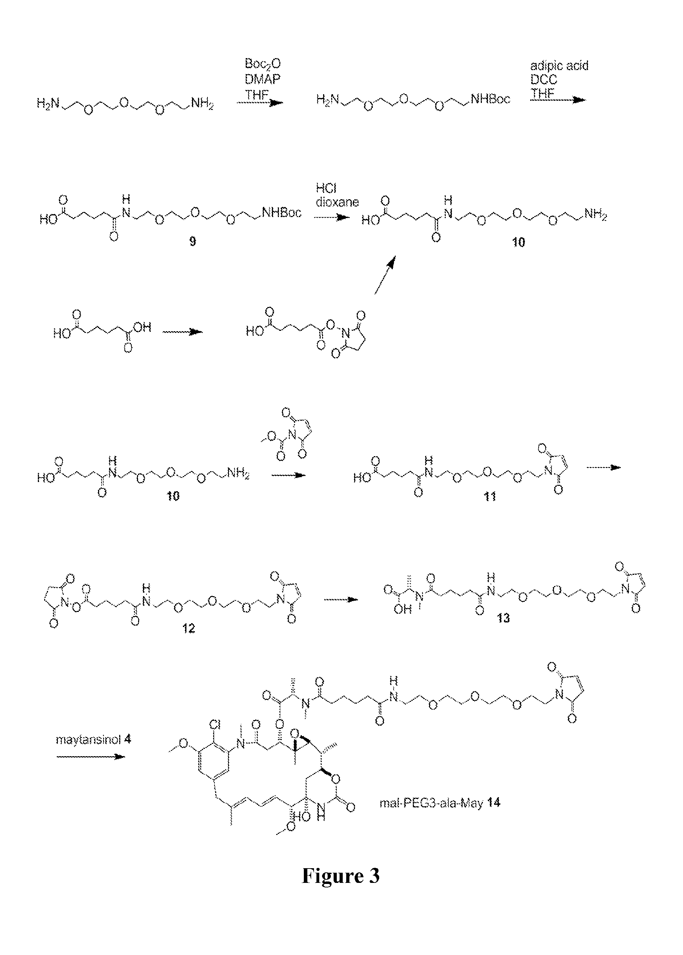

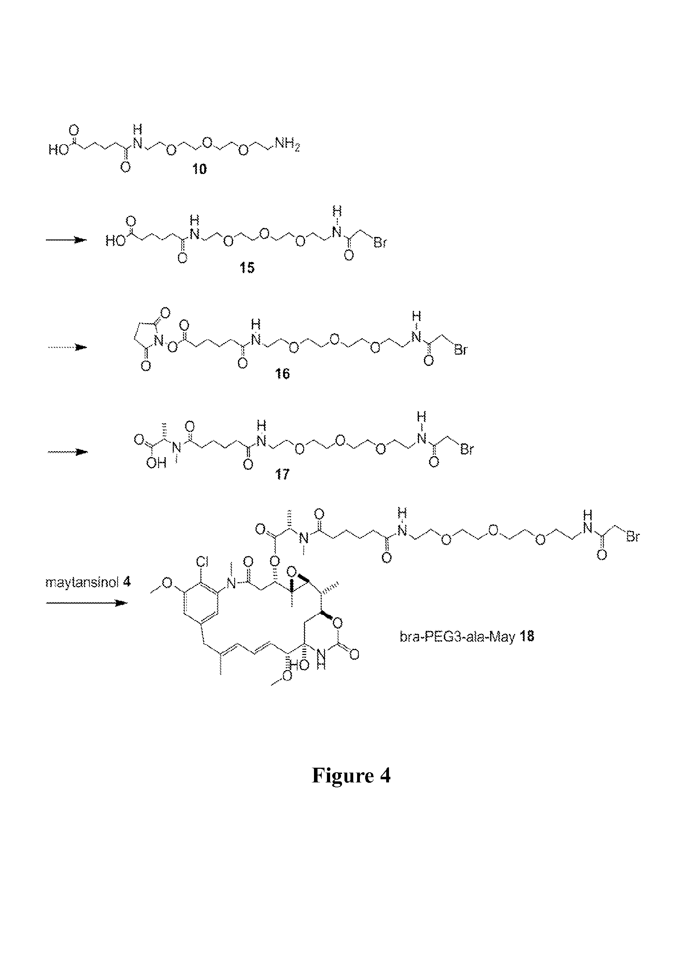

(1) BMPR1B (bone morphogenetic protein receptor-type IB, Genbank accession no.NM_001203)
   ten Dijke,P., et al Science 264 (5155):101-104 (1994), Oncogene 14 (11):1377-1382 (1997)); WO2004063362 (Claim 2); WO2003042661 (Claim 12); US2003134790-A1 (Page 38-39); WO2002102235 (Claim 13; Page 296); WO2003055443 (Page 91-92); WO200299122 (Example 2; Page 528-530); WO2003029421 (Claim 6); WO2003024392 (Claim 2; Fig 112); WO200298358 (Claim 1; Page 183); WO200254940 (Page 100-101); WO200259377(Page 349-350); WO200230268 (Claim 27; Page 376); WO200148204 (Example; Fig 4)
   NP_001194 bone morphogenetic protein receptor, type IB /pid=NP_001194.1 -
   Cross-references: MIM:603248; NP_001194.1; AY065994
(2) E16 (LAT1, SLC7A5, Genbank accession no. NM_003486)
   Biochem. Biophys. Res. Commun. 255 (2), 283-288 (1999), Nature 395 (6699):288-291 (1998), Gaugitsch, H.W., et al (1992) J. Biol. Chem. 267 (16):11267-11273); WO2004048938 (Example 2); WO2004032842 (Example IV); WO2003042661 (Claim 12); WO2003016475 (Claim 1); WO200278524 (Example 2); WO200299074 (Claim 19; Page 127-129); WO200286443 (Claim 27; Pages 222, 393); WO2003003906 (Claim 10; Page 293); WO200264798 (Claim 33; Page 93-95); WO200014228 (Claim 5; Page 133-136); US2003224454 (Fig 3); WO2003025138 (Claim 12; Page 150);
   NP_003477 solute carrier family 7 (cationic amino acid transporter, y+ system), member 5 /pid=NP_003477.3 - Homo sapiens
   Cross-references: MIM:600182; NP_003477.3; NM_015923; NMR_003486_1
(3) STEAP1 (six transmembrane epithelial antigen of prostate, Genbank accession no. NM_012449)
   Cancer Res. 61 (15), 5857-5860 (2001), Hubert, R.S., et al (1999) Proc. Natl. Acad. Sci. U.S.A. 96 (25):14523-14528); WO2004065577 (Claim 6); WO2004027049 (Fig 1L); EP1394274 (Example 11); WO2004016225 (Claim 2); WO2003042661 (Claim 12); US2003157089 (Example 5); US2003185830 (Example 5); US2003064397 (Fig 2); WO200289747 (Example 5; Page 618-619); WO2003022995 (Example 9; Fig 13A, Example 53; Page 173, Example 2; Fig 2A);
   NP_036581 six transmembrane epithelial antigen of the prostate
   Cross-references: MIM:604415; NP_036581.1; NM_012449_1
(4) 0772P (CA125, MUC16, Genbank accession no. AF361486)
   J. Biol. Chem. 276 (29):27371-27375 (2001)); WO2004045553 (Claim 14); WO200292836 (Claim 6; Fig 12); WO200283866 (Claim 15; Page 116-121); US2003124140 (Example 16); Cross-references: GI:34501467; AAK74120.3; AF361486_1
(5) MPF (MPF, MSLN, SMR, megakaryocyte potentiating factor, mesothelin,
   Genbank accession no. NM_005823) Yamaguchi, N., et al Biol. Chem. 269 (2), 805-808 (1994), Proc. Natl. Acad. Sci. U.S.A. 96 (20):11531-11536 (1999), Proc. Natl. Acad. Sci. U.S.A. 93 (1):136-140 (1996), J. Biol. Chem. 270 (37):21984-21990 (1995)); WO2003101283 (Claim 14); (WO2002102235 (Claim 13; Page 287-288); WO2002101075 (Claim 4; Page 308-309); WO200271928 (Page 320-321); WO9410312 (Page 52-57); Cross-references: MIM:601051; NP_005814.2; NM_005823_1
(6) Napi3b (NAPI-3B, NPTIIb, SLC34A2, solute carrier family 34 (sodium phosphate), member 2, type II sodium-dependent phosphate transporter 3b,Genbank accession no. NM_006424)
   J. Biol. Chem. 277 (22):19665-19672 (2002), Genomics 62 (2):281-284 (1999), Feild, J.A., et al (1999) Biochem. Biophys. Res. Commun. 258 (3):578-582); WO2004022778 (Claim 2); EP1394274 (Example 11); WO2002102235 (Claim 13; Page 326); EP875569 (Claim 1; Page 17-19); WO200157188 (Claim 20; Page 329); WO2004032842 (Example IV); WO200175177 (Claim 24; Page 139-140);
   Cross-references: MIM:604217; NP_006415.1; NMR_006424_1
(7) Sema 5b (FLJ10372, KIAA1445, Mm.42015, SEMA5B, SEMAG, Semaphorin 5b Hlog, sema domain, seven thrombospondin repeats (type 1 and type 1-like), transmembrane domain (TM) and short cytoplasmic domain, (semaphorin) 5B, Genbank accession no. AB040878)
   Nagase T., et al (2000) DNA Res. 7 (2):143-150); WO2004000997 (Claim 1); WO2003003984 (Claim 1); WO200206339 (Claim 1; Page 50); WO200188133 (Claim 1; Page 41-43, 48-58); WO2003054152 (Claim 20); WO2003101400 (Claim 11);
   Accession: Q9P283; EMBL; AB040878; BAA95969.1. Genew; HGNC:10737;
(8) PSCA hlg (2700050C12Rik, C530008O16Rik RIKEN cDNA 2700050C12, RIKEN cDNA 270005OC12 gene, Genbank accession no. AY358628); Ross et al (2002) Cancer Res. 62:2546-2553; US2003129192 (Claim 2); US2004044180 (Claim 12); US2004044179 (Claim 11); US2003096961 (Claim 11); US2003232056 (Example 5); WO2003105758 (Claim 12); US2003206918 (Example 5); EP1347046 (Claim 1); WO2003025148 (Claim 20);
   Cross-references: GI:37182378; AAQ88991.1; AY358628_1
(9) ETBR (Endothelin type B receptor, Genbank accession no. AY275463);
   Nakamuta M., et al Biochem. Biophys. Res. Commun. 177, 34-39, 1991; Ogawa Y., et al Biochem. Biophys. Res. Commun. 178, 248-255, 1991; Arai H., et al Jpn. Circ. J. 56, 1303-1307, 1992; Arai H., et al J. Biol. Chem. 268, 3463-3470, 1993; Sakamoto A., Yanagisawa M., et al Biochem. Biophys. Res. Commun. 178, 656-663, 1991; Elshourbagy N.A., et al J. Biol. Chem. 268, 3873-3879, 1993; Haendler B., et al J. Cardiovasc. Pharmacol. 20, s1-S4, 1992; Tsutsumi M., et al Gene 228, 43-49, 1999; Strausberg R.L., et al Proc. Natl. Acad. Sci. U.S.A. 99, 16899-16903, 2002; Bourgeois C., et al J. Clin. Endocrinol. Metab. 82, 3116-3123, 1997; Okamoto Y., et al Biol. Chem. 272, 21589-21596, 1997; Verheij J.B., et al Am. J. Med. Genet. 108, 223-225, 2002; Hofstra R.M.W., et al Eur. J. Hum. Genet. 5, 180-185, 1997; Puffenberger E.G., et al Cell 79, 1257-1266, 1994; Attie T., et al, Hum. Mol. Genet. 4, 2407-2409, 1995; Auricchio A., et al Hum. Mol. Genet. 5:351-354, 1996; Amiel J., et al Hum. Mol. Genet. 5, 355-357, 1996; Hofstra R.M.W., et al Nat. Genet. 12, 445-447, 1996; Svensson P.J., et al Hum. Genet. 103, 145-148, 1998; Fuchs S., et al Mol. Med. 7, 115-124, 2001; Pingault V., et al (2002) Hum. Genet. 111, 198-206; WO2004045516 (Claim 1); WO2004048938 (Example 2); WO2004040000 (Claim 151); WO2003087768 (Claim 1); WO2003016475 (Claim 1); WO2003016475 (Claim 1); WO200261087 (Fig 1); WO2003016494 (Fig 6); WO2003025138 (Claim 12; Page 144); WO200198351 (Claim 1; Page 124-125); EP522868 (Claim 8; Fig 2); WO200177172 (Claim 1; Page 297-299); US2003109676; US6518404 (Fig 3); US5773223 (Claim 1a; Col 31-34); WO2004001004;
(10) MSG783 (RNF124, hypothetical protein FLJ20315, Genbank accession no. NM_017763);
   WO2003104275 (Claim 1); WO2004046342 (Example 2); WO2003042661 (Claim 12); WO2003083074 (Claim 14; Page 61); WO2003018621 (Claim 1); WO2003024392 (Claim 2; Fig 93); WO200166689 (Example 6);
   Cross-references: LocusID:54894; NP_060233.2; NMR_017763_1
(11) STEAP2 (HGNC_8639, IPCA-1, PCANAP1, STAMP1, STEAP2, STMP, prostate cancer associated gene 1, prostate cancer associated protein 1, six transmembrane epithelial antigen of prostate 2, six transmembrane prostate protein, Genbank accession no. AF455138)
   Lab. Invest. 82 (11):1573-1582 (2002)); WO2003087306; US2003064397 (Claim 1; Fig 1); WO200272596 (Claim 13; Page 54-55); WO200172962 (Claim 1; Fig 4B); WO2003104270 (Claim 11); WO2003104270 (Claim 16); US2004005598 (Claim 22); WO2003042661 (Claim 12); US2003060612 (Claim 12; Fig 10); WO200226822 (Claim 23; Fig 2); WO200216429 (Claim 12; Fig 10);
   Cross-references: GI:22655488; AAN04080.1; AF455138_1
(12) TrpM4 (BR22450, FLJ20041, TRPM4, TRPM4B, transient receptor potential cation channel, subfamily M, member 4, Genbank accession no. NM_017636)
   Xu, X.Z., et al Proc. Natl. Acad. Sci. U.S.A. 98 (19):10692-10697 (2001), Cell 109 (3):397-407 (2002), J. Biol. Chem. 278 (33):30813-30820 (2003)); US2003143557 (Claim 4); WO200040614 (Claim 14; Page 100-103); WO200210382 (Claim 1; Fig 9A); WO2003042661 (Claim 12); WO200230268 (Claim 27; Page 391); US2003219806 (Claim 4); WO200162794 (Claim 14; Fig 1A-D);
   Cross-references: MIM:606936; NP_060106.2; NM_017636_1
(13) CRIPTO (CR, CR1, CRGF, CRIPTO, TDGF1, teratocarcinoma-derived growth factor, Genbank accession no. NP_003203 or NM_003212)
   Ciccodicola, A., et al EMBO J. 8 (7):1987-1991 (1989), Am. J. Hum. Genet. 49 (3):555-565 (1991)); US2003224411 (Claim 1); WO2003083041 (Example 1); WO2003034984 (Claim 12); WO200288170 (Claim 2; Page 52-53); WO2003024392 (Claim 2; Fig 58); WO200216413 (Claim 1; Page 94-95, 105); WO200222808 (Claim 2; Fig 1); US5854399 (Example 2; Col 17-18); US5792616 (Fig 2);
   Cross-references: MIM:187395; NP_003203.1; NMR_003212_1
(14) CD21 (CR2 (Complement receptor 2) or C3DR (C3d/Epstein Barr virus receptor) or Hs.73792 Genbank accession no. M26004)
   Fujisaku et al (1989) J. Biol. Chem. 264 (4):2118-2125); Weis J.J., et al J. Exp. Med. 167, 1047-1066, 1988; Moore M., et al Proc. Natl. Acad. Sci. U.S.A. 84, 9194-9198, 1987; Barel M., et al Mol. Immunol. 35, 1025-1031, 1998; Weis J.J., et al Proc. Natl. Acad. Sci. U.S.A. 83, 5639-5643, 1986; Sinha S.K., et al (1993) J. Immunol. 150, 5311-5320; WO2004045520 (Example 4); US2004005538 (Example 1); WO2003062401 (Claim 9); WO2004045520 (Example 4);
   WO9102536 (Fig 9.1-9.9); WO2004020595 (Claim 1);
   Accession: P20023; Q13866; Q14212; EMBL; M26004; AAA35786.1.
(15) CD79b (CD79B, CD79β, IGb (immunoglobulin-associated beta), B29, Genbank accession no. NM_000626 or 11038674)
   Proc. Natl. Acad. Sci. U.S.A. (2003) 100 (7):4126-4131, Blood (2002) 100 (9):3068-3076, Muller et al (1992) Eur. J. Immunol. 22 (6):1621-1625); WO2004016225 (claim 2, Fig 140); WO2003087768, US2004101874 (claim 1, page 102); WO2003062401 (claim 9); WO200278524 (Example 2); US2002150573 (claim 5, page 15); US5644033; WO2003048202 (claim 1, pages 306 and 309); WO 99/558658, US6534482 (claim 13, Fig 17A/B); WO200055351 (claim 11, pages 1145-1146);
   Cross-references: MIM:147245; NP_000617.1; NMR_000626_1
(16) FcRH2 (IFGP4, IRTA4, SPAP1A (SH2 domain containing phosphatase anchor protein 1a), SPAP1B, SPAP1C, Genbank accession no. NM_030764, AY358130)
   Genome Res. 13 (10):2265-2270 (2003), Immunogenetics 54 (2):87-95 (2002), Blood 99 (8):2662-2669 (2002), Proc. Natl. Acad. Sci. U.S.A. 98 (17):9772-9777 (2001), Xu, M.J., et al (2001) Biochem. Biophys. Res. Commun. 280 (3):768-775; WO2004016225 (Claim 2); WO2003077836; WO200138490 (Claim 5; Fig 18D-1-18D-2); WO2003097803 (Claim 12); WO2003089624 (Claim 25);
   Cross-references: MIM:606509; NP_110391.2; NM_030764_1
(17) HER2 (ErbB2, Genbank accession no. M11730)
   Coussens L., et al Science (1985) 230(4730):1132-1139); Yamamoto T., et al Nature 319, 230-234, 1986; Semba K., et al Proc. Natl. Acad. Sci. U.S.A. 82, 6497-6501, 1985; Swiercz J.M., et al J. Cell Biol. 165, 869-880, 2004; Kuhns J.J., et al J. Biol. Chem. 274, 36422-36427, 1999; Cho H.-S., et al Nature 421, 756-760, 2003; Ehsani A., et al (1993) Genomics 15, 426-429; WO2004048938 (Example 2); WO2004027049 (Fig 1I); WO2004009622; WO2003081210; WO2003089904 (Claim 9); WO2003016475 (Claim 1); US2003118592; WO2003008537 (Claim 1); WO2003055439 (Claim 29; Fig 1A-B); WO2003025228 (Claim 37; Fig 5C); WO200222636 (Example 13; Page 95-107); WO200212341 (Claim 68; Fig 7); WO200213847 (Page 71-74); WO200214503 (Page 114-117); WO200153463 (Claim 2; Page 41-46); WO200141787 (Page 15); WO200044899 (Claim 52; Fig 7); WO200020579 (Claim 3; Fig 2); US5869445 (Claim 3; Col 31-38);
   WO9630514 (Claim 2; Page 56-61); EP1439393 (Claim 7); WO2004043361 (Claim 7); WO2004022709; WO200100244 (Example 3; Fig 4); Accession: P04626; EMBL; M11767; AAA35808.1. EMBL; M11761; AAA35808.1.
(18) NCA (CEACAM6, Genbank accession no. M18728);
   Barnett T., et al Genomics 3, 59-66, 1988; Tawaragi Y., et al Biochem. Biophys. Res. Commun. 150, 89-96, 1988; Strausberg R.L., et al Proc. Natl. Acad. Sci. U.S.A. 99:16899-16903, 2002; WO2004063709; EP1439393 (Claim 7); WO2004044178 (Example 4); WO2004031238; WO2003042661 (Claim 12); WO200278524 (Example 2); WO200286443 (Claim 27; Page 427); WO200260317 (Claim 2);
   Accession: P40199; Q14920; EMBL; M29541; AAA59915.1. EMBL; M18728;
(19) MDP (DPEP1, Genbank accession no. BC017023)
   Proc. Natl. Acad. Sci. U.S.A. 99 (26):16899-16903 (2002)); WO2003016475 (Claim 1); WO200264798 (Claim 33; Page 85-87); JP05003790 (Fig 6-8); WO9946284 (Fig 9);
   Cross-references: MIM:179780; AAH17023.1; BC017023_1
(20) IL20Rα (IL20Ra, ZCYTOR7, Genbank accession no. AF184971);
   Clark H.F., et al Genome Res. 13, 2265-2270, 2003; Mungall A.J., et al Nature 425, 805-811, 2003; Blumberg H., et al Cell 104, 9-19, 2001; Dumoutier L., et al J. Immunol. 167, 3545-3549, 2001; Parrish-Novak J., et al J. Biol. Chem. 277, 47517-47523, 2002; Pletnev S., et al (2003) Biochemistry 42:12617-12624; Sheikh F., et al (2004) J. Immunol. 172, 2006-2010; EP1394274 (Example 11); US2004005320 (Example 5); WO2003029262 (Page 74-75); WO2003002717 (Claim 2; Page 63); WO200222153 (Page 45-47); US2002042366 (Page 20-21); WO200146261 (Page 57-59); WO200146232 (Page 63-65); WO9837193 (Claim 1; Page 55-59);
   Accession: Q9UHF4; Q6UWA9; Q96SH8; EMBL; AF184971; AAF01320.1.
(21) Brevican (BCAN, BEHAB, Genbank accession no. AF229053)
   Gary S.C., et al Gene 256, 139-147, 2000; Clark H.F., et al Genome Res. 13, 2265-2270, 2003; Strausberg R.L., et al Proc. Natl. Acad. Sci. U.S.A. 99, 16899-16903, 2002; US2003186372 (Claim 11); US2003186373 (Claim 11); US2003119131 (Claim 1; Fig 52); US2003119122 (Claim 1; Fig 52); US2003119126 (Claim 1); US2003119121 (Claim 1; Fig 52); US2003119129 (Claim 1); US2003119130 (Claim 1); US2003119128 (Claim 1; Fig 52); US2003119125 (Claim 1); WO2003016475 (Claim 1); WO200202634 (Claim 1);
(22) EphB2R (DRT, ERK, Hek5, EPHT3, Tyro5, Genbank accession no. NM_004442) Chan,J. and Watt, V.M., Oncogene 6 (6), 1057-1061 (1991) Oncogene 10 (5):897-905 (1995), Annu. Rev. Neurosci. 21:309-345 (1998), Int. Rev. Cytol. 196:177-244 (2000)); WO2003042661 (Claim 12); WO200053216 (Claim 1; Page 41); WO2004065576 (Claim 1); WO2004020583 (Claim 9); WO2003004529 (Page 128-132); WO200053216 (Claim 1; Page 42);
   Cross-references: MIM:600997; NP_004433.2; NMR_004442_1
(23) ASLG659 (B7h, Genbank accession no. AX092328)
   US20040101899 (Claim 2); WO2003104399 (Claim 11); WO2004000221 (Fig 3); US2003165504 (Claim 1); US2003124140 (Example 2); US2003065143 (Fig 60); WO2002102235 (Claim 13; Page 299); US2003091580 (Example 2); WO200210187 (Claim 6; Fig 10); WO200194641 (Claim 12; Fig 7b); WO200202624 (Claim 13; Fig 1A-1B); US2002034749 (Claim 54; Page 45-46); WO200206317 (Example 2; Page 320-321, Claim 34; Page 321-322); WO200271928 (Page 468-469); WO200202587 (Example 1; Fig 1); WO200140269 (Example 3; Pages 190-192); WO200036107 (Example 2; Page 205-207); WO2004053079 (Claim 12); WO2003004989 (Claim 1); WO200271928 (Page 233-234, 452-453); WO 0116318;
(24) PSCA (Prostate stem cell antigen precursor, Genbank accession no. AJ297436)
   Reiter R.E., et al Proc. Natl. Acad. Sci. U.S.A. 95, 1735-1740, 1998; Gu Z., et al Oncogene 19, 1288-1296, 2000; Biochem. Biophys. Res. Commun. (2000) 275(3):783-788; WO2004022709; EP1394274 (Example 11); US2004018553 (Claim 17); WO2003008537 (Claim 1); WO200281646 (Claim 1; Page 164); WO2003003906 (Claim 10; Page 288); WO200140309 (Example 1; Fig 17); US2001055751 (Example 1; Fig 1b); WO200032752 (Claim 18; Fig 1); WO9851805 (Claim 17; Page 97); WO9851824 (Claim 10; Page 94); WO9840403 (Claim 2; Fig 1B);
   Accession: 043653; EMBL; AF043498; AAC39607.1.
(25) GEDA (Genbank accession No. AY260763);
   AAP14954 lipoma HMGIC fusion-partner-like protein /pid=AAP14954.1 - Homo sapiens Species: Homo sapiens (human)
   WO2003054152 (Claim 20); WO2003000842 (Claim 1); WO2003023013 (Example 3, Claim 20); US2003194704 (Claim 45);
   Cross-references: GI:30102449; AAP14954.1; AY260763_1
(26) BAFF-R (B cell -activating factor receptor, BLyS receptor 3, BR3, Genbank accession No. AF116456); BAFF receptor /pid=NP_443177.1 - Homo sapiens Thompson, J.S., et al Science 293 (5537), 2108-2111 (2001); WO2004058309;
   WO2004011611; WO2003045422 (Example; Page 32-33); WO2003014294 (Claim 35; Fig 6B); WO2003035846 (Claim 70; Page 615-616); WO200294852 (Col 136-137); WO200238766 (Claim 3; Page 133); WO200224909 (Example 3; Fig 3);
   Cross-references: MIM:606269; NP_443177.1; NM_052945_1; AF132600
(27) CD22 (B-cell receptor CD22-B isoform, BL-CAM, Lyb-8, Lyb8, SIGLEC-2, FLJ22814, Genbank accession No. AK026467);
   Wilson et al (1991) J. Exp. Med. 173:137-146; WO2003072036 (Claim 1; Fig 1);
   Cross-references: MIM:107266; NP_001762.1; NM_001771_1
(28) CD79a (CD79A, CD79α, immunoglobulin-associated alpha, a B cell-specific protein that covalently interacts with Ig beta (CD79B) and forms a complex on the surface with Ig M molecules, transduces a signal involved in B-cell differentiation), pI: 4.84, MW: 25028 TM: 2 [P] Gene Chromosome: 19q13.2, Genbank accession No. NP_001774.10) WO2003088808, US20030228319; WO2003062401 (claim 9); US2002150573 (claim 4, pages 13-14); WO9958658 (claim 13, Fig 16); WO9207574 (Fig 1); US5644033; Ha et al (1992) J. Immunol. 148(5):1526-1531; Mueller et al (1992) Eur. J. Biochem. 22:1621-1625; Hashimoto et al (1994) Immunogenetics 40(4):287-295; Preud'homme et al (1992) Clin. Exp. Immunol. 90(1):141-146; Yu et al (1992) J. Immunol. 148(2) 633-637; Sakaguchi et al (1988) EMBO J. 7(11):3457-3464;
(29) CXCR5 (Burkitt's lymphoma receptor 1, a G protein-coupled receptor that is activated by the CXCL13 chemokine, functions in lymphocyte migration and humoral defense, plays a role in HIV-2 infection and perhaps development of AIDS, lymphoma, myeloma, and leukemia); 372 aa, pI: 8.54 MW: 41959 TM: 7 [P] Gene Chromosome: 11q23.3, Genbank accession No. NP_001707.1)
   WO2004040000; WO2004015426; US2003105292 (Example 2); US6555339 (Example 2); WO200261087 (Fig 1); WO200157188 (Claim 20, page 269); WO200172830 (pages 12-13); WO200022129 (Example 1, pages 152-153, Example 2, pages 254-256); WO9928468 (claim 1, page 38); US5440021 (Example 2, col 49-52); WO9428931 (pages 56-58); WO9217497 (claim 7, Fig 5); Dobner et al (1992) Eur. J. Immunol. 22:2795-2799; Barella et al (1995) Biochem. J. 309:773-779;
(30) HLA-DOB (Beta subunit of MHC class II molecule (Ia antigen) that binds peptides and presents them to CD4+ T lymphocytes); 273 aa, pI: 6.56 MW: 30820 TM: 1 [P] Gene Chromosome: 6p21.3, Genbank accession No. NP_002111.1)
   Tonnelle et al (1985) EMBO J. 4(11):2839-2847; Jonsson et al (1989) Immunogenetics 29(6):411-413; Beck et al (1992) J. Mol. Biol. 228:433-441; Strausberg et al (2002) Proc. Natl. Acad. Sci USA 99:16899-16903; Servenius et al (1987) J. Biol. Chem. 262:8759-8766; Beck et al (1996) J. Mol. Biol. 255:1-13; Naruse et al (2002) Tissue Antigens 59:512-519; WO9958658 (claim 13, Fig 15); US6153408 (Col 35-38); US5976551 (col 168-170); US6011146 (col 145-146); Kasahara et al (1989) Immunogenetics 30(1):66-68; Larhammar et al (1985) J. Biol. Chem. 260(26): 14111-14119;
(31) P2X5 (Purinergic receptor P2X ligand-gated ion channel 5, an ion channel gated by extracellular ATP, may be involved in synaptic transmission and neurogenesis, deficiency may contribute to the pathophysiology of idiopathic detrusor instability); 422 aa), pI: 7.63, MW: 47206 TM: 1 [P] Gene Chromosome: 17p13.3, Genbank accession No. NP_002552.2) Le et al (1997) FEBS Lett. 418(1-2):195-199; WO2004047749; WO2003072035 (claim 10); Touchman et al (2000) Genome Res. 10:165-173; WO200222660 (claim 20); WO2003093444 (claim 1); WO2003087768 (claim 1); WO2003029277 (page 82);
(32) CD72 (B-cell differentiation antigen CD72, Lyb-2) PROTEIN SEQUENCE Full maeaity...tafrfpd (1..359; 359 aa), pI: 8.66, MW: 40225 TM: 1 [P] Gene Chromosome: 9p13.3, Genbank accession No. NP_001773.1)
   WO2004042346 (claim 65); WO2003026493 (pages 51-52, 57-58); WO200075655 (pages 105-106); Von Hoegen et al (1990) J. Immunol. 144(12):4870-4877; Strausberg et al (2002) Proc. Natl. Acad. Sci USA 99:16899-16903;
(33) LY64 (Lymphocyte antigen 64 (RP105), type I membrane protein of the leucine rich repeat (LRR) family, regulates B-cell activation and apoptosis, loss of function is associated with increased disease activity in patients with systemic lupus erythematosis); 661 aa, pI: 6.20, MW: 74147 TM: 1 [P] Gene Chromosome: 5q12, Genbank accession No. NP_005573.1)
   US2002193567; WO9707198 (claim 11, pages 39-42); Miura et al (1996) Genomics 38(3):299-304; Miura et al (1998) Blood 92:2815-2822; WO2003083047; WO9744452 (claim 8, pages 57-61); WO200012130 (pages 24-26);
(34) FcRH1 (Fc receptor-like protein 1, a putative receptor for the immunoglobulin Fc domain that contains C2 type Ig-like and ITAM domains, may have a role in B-lymphocyte differentiation); 429 aa, pI: 5.28, MW: 46925 TM: 1 [P] Gene Chromosome: 1q21-1q22, Genbank accession No. NP_443170.1)
   WO2003077836; WO200138490 (claim 6, Fig 18E-1-18-E-2); Davis et al (2001) Proc. Natl. Acad. Sci USA 98(17):9772-9777; WO2003089624 (claim 8); EP1347046 (claim 1); WO2003089624 (claim 7);
(35) IRTA2 (Immunoglobulin superfamily receptor translocation associated 2, a putative immunoreceptor with possible roles in B cell development and lymphomagenesis; deregulation of the gene by translocation occurs in some B cell malignancies); 977 aa, pI: 6.88 MW: 106468 TM: 1 [P] Gene Chromosome: 1q21, Genbank accession No. Human:AF343662, AF343663, AF343664, AF343665, AF369794, AF397453, AK090423, AK090475, AL834187, AY358085; Mouse:AK089756, AY158090, AY506558; NP_112571.1
   WO2003024392 (claim 2, Fig 97); Nakayama et al (2000) Biochem. Biophys. Res. Commun. 277(1):124-127; WO2003077836; WO200138490 (claim 3, Fig 18B-1-18B-2);
(36) TENB2 (TMEFF2, tomoregulin, TPEF, HPP1, TR, putative transmembrane proteoglycan, related to the EGF/heregulin family of growth factors and follistatin); 374 aa, NCBI Accession: AAD55776, AAF91397, AAG49451, NCBI RefSeq: NP_057276; NCBI Gene: 23671; OMIM: 605734; SwissProt Q9UIK5; Genbank accession No. AF179274; AY358907, CAF85723, CQ782436
   WO2004074320 (SEQ ID NO 810); JP2004113151 (SEQ ID NOS 2, 4, 8); WO2003042661 (SEQ ID NO 580); WO2003009814 (SEQ ID NO 411); EP1295944 (pages 69-70); WO200230268 (page 329); WO200190304 (SEQ ID NO 2706); US2004249130; US2004022727; WO2004063355; US2004197325; US2003232350; US2004005563; US2003124579; Horie et al (2000) Genomics 67:146-152; Uchida et al (1999) Biochem. Biophys. Res. Commun. 266:593-602; Liang et al (2000) Cancer Res. 60:4907-12; Glynne-Jones et al (2001) Int J Cancer. Oct 15;94(2):178-84;
(37) PMEL17 (silver homolog; SILV; D12S53E; PMEL17; (SI); (SIL); ME20; gp100) BC001414; BT007202; M32295; M77348; NM_006928; McGlinchey, R.P. et al (2009) Proc. Natl. Acad. Sci. U.S.A. 106 (33), 13731-13736; Kummer, M.P. et al (2009) J. Biol. Chem. 284 (4), 2296-2306;
(38) TMEFF1 (transmembrane protein with EGF-like and two follistatin-like domains 1; Tomoregulin-1; H7365; C9orf2; C9ORF2; U19878; X83961) NM_080655; NM_003692; Harms, P.W. (2003) Genes Dev. 17 (21), 2624-2629; Gery, S. et al (2003) Oncogene 22 (18):2723-2727;
(39) GDNF-Ra1 (GDNF family receptor alpha 1; GFRA1; GDNFR; GDNFRA; RETL1; TRNR1; RET1L; GDNFR-alpha1; GFR-ALPHA-1; U95847; BC014962; NM_145793) NM_005264; Kim, M.H. et al (2009) Mol. Cell. Biol. 29 (8), 2264-2277; Treanor, J.J. et al (1996) Nature 382 (6586):80-83;
(40) Ly6E (lymphocyte antigen 6 complex, locus E; Ly67,RIG-E,SCA-2,TSA-1) NP_002337.1; NM_002346.2; de Nooij-van Dalen, A.G. et al (2003) Int. J. Cancer 103 (6), 768-774; Zammit, D.J. et al (2002) Mol. Cell. Biol. 22 (3):946-952;
(41) TMEM46 (shisa homolog 2 (Xenopus laevis); SHISA2) NP_001007539.1; NM_001007538.1; Furushima, K. et al (2007) Dev. Biol. 306 (2), 480-492; Clark, H.F. et al (2003) Genome Res. 13 (10):2265-2270;
(42) Ly6G6D (lymphocyte antigen 6 complex, locus G6D; Ly6-D, MEGT1) NP_067079.2; NM_021246.2; Mallya, M. et al (2002) Genomics 80 (1):113-123; Ribas, G. et al (1999) J. Immunol. 163 (1):278-287;
(43) LGR5 (leucine-rich repeat-containing G protein-coupled receptor 5; GPR49, GPR67) NP_003658.1; NM_003667.2; Salanti, G. et al (2009) Am. J. Epidemiol. 170 (5):537-545; Yamamoto, Y. et al (2003) Hepatology 37 (3):528-533;
(44) RET (ret proto-oncogene; MEN2A; HSCR1; MEN2B; MTC1; (PTC); CDHF12; Hs.168114; RET51; RET-ELE1) NP_066124.1; NM_020975.4; Tsukamoto, H. et al (2009) Cancer Sci. 100 (10):1895-1901; Narita, N. et al (2009) Oncogene 28 (34):3058-3068;
(45) LY6K (lymphocyte antigen 6 complex, locus K; LY6K; HSJ001348; FLJ35226) NP_059997.3; NM_017527.3; Ishikawa, N. et al (2007) Cancer Res. 67 (24):11601-11611; de Nooij-van Dalen, A.G. et al (2003) Int. J. Cancer 103 (6):768-774;
(46) GPR19 (G protein-coupled receptor 19; Mm.4787) NP_006134.1; NM_006143.2; Montpetit, A. and Sinnett, D. (1999) Hum. Genet. 105 (1-2):162-164; O'Dowd, B.F. et al (1996) FEBS Lett. 394 (3):325-329;
(47) GPR54 (KISSI receptor; KISS1R; GPR54; HOT7T175; AXOR12) NP_115940.2; NM_032551.4; Navenot, J.M. et al (2009) Mol. Pharmacol. 75 (6):1300-1306; Hata, K. et al (2009) Anticancer Res. 29 (2):617-623;
(48) ASPHD1 (aspartate beta-hydroxylase domain containing 1; LOC253982) NP_859069.2; NM_181718.3; Gerhard, D.S. et al (2004) Genome Res. 14 (10B):2121-2127;
(49) Tyrosinase (TYR; OCAIA; OCA1A; tyrosinase; SHEP3) NP_000363. 1; NM_000372.4; Bishop, D.T. et al (2009) Nat. Genet. 41 (8):920-925; Nan, H. et al (2009) Int. J. Cancer 125 (4):909-917;
(50) TMEM118 (ring finger protein, transmembrane 2; RNFT2; FLJ14627) NP_001103373.1; NM_001109903.1; Clark, H.F. et al (2003) Genome Res. 13 (10):2265-2270; Scherer, S.E. et al (2006) Nature 440 (7082):346-351
(51) GPR172A (G protein-coupled receptor 172A; GPCR41; FLJ11856; D15Ertd747e) NP_078807.1; NM_024531.3; Ericsson, T.A. et al (2003) Proc. Natl. Acad. Sci. U.S.A. 100 (11):6759-6764; Takeda, S. et al (2002) FEBS Lett. 520 (1-3):97-101.

The parent antibody may also be a fusion protein comprising an albumin-binding peptide (ABP) sequence (Dennis et al. (2002) "Albumin Binding As A General Strategy For Improving The Pharmacokinetics Of Proteins" J Biol Chem. 277:35035-35043; WO 01/45746). Antibodies of the invention include fusion proteins with ABP sequences taught by: (i) Dennis et al (2002) J Biol Chem. 277:35035-35043 at Tables III and IV, page 35038; (ii) US 20040001827 at [0076]; and (iii) WO 01/45746 at pages 12-13.

To prepare a cysteine engineered antibody by mutagenesis, DNA encoding an amino acid sequence variant of the starting polypeptide is prepared by a variety of methods known in the art. These methods include, but are not limited to, preparation by site-directed (or oligonucleotide-mediated) mutagenesis, PCR mutagenesis, and cassette mutagenesis of an earlier prepared DNA encoding the polypeptide. Variants of recombinant antibodies may be constructed also by restriction fragment manipulation or by overlap extension PCR with synthetic oligonucleotides. Mutagenic primers encode the cysteine codon replacement(s). Standard mutagenesis techniques can be employed to generate DNA encoding such mutant cysteine engineered antibodies. General guidance can be found in Sambrook et al Molecular Cloning, A Laboratory Manual, Cold Spring Harbor Laboratory Press, Cold Spring Harbor, N.Y., 1989; and Ausubel et al Current Protocols in Molecular Biology, Greene Publishing and Wiley-Interscience, New York, N.Y., 1993.

Site-directed mutagenesis is one method for preparing substitution variants, i.e. mutant proteins (Carter (1985) et al Nucleic Acids Res. 13:4431-4443; Ho et al (1989) Gene (Amst.) 77:51-59; and Kunkel et al (1987) Proc. Natl. Acad. Sci. USA 82:488). Starting DNA is altered by first hybridizing an oligonucleotide encoding the desired mutation to a single strand of such starting DNA. After hybridization, a DNA polymerase is used to synthesize an entire second strand, using the hybridized oligonucleotide as a primer, and using the single strand of the starting DNA as a template. Thus, the oligonucleotide encoding the desired mutation is incorporated in the resulting double-stranded DNA. Site-directed mutagenesis may be carried out within the gene expressing the protein to be mutagenized in an expression plasmid and the resulting plasmid may be sequenced to confirm the introduction of the desired cysteine replacement mutations (Liu et al (1998) J. Biol. Chem. 273:20252-20260). Site-directed mutagenesis protocols and formats are widely available, e.g. QuikChange® Multi Site-Directed Mutagenesis Kit (Stratagene, La Jolla, CA).

PCR mutagenesis is also suitable for making amino acid sequence variants of the starting polypeptide. See Higuchi, (1990) in PCR Protocols, pp.177-183, Academic Press; Ito et al (1991) Gene 102:67-70; Bernhard et al (1994) Bioconjugate Chem., 5:126-132; and Vallette et al (1989) Nuc. Acids Res., 17:723-733. Briefly, when small amounts of template DNA are used as starting material in a PCR, primers that differ slightly in sequence from the corresponding region in a template DNA can be used to generate relatively large quantities of a specific DNA fragment that differs from the template sequence only at the positions where the primers differ from the template.

Another method for preparing variants, cassette mutagenesis, is based on the technique described by Wells et al (1985) Gene, 34:315-323. The starting material is the plasmid (or other vector) comprising the starting polypeptide DNA to be mutated. The codon(s) in the starting DNA to be mutated are identified. There must be a unique restriction endonuclease site on each side of the identified mutation site(s). If no such restriction sites exist, they may be generated using the above described oligonucleotide-mediated mutagenesis method to introduce them at appropriate locations in the starting polypeptide DNA. The plasmid DNA is cut at these sites to linearize it. A double-stranded oligonucleotide encoding the sequence of the DNA between the restriction sites but containing the desired mutation(s) is synthesized using standard procedures, wherein the two strands of the oligonucleotide are synthesized separately and then hybridized together using standard techniques. This double-stranded oligonucleotide is referred to as the cassette. This cassette is designed to have 5' and 3' ends that are compatible with the ends of the linearized plasmid, such that it can be directly ligated to the plasmid. This plasmid now contains the mutated DNA sequence. Mutant DNA containing the encoded cysteine replacements can be confirmed by DNA sequencing.

Single mutations are also generated by oligonucleotide directed mutagenesis using double stranded plasmid DNA as template by PCR based mutagenesis (Sambrook and Russel, (2001) Molecular Cloning: A Laboratory Manual, 3rd edition; Zoller et al (1983) Methods Enzymol. 100:468-500; Zoller, M.J. and Smith, M. (1982) Nucl. Acids Res. 10:6487-6500).

### ENGINEERING AND THIOL REACTIVITY OF 4D5 ANTI-HER2 THIOFABS

Cysteine was introduced into each position of the heavy chain and light chain of the anti-HER2 hu4D5Fabv8 Fab fragment antibody (US 5821337; Carter et al (1992) Proc. Natl. Acad. Sci., 89:4285-4289). All 440 of the heavy chain mutants and light chain mutants were prepared according to the methods described herein. Thiol reactivity was measured according to the PHESELECTOR assay. Heavy chain sequences are numbered by the Sequential numbering system. Light chain sequences follow the Kabat numbering system. In the light chain, both Kabat and Sequential numbering denotes same numbers.

Heavy chain hu4D5Fabv8 mutants were selected for efficient binding to HER2 receptor protein and thiol reactivity with the biotinylation reagent, Biotin-PEO-maleimide (US 7521541). Certain heavy chain mutants had limited or compromised binding to HER2 ECD because this is an important residue for antigen binding (HER2), located in CDRs in the variable region of the antibody-Fab. Some of the residues located in the constant domain of the Fabs also resulted in poor HER2 binding because these residues may contribute to structure and folding of Fab, thus resulting in poor 4D5-Fab display on M13-page (Junutula, J.R. et al. (2008) J. Immunol Methods, 332:41-52). Heavy chain hu4D5Fabv8 mutants with poor HER2 ECD binding included cysteine mutations at positions 1, 21, 31, 33-36, 38, 48-50, 59, 87, 95, 101, 104, 129, 131, 132, 136, 153, 155, 159, 166, 169, 170, 172, 197, 198, 202, 215, 219. Wild type cysteine variants 22, 96, 147, 203, 223 were measured. Other heavy chain mutants had limited thiol reactivity with the biotinylation reagent.

The A121C free cysteine amino acid introduced by the cysteine engineering methods described herein and SEQ ID NO. 32, is designated by the Sequential number system. This residue at the beginning of the constant domain is also A118C as designated by the EU numbering system, or A114C by the Kabat system. The mutants as conjugated in the antibody-drug conjugates described herein (Figures 5a and 5b, Table 3, and Example 6 use the A118C EU system for designating antibodies comprising SEQ ID NO. 32.

The free cysteine amino acid residue is in the center with flanking residues in the sequences in the middle column of Table 1. The substituted amino acid and position in the heavy chain are designated in the left column. Heavy chain hu4D5Fabv8 mutants SEQ ID NOS: 1-49 of Table 1 have retained HER2 binding and thiol reactivity values of about 0.8 or higher, excluding wild type cysteine variants. Antibodies with SEQ ID NOS: 1-49 (Table 1) have demonstrated thiol reactivity and may be useful to form covalent attachments with a capture label, a detection label, a drug moiety, or a solid support. The heavy chain mutants of Table 1 may be conjugated as ThioFabs or ThioMabs for example as antibody-drug conjugates.

**Table 1 Efficient binding, thiol-reactive heavy chain hu4D5Fabv8 mutants**

| | | |
|---|---|---|
| HC-L4C | EVQCVESGG | SEQ ID NO: 1 |
| HC-G8C | QLVESCGGLVQ | SEQ ID NO: 2 |
| HC-G10C | VESGGCLVQPG | SEQ ID NO: 3 |
| HC-L20C | GGSLRCSCAAS | SEQ ID NO: 4 |
| HC-A23C | LRLSCCASGFN | SEQ ID NO: 5 |
| HC-G26C | SCAASCFNIKD | SEQ ID NO: 6 |
| HC-F27C | CAASGCNIKDT | SEQ ID NO: 7 |
| HC-T32C | FNIKDCYIHWV | SEQ ID NO: 8 |
| HC-Q39C | IHWVRCAPGKG | SEQ ID NO: 9 |
| HC-P41C | WVRQACGKGLE | SEQ ID NO: 10 |
| HC-K43C | RQAPGCGLEWV | SEQ ID NO: 11 |
| HC-G44C | QAPGKCLEWVA | SEQ ID NO: 12 |
| HC-W47C | GKGLECVARIY | SEQ ID NO: 13 |
| HC-S63C | TRYADCVKGRF | SEQ ID NO: 14 |
| HC-F68C | SVKGRCTISAD | SEQ ID NO: 15 |
| HC-D73C | FTISACTSKNT | SEQ ID NO: 16 |
| HC-K76C | SADTSCNTAYL | SEQ ID NO: 17 |
| HC-T78C | DTSKNCAYLQM | SEQ ID NO: 18 |
| HC-Y80C | SKNTACLQMNS | SEQ ID NO: 19 |
| HC-L81C | KNTAYCQMNSL | SEQ ID NO: 20 |
| HC-Q82C | NTAYLCMNSLR | SEQ ID NO: 21 |
| HC-L86C | LQMNSCRAEDT | SEQ ID NO: 22 |
| HC-A88C | MNSLRCEDTAV | SEQ ID NO: 23 |
| HC-D90C | SLRAECTAVYY | SEQ ID NO: 24 |
| HC-V93C | AEDTACYYCSR | SEQ ID NO: 25 |
| HC-Y94C | EDTAVCYCSRW | SEQ ID NO: 26 |
| HC-R98C | VYYCSCWGGDG | SEQ ID NO: 27 |
| HC-G100C | YCSRWCGDGFY | SEQ ID NO: 28 |
| HC-D108C | GFYAMCYWGQG | SEQ ID NO: 29 |
| HC-G113C | DYWGQCTLVTV | SEQ ID NO: 30 |
| HC-T117C | QGTLVCVSSAS | SEQ ID NO: 31 |
| HC-A121C | VTVSSCSTKGP | SEQ ID NO: 32 |
| HC-G125C | SASTKCPSVFP | SEQ ID NO: 33 |
| HC-G141C | KSTSGCTAALG | SEQ ID NO: 34 |
| HC-P154C | VKDYFCEPVTV | SEQ ID NO: 35 |
| HC-N162C | VTVSWCSGALT | SEQ ID NO: 36 |
| HC-S163C | TVSWNCGALTS | SEQ ID NO: 37 |
| HC-G164C | VSWNSCALTSG | SEQ ID NO: 38 |
| HC-S168C | SGALTCGVHTF | SEQ ID NO: 39 |
| HC-F173C | SGVHTCPAVLQ | SEQ ID NO: 40 |
| HC-T190C | LSSVVCVPSSS | SEQ ID NO: 41 |
| HC-S194C | VTVPSCSLGTQ | SEQ ID NO: 42 |
| HC-T200C | SLGTQCYICNV | SEQ ID NO: 43 |
| HC-V205C | TYICNCNHKPS | SEQ ID NO: 44 |
| HC-N211C | NHKPSCTKVDK | SEQ ID NO: 45 |
| HC-T212C | HKPSNCKVDKK | SEQ ID NO: 46 |
| HC-V214C | PSNTKCDKKVE | SEQ ID NO: 47 |
| HC-K217C | TKVDKCVEPKS | SEQ ID NO: 48 |
| HC-T226C | KSCDKCH | SEQ ID NO: 49 |

Light chain hu4D5Fabv8 mutants were selected for efficient binding to HER2 receptor protein and thiol reactivity with the biotinylation reagent, Biotin-PEO-maleimide (US 7521541). Certain light chain mutants had limited or compromised binding to HER2 because this is an important residue for antigen binding (HER2), located in CDRs in the variable region of the antibody-Fab. Some of the residues located in constant domain of Fab also resulted in poor HER2 binding because these residues may contribute to structure and folding of Fab, thus resulting in poor 4D5-Fab display on M13-page (Junutula, J.R. et al. (2008) J. Immunol Methods, 332:41-52). Light chain hu4D5Fabv8 mutants with poor binding to HER2 included cysteine mutants at positions 4, 29-32, 35, 36, 50, 82, 86, 89-91, 113, 115, 117, 120, 126, 128, 139, 141, 146, 148, 179, 186, 192,202. Wild type cysteine variants 23, 134, 194, 214 were measured. Other light chain mutants had limited thiol reactivity with the biotinylation reagent.

The V205C free cysteine amino acid residue introduced by the cysteine engineering methods described herein and SEQ ID NO. 96, is designated by the Kabat and Sequential number systems. The V205C mutants as conjugated in the antibody-drug conjugates described herein (Figures 5a and 5b, Table 3, and Example 6 comprise SEQ ID NO. 96.

The free cysteine amino acid residue is in the center with flanking residues in the sequences in the middle column of Table 2. The substituted amino acid and position in the light chain are designated in the left column. Light chain hu4D5Fabv8 mutants SEQ ID NOS: 50-98 of Table 2 have retained HER2 binding and thiol reactivity values of about 0.8 or higher, excluding wild type cysteine variants. Antibodies with SEQ ID NOS: 50-98 (Table 2) have demonstrated thiol reactivity and may be useful to form covalent attachments with a capture label, a detection label, a drug moiety, or a solid support. The light chain mutants of Table 2 may be conjugated as ThioFabs or ThioMabs for example as antibody-drug conjugates.

**Table 2 Efficient binding, thiol-reactive light chain hu4D5Fabv8 mutants**

| | | |
|---|---|---|
| LC-S9C | MTQSPCSLSAS | SEQ ID NO: 50 |
| LC-L46C | GKAPKCLIYSA | SEQ ID NO: 51 |
| LC-Y49C | PKLLICSASFL | SEQ ID NO: 52 |
| LC-F53C | IYSASCLYSGV | SEQ ID NO: 53 |
| LC-T72C | SGTDFCLTISS | SEQ ID NO: 54 |
| LC-L73C | GTDFTCTISSL | SEQ ID NO: 55 |
| LC-T74C | TDFTLCISSLQ | SEQ ID NO: 56 |
| LC-I75C | DFTLTCSSLQP | SEQ ID NO: 57 |
| LC-S77C | TLTISCLQPED | SEQ ID NO: 58 |
| LC-Q79C | TISSLCPEDFA | SEQ ID NO: 59 |
| LC-P80C | ISSLQCEDFAT | SEQ ID NO: 60 |
| LC-Y92C | YCQQHCTTPPT | SEQ ID NO: 61 |
| LC-P95C | QHYTTCPTFGQ | SEQ ID NO: 62 |
| LC-G99C | TPPTFCQGTKV | SEQ ID NO: 63 |
| LC-G101C | PTFGQCTKVEI | SEQ ID NO: 64 |
| LC-K103C | FGQGTCVEIKR | SEQ ID NO: 65 |
| LC-E105C | QGTKVCIKRTV | SEQ ID NO: 66 |
| LC-V110C | EIKRTCAAPSV | SEQ ID NO: 67 |
| LC-A112C | KRTVACPSVFI | SEQ ID NO: 68 |
| LC-S114C | TVAAPCVFIFP | SEQ ID NO: 69 |
| LC-F116C | AAPSVCIFPPS | SEQ ID NO: 70 |
| LC-F118C | PSVFICPPSDE | SEQ ID NO: 71 |
| LC-S121C | FIFPPCDEQLK | SEQ ID NO: 72 |
| LC-L125C | PSDEQCKSGTA | SEQ ID NO: 73 |
| LC-S127C | DEQLKCGTASV | SEQ ID NO: 74 |
| LC-T129C | QLKSGCASVVC | SEQ ID NO: 75 |
| LC-A130C | LKSGTCSVVCL | SEQ ID NO: 76 |
| LC-S131C | KSGTACVVCLL | SEQ ID NO: 77 |
| LC-N137C | VVCLLCNFYPR | SEQ ID NO: 78 |
| LC-N138C | VCLLNCFYPRE | SEQ ID NO: 79 |
| LC-Y140C | LLNNFCPREAK | SEQ ID NO: 80 |
| LC-R142C | NNFYPCEAKVQ | SEQ ID NO: 81 |
| LC-A144C | FYPRECKVQWK | SEQ ID NO: 82 |
| LC-Q147C | REAKVCWKVDN | SEQ ID NO: 83 |
| LC-K149C | AKVQWCVDNAL | SEQ ID NO: 84 |
| LC-D151C | VQWKVCNALQS | SEQ ID NO: 85 |
| LC-Q155C | VDNALCSGNSQ | SEQ ID NO: 86 |
| LC-Q160C | QSGNSCESVTE | SEQ ID NO: 87 |
| LC-A184C | LTLSKCDYEKH | SEQ ID NO: 88 |
| LC-D185C | TLSKACYEKHK | SEQ ID NO: 89 |
| LC-K188C | KADYECHKVYA | SEQ ID NO: 90 |
| LC-T197C | YACEVCHQGLS | SEQ ID NO: 91 |
| LC-G200C | EVTHQCLSSPV | SEQ ID NO: 92 |
| LC-L201C | VTHQGCSSPVT | SEQ ID NO: 93 |
| LC-S203C | HQGLSCPVTKS | SEQ ID NO: 94 |
| LC-P204C | QGLSSCVTKSF | SEQ ID NO: 95 |
| LC-V205C | GLSSPCTKSFN | SEQ ID NO: 96 |
| LC-T206C | LSSPVCKSFNR | SEQ ID NO: 97 |
| LC-K207C | SSPVTCSFNRG | SEQ ID NO: 98 |

### PREPARATION OF CYSTEINE ENGINEERED ANTIBODIES FOR CONJUGATION

Under certain conditions, the cysteine engineered antibodies may be made reactive for conjugation with drug-linker intermediates of the invention by treatment with a reducing agent such as DTT (Cleland's reagent, dithiothreitol) or TCEP (tris(2-carboxyethyl)phosphine hydrochloride; Getz et al (1999) Anal. Biochem. Vol 273:73-80; Soltec Ventures, Beverly, MA). Full length, cysteine engineered monoclonal antibodies (ThioMabs) expressed in CHO cells (Gomez et al (2010) Biotechnology and Bioeng. 105(4):748-760; Gomez et al (2010) Biotechnol. Prog. 26:1438-1445) were reduced with about a 50 fold excess of DTT overnight at room temperature to reduce disulfide bonds which may form between the newly introduced cysteine residues and the cysteine present in the culture media. The reduced ThioMab was diluted and loaded onto HiTrap SP FF column in 10 mM sodium acetate, pH 5, and eluted with 50 mM Tris-Cl, pH 7.5 containing 150 mM sodium chloride. Disulfide bonds were reestablished between cysteine residues present in the parent Mab by carrying out reoxidation with 15X DHAA at room temperature for 3 hrs in 50 mM Tris-Cl, pH 7.5. Other oxidants, i.e. oxidizing agents, and oxidizing conditions, which are known in the art may be used. Ambient air oxidation is also effective. This mild, partial reoxidation step forms intrachain disulfides efficiently with high fidelity. An approximate 1.5 fold excess of drug-linker intermediate, e.g. **5, 8, 14, 18,** was added, mixed, and let stand for about an hour at room temperature to effect conjugation and form the ThioMab antibody-drug conjugate. The conjugation mixture was loaded and eluted through a HiTrap SP FF column to remove excess drug-linker intermediate and other impurities.

### N-METHYL ALANINYL MAYTANSINOL DRUG MOIETY

The drug moiety (D) of the antibody-drug conjugates (ADC) of the invention is a maytansinoid derivative which has a cytotoxic or cytostatic effect through any mechanism of action including microtubulin inhibition, mitosis inhibition, topoisomerase inhibition, or DNA intercalation.

Maytansine compounds inhibit cell proliferation by inhibiting the formation of microtubules during mitosis through inhibition of polymerization of the microtubulin protein, tubulin (Remillard et al (1975) Science 189:1002-1005). Maytansine and maytansinoids are highly cytotoxic but their clinical use in cancer therapy has been greatly limited by their severe systemic side-effects primarily attributed to their poor selectivity for tumors. Clinical trials with maytansine had been discontinued due to serious adverse effects on the central nervous system and gastrointestinal system (Issel et al (1978) Can. Treatment. Rev. 5:199-207).

Maytansinoid drug moieties are attractive drug moieties in antibody-drug conjugates because they are: (i) relatively accessible to prepare by fermentation or chemical modification, derivatization of fermentation products, (ii) amenable to derivatization with functional groups suitable for conjugation through the non-disulfide linkers to antibodies, (iii) stable in plasma, and (iv) effective against a variety of tumor cell lines (US 2005/0169933; WO 2005/037992; US 5208020).

Maytansinoid derivatives include N-methyl alaninyl maytansinol compounds prepared from natural sources according to known methods, produced using genetic engineering techniques (Yu et al (2002) Proc. Nat. Acad. Sci. (USA) 99:7968-7973; US 6790954; US 7192750). First isolated from an African shrub (US 3896111), maytansines are most efficiently obtained by microbial fermentation (US 4151042; US 6790954; US 7192750; US 7432088) which yields C-3 ester ansamitocin mixture. Reduction of the C-3 esters yields maytansinol (US 7411063; 6333410). The C-3 hydroxyl of maytansinol may be selectively derivatized (US 7301019; US 7276497; US 7473796; US 7598375), including alaninyl esters (US 4137230; US 4260608; US 5208020; and Chem. Pharm. Bull. (1984) 12:3441).

The N-methyl alaninyl maytansinol drug moiety (D) of the antibody-drug conjugates (ADC) of Formula I have the structure: 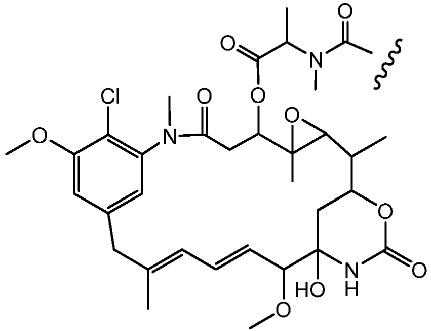 where the wavy line indicates the attachment site to the linker (L).

All stereoisomers of the maytansinoid drug moiety are contemplated for the compounds of the invention, i.e. any combination of R and S configurations at the chiral carbons of D. In one embodiment, the maytansinoid drug moiety (D) will have the following stereochemistry:

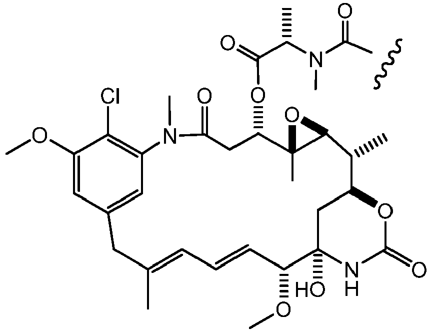

The N-methyl alaninyl maytansinol drug moiety (D) of the antibody-drug conjugates and drug-linker intermediates of Formula I of the invention comprise an amide-alkyl or amide-ethyleneoxy linkage to the N-methyl alaninyl group and not an alkylthio-maleimido linkage attached to the N-methyl alaninyl group of the maytansinoid drug moiety, such as the mpeo-DM1 or mcc-DM1 linkage, exemplified in US 2005/0276812 at pages 29 and 32, respectively.

### N-METHYL ALANINYL MAYTANSINOL DRUG-LINKER INTERMEDIATES

The invention includes N-methyl alaninyl maytansinol drug-linker intermediate compounds where the linker is attached to the C-3 alaninyl maytansinoid moiety and having Formula I: 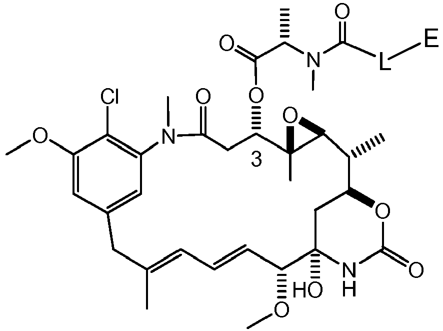
L is 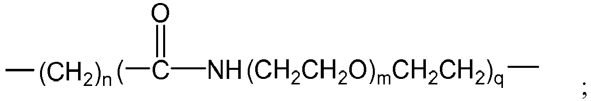
E is 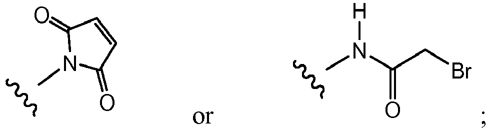
n is 2, 3, 4, 5, or 6;
m is 2, 3 or 4; and
q is 0 or 1.

Linker (L) is a bifunctional or multifunctional moiety which can be used to link one or more maytansinol drug moieties (D) and an antibody unit (Ab) to form antibody-drug conjugates (ADC) of Formula Ia or Ib. Antibody-drug conjugates (ADC) can be conveniently prepared using a Linker having reactive functionality for binding to the Drug and to the Antibody. A cysteine thiol of a cysteine engineered antibody (Ab) can form a bond with an electrophilic functional group (E) of a linker reagent or drug-linker intermediate. Bromoacetamido and maleimide functional groups are known to be reactive with thiols, including cysteine thiols of proteins (Schelte et al (2000) Bioconjugate Chem. 11:118-123; Alley et al (2008) Bioconjugate Chem. 19:759-765). In one aspect, a Linker has a reactive site which has an electrophilic group that is reactive to a nucleophilic cysteine present on an antibody. The cysteine thiol of the antibody is reactive with an electrophilic group on a Linker and forms a covalent bond to a Linker. Useful electrophilic groups include, but are not limited to, maleimide and haloacetamide groups. Cysteine engineered antibodies react with linker reagents or drug-linker intermediates, with electrophilic functional groups such as maleimide or α-halo carbonyl, according to the conjugation method at page 766 of Klussman, et al (2004), Bioconjugate Chemistry 15(4):765-773, and according to the protocol of Example 6.

Examples of thiol-reactive, electrophilic functional groups include, but are not limited to, maleimide, α-haloacetyl, activated esters such as succinimide esters, 4-nitrophenyl esters, pentafluorophenyl esters, tetrafluorophenyl esters, anhydrides, acid chlorides, sulfonyl chlorides, isocyanates, isothiocyanates, vinyl sulfone, chlorotriazine, 2-halopyridyl, chloropyrimidine, and enamide.

The linker moiety of a drug-linker intermediate may be an alkyl of 2, 3, 4, 5, or 6 methylene groups where L is -(CH₂)ₙ- and n is 2, 3, 4, 5, or 6. An exemplary embodiment is the mal-mc-ala-May drug-linker intermediate **5** of Figure 1 and Example 1 where E is maleimide, and n is 5. Acylation of 2,5-dioxopyrrolidin-1-yl 6-(2,5-dioxo-2,5-dihydro-1H-pyrrol-1-yl)hexanoate **1** with (S)-2-(methylamino)propanoic acid (N-methyl *S-*alanine) **2** gives (S)-2-(6-(2,5-dioxo-2,5-dihydro-1H-pyrrol-1-yl)-N-methylhexanamido)propanoic acid **3.** Coupling of maytansinol **4** at the 3-hydroxyl with **3** gives mal-hex-ala-May **5,** ready for conjugation with an antibody to give the antibody-drug conjugate, Ab-hex-mc-ala-May.

Another exemplary embodiment is bra-hex-ala-May **8** of Figure 2 and Example 2 where E is 2-bromoacetamide, and n is 5. Acylation of 2,5-dioxopyrrolidin-1-yl 6-(2-bromoacetamido)hexanoate **6** with (S)-2-(methylamino)propanoic acid (N-methyl *S*-alanine) **2** gave (S)-2-(6-(2-bromoacetamido)-N-methylhexanamido)propanoic acid 7. Coupling of maytansinol **4** at the 3-hydroxyl with 7 gives bra-hex-ala-May **8,** ready for conjugation with an antibody to give the antibody-drug conjugate, Ab-acet-hex-ala-May.

The linker moiety L of a drug-linker intermediate may comprise ethyleneoxy (PEG) units where L is 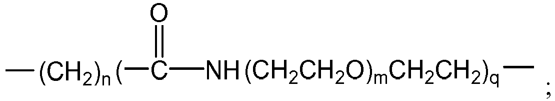 n is 2, 3, 4, 5, or 6; m is 2, 3 or 4; and q is 1. An exemplary embodiment is the mal-PEG3-ala-May drug-linker intermediate **14** of Figure 3 and Example 3 where E is maleimide, n is 4, and m is 3. The mono N-hydroxysuccinimide (NHS) ester, 6-(2,5-dioxopyrrolidin-1-yloxy)-6-oxohexanoic acid **9,** formed from adipic acid, is reacted with 2,2'-(2,2'-oxybis(ethane-2,1-diyl)bis(oxy))diethanamine to give 1-amino-13-oxo-3,6,9-trioxa-12-azaoctadecan-18-oic acid **10.** The maleimide of **10** is formed with methyl 2,5-dioxo-2,5-dihydro-1H-pyrrole-1-carboxylate to give 1-(2,5-dioxo-2,5-dihydro-1H-pyrrol-1-yl)-13-oxo-3,6,9-trioxa-12-azaoctadecan-18-oic acid **11.** The NHS ester of **11** is formed with N-hydroxysuccinimide and DCC to give 2,5-dioxopyrrolidin-1-yl 1-(2,5-dioxo-2,5-dihydro-1H-pyrrol-1-yl)-13-oxo-3,6,9-trioxa-12-azaoctadecan-18-oate **12.** Amidation of **12** with (S)-2-(methylamino)propanoic acid (N-methyl *S*-alanine) **2** gave (S)-1-(2,5-dioxo-2,5-dihydro-1H-pyrrol-1-yl)-19,20-dimethyl-13,18-dioxo-3,6,9-trioxa-12,19-diazahenicosan-21-oic acid **13.** Coupling at the 3-hydroxyl of maytansinol **4** with **13** gives mal-PEG3-ala-May drug-linker intermediate **14,** ready for conjugation with an antibody to give the antibody-drug conjugate, Ab-mal-PEG3-ala-May.

Alternatively, maytansinol **4** is reacted with N,N-diisopropylethylamine, zinc triflate, and (*S*)-3,4-dimethyloxazolidine-2,5-dione **2a** in THF/DMF to give 3-(*S*-(N-methylalaninyl)maytansinol **4a** (Figure 1b). Reagent **2a** is prepared from (S)-2-(methylamino)propanoic acid (N-methyl *S*-alanine) **2** and phosphorus trichloride in DCM. 3-(*S*-(N-methylalaninyl)maytansinol **4a** is coupled with 1-(2,5-dioxo-2,5-dihydro-1H-pyrrol-1-yl)-13-oxo-3,6,9-trioxa-12-azaoctadecan-18-oic acid **11,** N-(3-dimethylaminopropyl)-N'-ethylcarbodiimide hydrochloride, and N,N-diisopropylethylamine to provide mal-PEG3-ala-May **14** (Figure 3).

Another exemplary embodiment is bra-PEG3-ala-May 1**8** of Figure 4 and Example 4 where E is 2-bromoacetamide, n is 4, and m is 3. 1-Amino-13-oxo-3,6,9-trioxa-12-azaoctadecan-18-oic acid **10** is acylated with bromoacetyl bromide to give 1-bromo-2,16-dioxo-6,9,12-trioxa-3,15-diazahenicosan-21-oic acid **15.** The NHS ester of **15** is formed with N-hydroxysuccinimide and DCC in DCM to give 2,5-dioxopyrrolidin-1-yl 1-bromo-2,16-dioxo-6,9,12-trioxa-3,15-diazahenicosan-21-oate **16.** Amidation of **16** with (S)-2-(methylamino)propanoic acid (N-methyl *S*-alanine) **2** gave linker reagent, (S)-1-bromo-22,23-dimethyl-2,16,21-trioxo-6,9,12-trioxa-3,15,22-triazatetracosan-24-oic acid **17.** Coupling at the 3-hydroxyl of maytansinol **4** with **17** gives bra-PEG3-ala-May drug-linker intermediate **18,** ready for conjugation with an antibody to give the antibody-drug conjugate, Ab-acet-PEG3-ala-May.

Alternatively, 3-(S-(N-methylalaninyl)maytansinol **4a** (Figure 1b) was coupled with 1-bromo-2,16-dioxo-6,9,12-trioxa-3,15-diazahenicosan-21-oic acid **15,** N-(3-dimethylaminopropyl)-N'-ethylcarbodiimide hydrochloride, and N,N-diisopropylethylamine to provide bra-PEG3-ala-May **18** (Figure 4).

### ALANINYL MAYTANSINOID ANTIBODY-DRUG CONJUGATES

The antibody-drug conjugates of the invention are comprised of an N-methyl alaninyl maytansinol drug moiety covalently attached through a linker moiety to the reactive cysteine thiol group of an antibody.

An exemplary embodiment of an antibody-drug conjugate (ADC) compound comprises a cysteine engineered antibody (Ab), and an N-methyl alaninyl maytansinol drug moiety (D) wherein the antibody has one or more free cysteine amino acids, and the antibody is attached through the one or more free cysteine amino acids by a linker moiety (L) to D; the composition having the formula:

Ab-(L-D)ₚ

where p is 1, 2, 3, or 4. The number of drug moieties which may be conjugated via a thiol reactive linker moiety to an antibody molecule is limited by the number of cysteine residues which are introduced by the methods described herein. Exemplary ADC prepared from drug-linker intermediates of Formula I therefore comprise antibodies which have 1, 2, 3, or 4 engineered cysteine amino acids.

Exemplary embodiments of an alaninyl maytansinoid antibody-drug conjugate are Formula Ia where L comprises a maleimide moiety, and Formula Ib where L comprises an acetamidomethyl moiety. 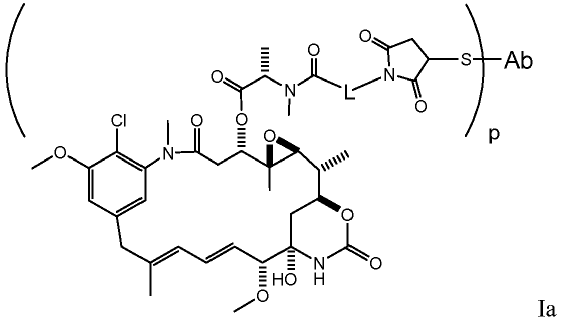 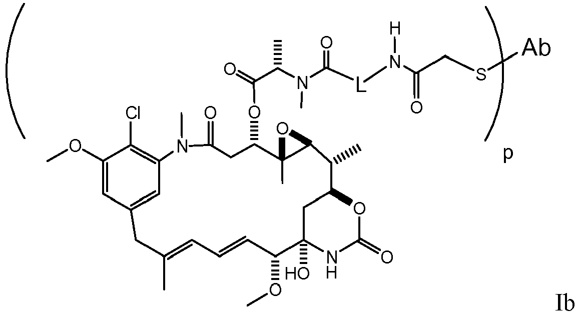
L is 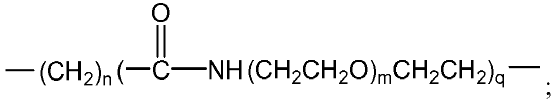
n is 2, 3, 4, 5, or 6;
m is 2, 3 or 4;
q is 0 or 1;
p is 1 to 4; and
Ab is an antibody.

Exemplary embodiments of Formula Ia alaninyl maytansinoid antibody-drug conjugates include Ab-mal-hex-ala-May: 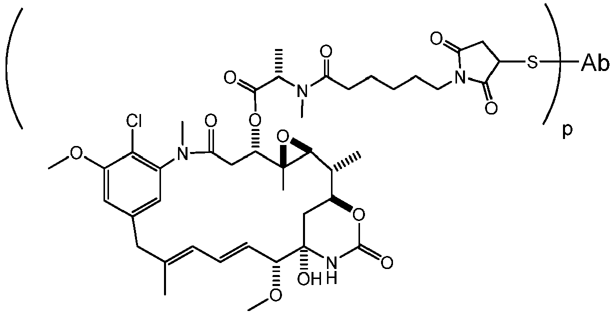 and Ab-mal-PEG3-ala-May: 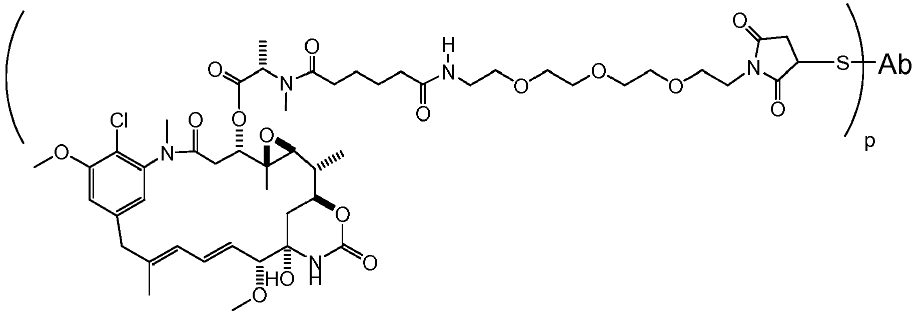 Exemplary embodiments of Formula Ib alaninyl maytansinoid antibody-drug conjugates include Ab-acet-hex-ala-May: 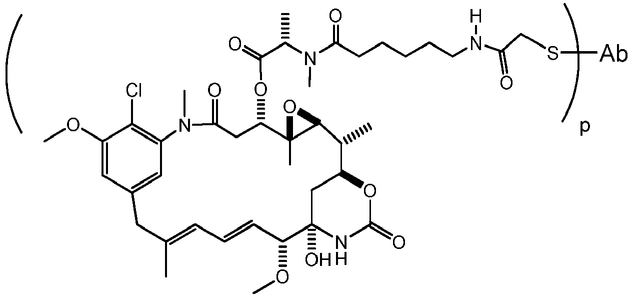 and Ab-acet-PEG3-ala-May:

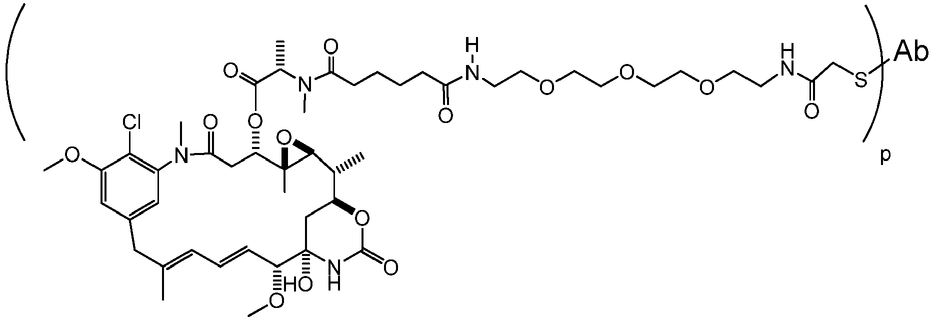

The ADC compounds of the invention include those with utility for anticancer activity. In particular, the compounds include a cysteine-engineered antibody conjugated, i.e. covalently attached by a linker, to a drug moiety, i.e. toxin. When the drug is not conjugated to an antibody, the drug has a cytotoxic or cytostatic effect. The biological activity of the drug moiety is thus modulated by conjugation to an antibody. The antibody-drug conjugates (ADC) of the invention selectively deliver an effective dose of a cytotoxic agent to tumor tissue whereby greater selectivity, i.e. a lower efficacious dose, may be achieved.

### IN VITRO CELL PROLIFERATION ASSAYS

Generally, the cytotoxic or cytostatic activity of an antibody-drug conjugate (ADC) is measured by: exposing mammalian cells having receptor proteins, e.g. HER2, to the antibody of the ADC in a cell culture medium; culturing the cells for a period from about 6 hours to about 5 days; and measuring cell viability. Cell-based *in vitro* assays were used to measure viability (proliferation), cytotoxicity, and induction of apoptosis (caspase activation) of the ADC of the invention.

The *in vitro* potency of antibody-drug conjugates was measured by a cell proliferation assay (Example 7). The CellTiter-Glo^{®} Luminescent Cell Viability Assay is a commercially available (Promega Corp., Madison, WI), homogeneous assay method based on the recombinant expression of *Coleoptera* luciferase (US Patent Nos. 5583024; 5674713 and 5700670). This cell proliferation assay determines the number of viable cells in culture based on quantitation of the ATP present, an indicator of metabolically active cells (Crouch et al (1993) J. Immunol. Meth. 160:81-88; US 6602677). The CellTiter-Glo^{®} Assay was conducted in 96 well format, making it amenable to automated high-throughput screening (HTS) (Cree et al (1995) AntiCancer Drugs 6:398-404). The homogeneous assay procedure involves adding the single reagent (CellTiter-Glo^{®} Reagent) directly to cells cultured in serum-supplemented medium. Cell washing, removal of medium and multiple pipetting steps are not required. The system detects as few as 15 cells/well in a 384-well format in 10 minutes after adding reagent and mixing. The cells may be treated continuously with ADC, or they may be treated and separated from ADC. Generally, cells treated briefly, i.e. 3 hours, showed the same potency effects as continuously treated cells.

The homogeneous "add-mix-measure" format results in cell lysis and generation of a luminescent signal proportional to the amount of ATP present. The amount of ATP is directly proportional to the number of cells present in culture. The CellTiter-Glo^{®} Assay generates a "glow-type" luminescent signal, produced by the luciferase reaction, which has a half-life generally greater than five hours, depending on cell type and medium used. Viable cells are reflected in relative luminescence units (RLU). The substrate, Beetle Luciferin, is oxidatively decarboxylated by recombinant firefly luciferase with concomitant conversion of ATP to AMP and generation of photons.

*In vitro,* SK-BR-3 cell proliferation assay results at 5 days versus multiple concentrations of test samples are shown in Table 3.

All ThioMab antibody-drug conjugates showed similar *in vitro* potency (IC₅₀ values with 10-12 ng/ml) regardless of linker and these conjugates displayed about two fold less potency compared to TMAb-mcc-DM1 (IC₅₀: 5ng/ml) (Table 3). The decreased *in vitro* potency with ThioMab conjugates is proportional to conventional ADC when the drug loading of ThioMab ADC (1.8 DAR) is compared with the drug loading to conventional ADC (3.5 DAR). The control unconjugated trastuzumab or thio-trastuzumab variants showed very little or no activity up to 10,000ng/ml concentration tested in the assay.

**Table 3**

| Test sample | Drug/antibody (DAR) | IC₅₀ (ng/ml) |
|---|---|---|
| trastuzumab | none | > 10,000 |
| thio-trastuzumab LC V205C | none | > 10,000 |
| thio-trastuzumab HC A118C | none | > 10,000 |
| (4) HC-A118C-Thio-TMAb-mal-PEG3-ala-May | 1.8 | 10.7 |
| (9) HC A118C-Thio-TMAb-mal-hex-ala-May | 1.9 | 12.1 |
| (6) TMAb-mcc-DM1 (T-DM1) | 3.4 | 5.0 |
| (5) LC-V205C-Thio-TMAb-mal-hex-ala-May | 1.8 | 11.8 |
| (3) LC-V205C-Thio-TMAb-mal-PEG3-ala-May | 1.8 | 10.9 |

Trastuzumab-mcc-DM1 (trastuzumab emtansine, TMAb-mcc-DM1, T-DM1) is an antibody-drug conjugate (CAS Reg. No. 139504-50-0), and has the structure: 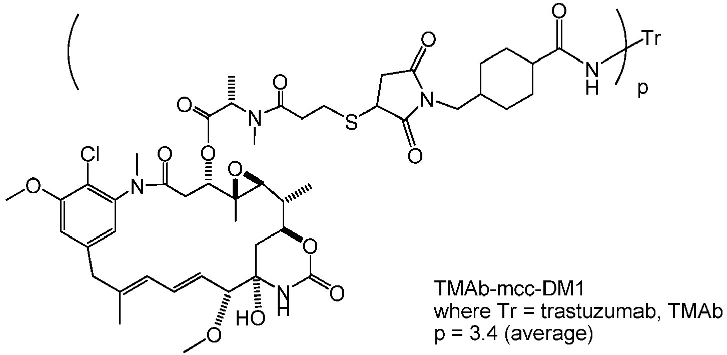 where Tr is trastuzumab linked through a maleimidomethyl) cyclohexane-1-carboxylate linker moiety (mcc) formed from linker reagent succinimidyl 4-(N-maleimidomethyl) cyclohexane-1-carboxylate (SMCC, Pierce Biotechnology, Inc) to the thiol group of thiol maytansinoid drug moiety DM1 (US 5208020; US 6441163). The drug to antibody ratio or drug loading is represented by p in the above structure of trastuzumab-mcc-DM1, and ranges in integer values from 1 to about 8. The drug loading value p is 1 to 8. Trastuzumab-mcc-DM1 includes all mixtures of variously loaded and attached antibody-drug conjugates where 1, 2, 3, 4, 5, 6, 7, and 8 drug moieties are covalently attached to the antibody trastuzumab (US 7097840; US 2005/0276812; US 2005/0166993).

### IN VIVO EFFICACY

The *in vivo* efficacy of TMAb-mcc-DM1 (6) and various ThioMab conjugates with mpeo (2) hex (5) and PEG3 (3), (4), (7), (8) linkers covalently attached to DM1 (Example 6) were tested in the MMTV-HER2 Fo5 trastuzumab-resistant mammary tumor model (Example 8) and these results were presented in the Figures 5a, 5b and 6. MMTV-HER2 Fo5 tumor explants were implanted into the No. 2/3 mammary fat pad of CRL nu/nu mice. When tumors reached an average volume of 180 mm³, mice were randomized and then given a single intravenous dose of DM1 conjugates (at 10 mg/kg) on Study Day 0.

Figures 5a and 5b show plots of the in vivo fitted tumor volume change over time in MMTV-HER2 Fo5 transgenic mammary tumors inoculated into the mammary fat pad of CRL nu/nu mice after dosing with: (1) Vehicle (ADC buffer), (2) LC-V205C-Thio-TMAb-mpeo-DM1, (3) LC-V205C-Thio-TMAb-mal-PEG3-ala-May, (4) HC-A118C-Thio-TMAb-mal-PEG3-ala-May, (5) LC-V205C Thio-TMAb-mal-hex-ala-May, (6) TMAb-mcc-DM1 (trastuzumab-mcc-DM1, T-DM1), (7) LC-V205C-Thio anti-gD5B6-mal-PEG3-ala-May, (8) LC-V205C-Thio anti-gD5B6-mal-hex-ala-May (Examples 6, 8). All antibody drug conjugates (single doses) were dosed intravenously at 10 mg/kg. Anti-gD5B6 is a control antibody and its corresponding antigen does not express in Fo5 tumor tissues.

The (6) TMAb-mcc-DM showed partial inhibition in tumor growth at 10 mg/kg, which equates to a DM1 dose of 560 µg/m². All ThioTMAb-maytansinoid conjugates had comparable activity at the same antibody concentration despite having lower drug load. The (3) LC-V205C-Thio-TMAb-mal-PEG3-ala-May showed slightly improved activity on a mg/kg dose comparison over (2) LC-V205C Thio-TMAb-mpeo-DM1 (Figure 5a). Even though ThioMab conjugates with hex and PEG3 linkers showed about 2-fold less potency *in vitro* due to lower drug load, they showed comparable *in vivo* efficacy to TMAb-mcc-DM1 indicating that the hex and PEG3 alaninyl maytansinol linker-drug moieties may have improved pharmacokinetic properties in antibody-drug conjugates.

Figure 6 shows a plot of the in vivo fitted tumor volume change over time in MMTV-HER2 Fo5 transgenic mammary tumors inoculated into the mammary fat pad of CRL nu/nu mice after dosing with: (1) Vehicle: Histidine Buffer #8: 20mM Histidine Acetate, pH 5.5, 240mM Sucrose, 0.02% PS 20, (4) HC-A118C-Thio-TMAb-mal-PEG3-ala-May, 5 mg/kg dose, 150 µg/m² drug exposure, (4) HC-A118C-Thio-TMAb-mal-PEG3-ala-May, 10 mg/kg dose, 300 µg/m² drug exposure, (10) HC-A118C Thio anti-gD5B6-bra-PEG3-ala-May, 5 mg/kg dose, 120 µg/m² drug exposure, (10) HC-A118C Thio anti-gD5B6-bra-PEG3-ala-May, 10 mg/kg dose, 240 µg/m² drug exposure, (11) HC-A118C Thio TMAb-bra-PEG3-ala-May, 5 mg/kg dose, 115 µg/m² drug exposure, (11) HC-A118C Thio TMAb-bra-PEG3-ala-May, 10 mg/kg dose, 230 µg/m² drug exposure, (12) HC-A118C, LC-V205C Thio-TMAb-mal-PEG3-ala-May, 5 gm/kg dose, 320 µg/m² drug exposure, (12) HC-A118C, LC-V205C Thio-TMAb-mal-PEG3-ala-May, 10 gm/kg dose, 640 µg/m² drug exposure. All antibody-drug conjugates (single doses) were dosed once intravenously at the start of the study. A group of nine animals were dosed with an antibody-drug conjugate at a particular dose.

Vehicle and negative control (10) anti-gD5B6 antibody-drug conjugate showed no tumor growth inhibitory effects. Anti-HER2 antibody-drug conjugates (4), (11), and (12) showed dose-dependent and maytansinoid drug-exposure dependent tumor growth inhibition. The antibody of (12) HC-A118C, LC-V205C Thio-TMAb-mal-PEG3-ala-May is a double-mutant, with cysteines introduced at A118 of the heavy chain and V205 of the light chain. Drug-linker intermediate, mal-PEG3-ala-May **14** (Example 3) conjugated to the double mutant HC-A118C, LC-V205C Thio-TMAb near-quantitatively at drug/antibody ratio of 3.9. The nine test group animals dosed at 10 mg/kg of conjugate (12) showed two partial responses.

### ADMINISTRATION OF ANTIBODY-DRUG CONJUGATES

The antibody-drug conjugates (ADC) of the invention may be administered by any route appropriate to the condition to be treated. The ADC will typically be administered parenterally, i.e. infusion, subcutaneous, intramuscular, intravenous, intradermal, intrathecal and epidural.

### PHARMACEUTICAL FORMULATIONS

Pharmaceutical formulations of therapeutic antibody-drug conjugates (ADC) of the invention are typically prepared for parenteral administration, i.e. bolus, intravenous, intratumor injection with a pharmaceutically acceptable parenteral vehicle and in a unit dosage injectable form. An antibody-drug conjugate (ADC) having the desired degree of purity is optionally mixed with pharmaceutically acceptable diluents, carriers, excipients or stabilizers (Remington's Pharmaceutical Sciences (1980) 16th edition, Osol, A. Ed.), in the form of a lyophilized formulation or an aqueous solution.

### ANTIBODY-DRUG CONJUGATE TREATMENTS

It is contemplated that the antibody-drug conjugates (ADC) of the present invention may be used to treat various diseases or disorders, e.g. characterized by the overexpression of a tumor antigen. Exemplary conditions or hyperproliferative disorders include benign or malignant tumors; leukemia and lymphoid malignancies. Others include neuronal, glial, astrocytal, hypothalamic, glandular, macrophagal, epithelial, stromal, blastocoelic, inflammatory, angiogenic and immunologic, including autoimmune, disorders.

Generally, the disease or disorder to be treated is a hyperproliferative disease such as cancer. Examples of cancer to be treated herein include, but are not limited to, carcinoma, lymphoma, blastoma, sarcoma, and leukemia or lymphoid malignancies. More particular examples of such cancers include squamous cell cancer (e.g. epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, as well as head and neck cancer.

Autoimmune diseases for which the ADC compounds may be used in treatment include rheumatologic disorders (such as, for example, rheumatoid arthritis, Sjögren's syndrome, scleroderma, lupus such as systemic lupus erythematosus (SLE) and lupus nephritis, polymyositis/dermatomyositis, cryoglobulinemia, anti-phospholipid antibody syndrome, and psoriatic arthritis), osteoarthritis, autoimmune gastrointestinal and liver disorders (such as, for example, inflammatory bowel diseases (e.g., ulcerative colitis and Crohn's disease), autoimmune gastritis and pernicious anemia, autoimmune hepatitis, primary biliary cirrhosis, primary sclerosing cholangitis, and celiac disease), vasculitis (such as, for example, ANCA-associated vasculitis, including Churg-Strauss vasculitis, Wegener's granulomatosis, and polyarteriitis), autoimmune neurological disorders (such as, for example, multiple sclerosis, opsoclonus myoclonus syndrome, myasthenia gravis, neuromyelitis optica, Parkinson's disease, Alzheimer's disease, and autoimmune polyneuropathies), renal disorders (such as, for example, glomerulonephritis, Goodpasture's syndrome, and Berger's disease), autoimmune dermatologic disorders (such as, for example, psoriasis, urticaria, hives, pemphigus vulgaris, bullous pemphigoid, and cutaneous lupus erythematosus), hematologic disorders (such as, for example, thrombocytopenic purpura, thrombotic thrombocytopenic purpura, post-transfusion purpura, and autoimmune hemolytic anemia), atherosclerosis, uveitis, autoimmune hearing diseases (such as, for example, inner ear disease and hearing loss), Behcet's disease, Raynaud's syndrome, organ transplant, and autoimmune endocrine disorders (such as, for example, diabetic-related autoimmune diseases such as insulin-dependent diabetes mellitus (IDDM), Addison's disease, and autoimmune thyroid disease (e.g., Graves' disease and thyroiditis)). More preferred such diseases include, for example, rheumatoid arthritis, ulcerative colitis, ANCA-associated vasculitis, lupus, multiple sclerosis, Sjögren's syndrome, Graves' disease, IDDM, pernicious anemia, thyroiditis, and glomerulonephritis.

For the prevention or treatment of disease, the appropriate dosage of an ADC will depend on the type of disease to be treated, as defined above, the severity and course of the disease, whether the molecule is administered for preventive or therapeutic purposes, previous therapy, the patient's clinical history and response to the antibody, and the discretion of the attending physician. The molecule is suitably administered to the patient at one time or over a series of treatments. Depending on the type and severity of the disease, about 1 µg/kg to 15 mg/kg (e.g. 0.1-20 mg/kg) of molecule is an initial candidate dosage for administration to the patient, whether, for example, by one or more separate administrations, or by continuous infusion. A typical daily dosage might range from about 1 µg/kg to 100 mg/kg or more, depending on the factors mentioned above. An exemplary dosage of ADC to be administered to a patient is in the range of about 0.1 to about 10 mg/kg of patient weight

### ARTICLES OF MANUFACTURE

In another embodiment of the invention, an article of manufacture, or "kit", containing materials useful for the treatment of the disorders described above is provided. The article of manufacture comprises a container and a label or package insert on or associated with the container. Suitable containers include, for example, bottles, vials, syringes, blister pack, etc. The containers may be formed from a variety of materials such as glass or plastic. The container holds an antibody-drug conjugate (ADC) composition which is effective for treating the condition and may have a sterile access port (for example the container may be an intravenous solution bag or a vial having a stopper pierceable by a hypodermic injection needle). At least one active agent in the composition is an ADC. The label or package insert indicates that the composition is used for treating the condition of choice, such as cancer. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

### EXAMPLES

### Example 1 Synthesis of mal-hex-ala-May 5

Acylation of 2,5-dioxopyrrolidin-1-yl 6-(2,5-dioxo-2,5-dihydro-1H-pyrrol-1-yl)hexanoate **1** with (S)-2-(methylamino)propanoic acid (N-methyl *S*-alanine) **2** gives (S)-2-(6-(2,5-dioxo-2,5-dihydro-1H-pyrrol-1-yl)-N-methylhexanamido)propanoic acid **3** (Figure 1a). Coupling of maytansinol **4** at the 3-hydroxyl with **3** gives mal-hex-ala-May **5.** MS [M+H]⁺ 843.5. ¹H NMR (400 MHz, CD₃OD): δ 7.11 (s, 1H), 6.76 (s, 2H), 6.72 - 6.65 (m, 2H), 6.60 (dd, *J* = 14.7, 11.4 Hz, 1H), 5.69 (dd, *J* = 14.9, 9.1 Hz, 1H), 5.49 (q, *J* = 6.7 Hz, 1H), 4.65 (dd, *J* = 11.9, 2.1 Hz, 1H), 4.19 (td, *J* = 10.3, 4.1 Hz, 1H), 3.97 (s, 3H), 3.62 - 3.55 (m, 2H), 3.41 - 3.34 (m, 5H), 3.23 (d, *J=* 12.7 Hz, 1H), 3.20 (s, 3H), 2.94 (d, *J=* 9.6 Hz, 1H), 2.84 (s, 3H), 2.72 - 2.62 (m, 1H), 2.56 - 2.45 (m, 1H), 2.33 - 2.23 (m, 1H), 2.14 (dd, *J* = 14.1, 1.8 Hz, 1H), 1.68 (s, 3H), 1.65 - 1.42 (m, 7H), 1.29 (d, *J* = 6.8 Hz, 3H), 1.28 - 1.25 (m, 2H), 1.23 (d, *J* = 6.3 Hz, 3H), 0.84 (s, 3H).

### Example 2 Synthesis of bra-hex-ala-May 8

Acylation of 2,5-dioxopyrrolidin-1-yl 6-(2-bromoacetamido)hexanoate **6** with (S)-2-(methylamino)propanoic acid (N-methyl S-alanine) **2** gave (S)-2-(6-(2-bromoacetamido)-N-methylhexanamido)propanoic acid **7** (Figure 2). Coupling of maytansinol **4** at the 3-hydroxyl with **7** gives bra-hex-ala-May **8.**

### Example 3 Synthesis of mal-PEG3-ala-May 14

To a solution of 2,2'-(2,2'-oxybis(ethane-2,1-diyl)bis(oxy))diethanamine (Chem-Impex, 5.00 g, 0.0260 mol) in THF (525 mL) was added 4-dimethylaminopyridine (320 mg, 0.0026 mol). To this was added a solution of di-tert-butyldicarbonate (5.68 g, 0.0260 mol) in THF (100 mL) over a period of 1 h, using an addition funnel, all at room temp. (Figure 3). The reaction initially became cloudy, but then cleared. Following McReynolds K.D. et al., (2002), Bioorg Med Chem, 10:625, the reaction was stirred an additional 2 h and then concentrated and purified by ISCO (0-20% MeOH/DCM) to provide tert-butyl 2-(2-(2-(2-aminoethoxy)ethoxy)ethoxy)ethylcarbamate as a light yellow oil (2.70 g, 36%). MS [M+H]⁺ 293.3. ¹H NMR (400 MHz, CDCl₃): δ 5.79 (s, 1H), 3.69 - 3.57 (m, 8H), 3.56 - 3.47 (m, 4H), 3.32 - 3.23 (m, 2H), 2.84 *(t, J=* 5.1 Hz, 2H), 1.44 (s, 9H).

To a flask containing tert-butyl 2-(2-(2-(2-aminoethoxy)ethoxy)ethoxy)ethylcarbamate (1.219 g, 4.169 mmol) and hexanedioic acid (adipic acid, 3.046 g, 20.84 mmol) in tetrahydrofuran (100 mL) was added N,N'-dicyclohexylcarbodiimide (1.23 g, 5.98 mmol). The reaction was stirred at room temperature and became cloudy. After 2 h, the solution was cooled to 0 °C and the byproduct N,N'-dicyclohexylurea was removed by filtration. The reaction was concentrated onto silica gel and purified via ISCO (40 g column, 0-10% MeOH/DCM). Concentration provided 2,2-dimethyl-4,18-dioxo-3,8,11,14-tetraoxa-5,17-diazatricosan-23-oic acid **9** as a clear oil (1.28 g, 73%). MS [M+H]⁺ 421.4. ¹H NMR (400 MHz, CDCl₃): δ 10.55 (s, 1H), 6.65 (s, 1H), 5.27 (s, 1H), 3.67 - 3.62 (m, 8H), 3.60 - 3.52 (m, 4H), 3.48 - 3.41 (m, 2H), 3.35 - 3.23 (m, 2H), 2.35 (t, J = 6.3 Hz, 3H), 2.25 (t, J = 6.5 Hz, 2H), 1.44 (s, 9H).

To a vial containing 2,2-dimethyl-4,18-dioxo-3,8,11,14-tetraoxa-5,17-diazatricosan-23-oic acid **9** (682.3 mg, 0.001623 mol) was added 4 mL of 4 M hydrogen chloride in 1,4-dioxane. The reaction was stirred for 30 min and then concentrated. A saturated aqueous solution of sodium bicarbonate (5.1 mL) was added. The solution was cooled to 0 °C and stirred for 10 min, and then N-methoxycarbonylmaleimide (251.7 mg, 1.622 mmol) was added. The reaction was stirred for 20 min more at 0 °C, and then warmed to room temp. The solution was diluted with DMF, quenched with 5 drops of formic acid, filtered, and purified by rp-HPLC to provide 1-amino-13-oxo-3,6,9-trioxa-12-azaoctadecan-18-oic acid **10** as a clear oil (297 mg, 46%). MS [M+H]⁺ 401.3. ¹H NMR (400 MHz, CDCl₃): δ 8.66 (s, 1H), 6.56 (t, *J=* 4.8 Hz, 1H), 3.73 (t, *J* = 5.6 Hz, 2H), 3.66 - 3.60 (m, 9H), 3.56 (t, *J* = 5.0 Hz, 2H), 3.47 - 3.41 (m, 2H), 2.35 *(t, J=* 6.7 Hz, 2H), 2.24 *(t, J=* 6.9 Hz, 2H), 1.77 - 1.58 (m, 4H).

The maleimide of **10** is formed with methyl 2,5-dioxo-2,5-dihydro-1H-pyrrole-1-carboxylate to give 1-(2,5-dioxo-2,5-dihydro-1H-pyrrol-1-yl)-13-oxo-3,6,9-trioxa-12-azaoctadecan-18-oic acid **11** (Hermanson, G.T. "Bioconjugate Techniques", Second Edition, (2008) Academic Press, Elsevier). The NHS ester of **11** is formed with N-hydroxysuccinimide and DCC in DCM to give 2,5-dioxopyrrolidin-1-yl,1-(2,5-dioxo-2,5-dihydro-1H-pyrrol-1-yl)-13-oxo-3,6,9-trioxa-12-azaoctadecan-18-oate **12.** Amidation of **12** with (S)-2-(methylamino)propanoic acid (N-methyl *S*-alanine) **2** gave (S)-1-(2,5-dioxo-2,5-dihydro-1H-pyrrol-1-yl)-19,20-dimethyl-13,18-dioxo-3,6,9-trioxa-12,19-diazahenicosan-21-oic acid **13.** Coupling at the 3-hydroxyl of maytansinol **4** with **13** gives mal-PEG3-ala-May drug-linker intermediate **14**

Alternatively, to a solution of maytansinol **4** (50.0 mg, 0.0885 mmol) in DMF (1.12 mL, 14.5 mmol) and THF (380 µL, 4.6 mmol) was added N,N-diisopropylethylamine (62 µL, 0.35 mmol), zinc triflate (129 mg, 0.354 mmol), and (S)-3,4-dimethyloxazolidine-2,5-dione **2a** (80.0 mg, 0.619 mmol) (Figure 1b). The reaction was stirred for 24 h and ethyl acetate (2 mL) was added and then over 5 min, 2 mL of a saturated 1:1 sodium bicarbonate (aq)/sodium chloride (aq) solution was added. The solution was stirred for 30 min, and the salts were filtered and rinsed with ethyl acetate. The two phases were separated and the aqueous phase was extracted with 3 x 2 mL of ethyl acetate. The combined organic phases were concentrated to 0.25 mL. 2 mL of ethyl acetate was added, and the solution was again reduced to 0.25 mL. This dilution and concentration was done once more. Finally, ethyl acetate was added to give around 2 mL of solution and salts that had precipitated were filtered through a 0.45 micron syringe filter to give 3-(*S*-(N-methylalaninyl)maytansinol **4a** (Figure 1b).

To a solution of 3-(*S*-(N-methylalaninyl)maytansinol **4a** was added 1-(2,5-dioxo-2,5-dihydro-1H-pyrrol-1-yl)-13-oxo-3,6,9-trioxa-12-azaoctadecan-18-oic acid **11** (65.5 mg, 0.164 mmol), N-(3-Dimethylaminopropyl)-N'-ethylcarbodiimide hydrochloride (31.4 mg, 0.164 mmol), and N,N-Diisopropylethylamine (7.71 µL, 0.0442 mmol). The reaction was stirred for 2 hr, and the reaction was filtered and purified on RP-HPLC to provide mal-PEG3-ala-May **14** as a clear oil (48.8 mg, 53%). MS [M+H]⁺ 1032.7. ¹H NMR (400 MHz, CDCl₃): δ 6.83 (s, 1H), 6.71 (s, 2H), 6.70 - 6.64 (m, 2H), 6.47 - 6.37 (m, 2H), 6.27 (t, *J* = 4.8 Hz, 1H), 5.67 (dd, *J=* 15.3, 9.1 Hz, 1H), 5.35 (q, *J* = 6.7 Hz, 1H), 4.78 (dd, *J=* 12.0, 2.8 Hz, 1H), 4.29 *(t, J=* 10.8 Hz, 1H), 3.98 (s, 3H), 3.72 (t, *J* = 5.7 Hz, 2H), 3.67 - 3.56 (m, 12H), 3.54 - 3.48 (m, 3H), 3.44 - 3.38 (m, 2H), 3.36 (s, 3H), 3.19 (s, 3H), 3.11 (d, *J* = 12.7 Hz, 1H), 3.01 (d, *J=* 9.6 Hz, 1H), 2.84 (s, 3H), 2.60 (dd, *J=* 14.1, 12.4 Hz, 1H), 2.48 - 2.38 (m, 1H), 2.30 - 2.13 (m, 4H), 1.75 - 1.58 (m, 8H), 1.52 - 1.40 (m, 1H), 1.29 (t, *J=* 6.0 Hz, 6H), 1.22 (d, *J* = 12.9 Hz, 1H), 0.80 (s, 3H).

### Example 4 Synthesis of bra-PEG3-ala-May 18

To a vial containing 2,2-dimethyl-4,18-dioxo-3,8,11,14-tetraoxa-5,17-diazatricosan-23-oic acid **9** (321.8 mg, 0.7653 mmol) was added 1 mL methylene chloride and 1 mL trifluoroacetic acid. The reaction was stirred for 30 min and concentrated to give 1-Amino-13-oxo-3,6,9-trioxa-12-azaoctadecan-18-oic acid **10** (Figure 3).

N,N-dimethylformamide (7.65 mL) was added to the vial of **10** and the solution was cooled to 0 °C (Figure 4). Bromoacetyl bromide (73 µL, 0.842 mmol) was added followed by N,N-diisopropylethylamine (160 µL, 0.918 mmol). After stirring for 45 min, 2 mL of water with 0.1% formic acid was added to the solution and the product was purified by RP-HPLC to provide 1-bromo-2,16-dioxo-6,9,12-trioxa-3,15-diazahenicosan-21-oic acid **15** as a clear oil (89.6 mg, 27%). MS [M+H]⁺ 441.3. ¹H NMR (400 MHz, CDCl₃): δ 7.12 (s, 1H), 6.48 (s, 1H), 3.89 (s, 2H), 3.69 - 3.54 (m, 12H), 3.53 - 3.41 (m, 4H), 2.36 (t, *J* = 6.6 Hz, 2H), 2.24 *(t, J=* 6.8 Hz, 2H), 1.76 - 1.59 (m, 4H).

The NHS ester of **15** is formed with N-hydroxysuccinimide and DCC in DCM to give 2,5-dioxopyrrolidin-1-yl 1-bromo-2,16-dioxo-6,9,12-trioxa-3,15-diazahenicosan-21-oate **16.** Amidation of **16** with (S)-2-(methylamino)propanoic acid (N-methyl S-alanine) **2** gives linker reagent, (S)-1-bromo-22,23-dimethyl-2,16,21-trioxo-6,9,12-trioxa-3,15,22-triazatetracosan-24-oic acid **17.** Coupling at the 3-hydroxyl of maytansinol **4** with **17** gives bra-PEG3-ala-May drug-linker intermediate **18** (Figure 4).

Alternatively, to a solution of 3-(*S*-(N-methylalaninyl)maytansinol **4a** (Figure 1b) was added 1-bromo-2,16-dioxo-6,9,12-trioxa-3,15-diazahenicosan-21-oic acid **15** (72.2 mg, 0.164 mmol), N-(3-Dimethylaminopropyl)-N'-ethylcarbodiimide hydrochloride (31.4 mg, 0.164 mmol), and N,N-Diisopropylethylamine (7.71 µL, 0.0442 mmol) . The reaction was stirred for 2 h, and the reaction was filtered and purified on RP-HPLC to provide bra-PEG3-ala-May **18** as a clear oil (53.3 mg, 56%). MS [M+H]⁺ 1073.0. ¹H NMR (400 MHz, CDCl₃): δ 7.10 (s, 1H), 6.83 (s, 1H), 6.66 (s, 1H), 6.64 (s, 1H), 6.48 - 6.37 (m, 2H), 6.32 (t, *J* = 4.8 Hz, 1H), 5.68 (dd, *J* = 15.3, 9.1 Hz, 1H), 5.31 (q, *J* = 6.5 Hz, 1H), 4.79 (dd, *J* = 12.0, 2.8 Hz, 1H), 4.29 (t, *J* = 11.2 Hz, 1H), 3.98 (s, 3H), 3.87 (s, 2H), 3.65 - 3.62 (m, 9H), 3.61 - 3.58 (m, 3H), 3.54 (t, *J* = 5.2 Hz, 2H), 3.50 - 3.47 (m, 3H), 3.42 (dd, *J* = 9.8, 4.9 Hz, 2H), 3.36 (s, 3H), 3.19 (s, 3H), 3.12 (d, *J* = 12.7 Hz, 1H), 3.00 (d, *J* = 9.6 Hz, 1H), 2.85 (s, 3H), 2.60 (dd, *J* = 14.1, 12.4 Hz, 1H), 2.49 - 2.12 (m, 6H), 1.69 - 1.57 (m, 7H), 1.46 (qd, *J* = 12.8, 6.4 Hz, 1H), 1.31 (d, *J* = 6.9 Hz, 3H), 1.28 (d, *J* = 6.3 Hz, 3H), 1.23 (d, *J* = 13.0 Hz, 1H), 0.80 (s, 3H).

### Example 5 Preparation of cysteine engineered antibodies for conjugation by reduction and reoxidation

Light chain amino acids are numbered according to Kabat (Kabat et al., Sequence of proteins of immunological interest, (1991) 5th Ed., US Dept of Health and Human Service, National Institutes of Health, Bethesda, MD). Heavy chain amino acids are numbered according to the EU numbering system (Edelman et al (1969) Proc. Natl. Acad of Sciences 63(1):78-85), except where noted as the Kabat system. Single letter amino acid abbreviations are used.

Full length, cysteine engineered monoclonal antibodies (ThioMabs) expressed in CHO cells bear cysteine adducts (cystines) or glutathionylated on the engineered cysteines due to cell culture conditions. To liberate the reactive thiol groups of the engineered cysteines, the ThioMabs are dissolved in 500 mM sodium borate and 500 mM sodium chloride at about pH 8.0 and reduced with about a 50-100 fold excess of 1 mM TCEP (tris(2-carboxyethyl)phosphine hydrochloride (Getz et al (1999) Anal. Biochem. Vol 273:73-80; Soltec Ventures, Beverly, MA) for about 1-2 hrs at 37 °C. Alternatively, DTT can be used as reducing agent. The formation of inter-chain disulfide bonds was monitored either by non-reducing SDS-PAGE or by denaturing reverse phase HPLC PLRP column chromatography. The reduced ThioMab is diluted and loaded onto a HiTrap S column in 10 mM sodium acetate, pH 5, and eluted with PBS containing 0.3M sodium chloride. The eluted reduced ThioMab is treated with 2 mM dehydroascorbic acid (dhAA) at pH 7 for 3 hours, or 2 mM aqueous copper sulfate (CuSO₄) at room temperature overnight. Ambient air oxidation may also be effective. The buffer is exchanged by elution over Sephadex G25 resin and eluted with PBS with 1mM DTPA. The thiol/Ab value is checked by determining the reduced antibody concentration from the absorbance at 280 nm of the solution and the thiol concentration by reaction with DTNB (Aldrich, Milwaukee, WI) and determination of the absorbance at 412 nm.

Liquid chromatography/Mass Spectrometric Analysis was performed on a TSQ Quantum Triple quadrupole mass spectrometer with extended mass range (Thermo Electron, San Jose California). Samples were chromatographed on a PRLP-S, 1000 A, microbore column (50mm × 2.1mm, Polymer Laboratories, Shropshire, UK) heated to 75 °C. A linear gradient from 30-40% B (solvent A: 0.05% TFA in water, solvent B: 0.04% TFA in acetonitrile) was used and the eluent was directly ionized using the electrospray source. Data were collected by the Xcalibur data system and deconvolution was performed using ProMass (Novatia, LLC, New Jersey). Prior to LC/MS analysis, antibodies or drug conjugates (50 micrograms) were treated with PNGase F (2 units/ml; PROzyme, San Leandro, CA) for 2 hours at 37 °C to remove N-linked carbohydrates.

Hydrophobic Interaction Chromatography (HIC) samples were injected onto a Butyl HIC NPR column (2.5 micron particle size, 4.6 mm × 3.5 cm) (Tosoh Bioscience) and eluted with a linear gradient from 0 to 70% B at 0.8 ml/min (A: 1.5 M ammonium sulfate in 50 mM potassium phosphate, pH 7, B: 50 mM potassium phosphate pH 7, 20% isopropanol). An Agilent 1100 series HPLC system equipped with a multi wavelength detector and Chemstation software was used to resolve and quantitate antibody species with different ratios of drugs per antibody.

### Example 6 Conjugation of drug-linker intermediates to antibodies

After the reduction and reoxidation procedures of Example 5, the cysteine engineered antibody is dissolved in PBS (phosphate buffered saline) buffer and chilled on ice. About 1.5 molar equivalents relative to engineered cysteines per antibody of a maytansinoid drug linker intermediate such as **5, 8, 14,** and **18,** with a thiol-reactive functional group such as maleimido or bromo-acetamide, is dissolved in DMSO, diluted in acetonitrile and water, and added to the chilled reduced, reoxidized antibody in PBS. After about one hour, an excess of maleimide is added to quench the reaction and cap any unreacted antibody thiol groups. The reaction mixture is concentrated by centrifugal ultrafiltration and the cysteine engineered trastuzumab antibody drug conjugate is purified and desalted by elution through G25 resin in PBS, filtered through 0.2 µm filters under sterile conditions, and frozen for storage.

By the procedure above, the following cysteine engineered, N-methyl alaninyl maytansinol antibody drug conjugates of Formula I were prepared:

| Figs. 5a, 5b, 6 | Antibody-drug conjugate | Drug/Antibody (DAR) | Drug-linker intermediate |
|---|---|---|---|
| (3) | LC-V205C Thio-TMAb-mal-PEG3-ala-May | 1.8 | **14** |
| (4) | HC-A118C Thio-TMAb-mal-PEG3-ala-May | 1.8 | **14** |
| (5) | LC-V205C Thio-TMAb-mal-hex-ala-May | 1.8 | **5** |
| (7) | LC-V205C Thio anti-gD5B6-mal-PEG3-ala-May | 1.8 | **14** |
| (8) | LC-V205C Thio anti-gD5B6-mal-hex-ala-May | 1.8 | **5** |
| (9) | HC-A118C Thio TMAb-mal-hex-ala-May | 1.9 | **5** |
| (10) | HC-A118C Thio anti-gD5B6-bra-PEG3-ala-May | 1.5 | **18** |

Maleimide DM1 conjugates, (2) LC V205C Thio-TMAb-mpeo-DM1, average drug loading DAR = 1.7, and (6) TMAb-mcc-DM1, average drug loading DAR = 3.4, were prepared according to the procedures in Example 4 of US 2005/0276812.

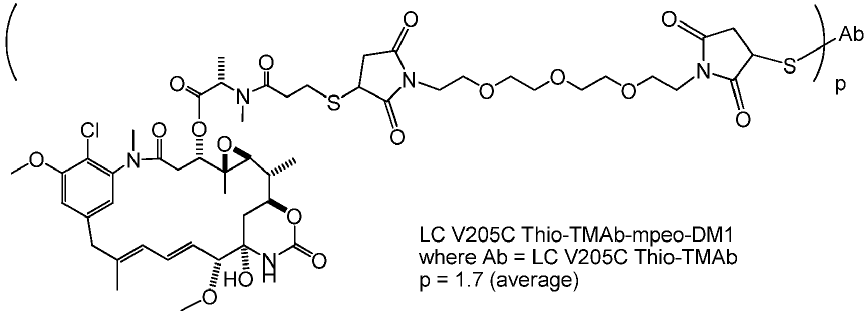

### Example 7 In vitro cell proliferation assay

Efficacy of ADC was measured by a cell proliferation assay employing the following protocol (CELLTITER GLO™ Luminescent Cell Viability Assay, Promega Corp. Technical Bulletin TB288; Mendoza et al (2002) Cancer Res. 62:5485-5488):
1. An aliquot of 100 µl of cell culture containing about 10⁴ cells (SKBR-3, BT474, MCF7 or MDA-MB-468) in medium was deposited in each well of a 96-well, opaque-walled plate.
2. Control wells were prepared containing medium and without cells.
3. ADC was added to the experimental wells and incubated for 3-5 days.
4. The plates were equilibrated to room temperature for approximately 30 minutes.
5. A volume of CELLTITER GLO™ Reagent equal to the volume of cell culture medium present in each well was added.
6. The contents were mixed for 2 minutes on an orbital shaker to induce cell lysis.
7. The plate was incubated at room temperature for 10 minutes to stabilize the luminescence signal.
8. Luminescence was recorded and reported in graphs as RLU = relative luminescence units.
Data are plotted as the mean of luminescence for each set of replicates, with standard deviation error bars. The protocol is a modification of the CELLTITER GLO™ Luminescent Cell
Media: SK-BR-3 grow in 50/50/10%FBS/glutamine/250 µg/mL G-418 OVCAR-3 grow in RPMI/20%FBS/glutamine

### Example 8 Tumor growth inhibition, in vivo efficacy in high expressing HER2 transgenic explant mice

The Fo5 mouse mammary tumor model was employed to evaluate the *in vivo* efficacy of (6) TMAb-mcc-DM1 and various Thio-TMAb-May conjugates of the invention (Example 6), after single dose intravenous injections, and as described previously (Phillips GDL, Li GM, Dugger DL, et al. Targeting HER2-Positive Breast Cancer with Trastuzumab-DM1, an Antibody-Cytotoxic Drug Conjugate. (2008) Cancer Res. 68:9280-90). The Fo5 model is a transgenic mouse model in which the human HER2 gene is over-expressed in mammary epithelium under transcriptional regulation of the murine mammary tumor virus promoter (MMTV-HER2). The HER2 overexpression causes spontaneous development of a mammary tumor. The mammary tumor of one of these founder animals (founder #5 [Fo5]) has been propagated in subsequent generations of FVB mice by serial transplantation of tumor fragments (~ 2 × 2 mm in size). All studies were conducted in accordance with the Guide for the Care and Use of Laboratory Animals. Each antibody-drug conjugate (single dose) was dosed in nine animals intravenously at the start of the study, and 14 days post-transplant. Initial tumor size was about 200 mm³ volume. Measurements of tumor growth inhibition over time by antibody-drug conjugates of the invention and controls are shown in Figures 5a, 5b, and 6.

## Claims

1. A compound selected from Formula I: 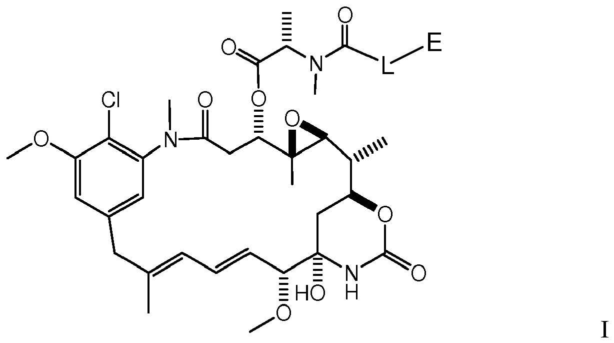 wherein:
L is 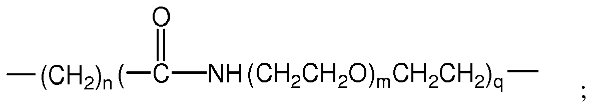
E is 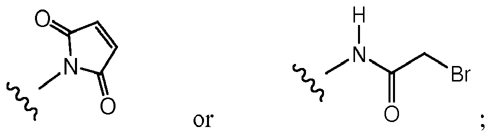
n is 2, 3, 4, 5, or 6;
m is 2, 3 or 4; and
q is 0 or 1.

2. The compound of claim 1 wherein L is
- (CH₂)ₙ-.

3. The compound of claim 2 wherein n is 5.

4. The compound of claim 1 wherein L is

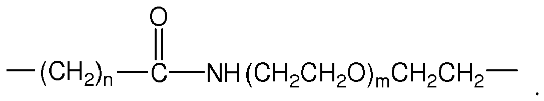

5. The compound of claim 4 wherein n is 4 and m is 3.

6. The compound of claim 1 having the structure: 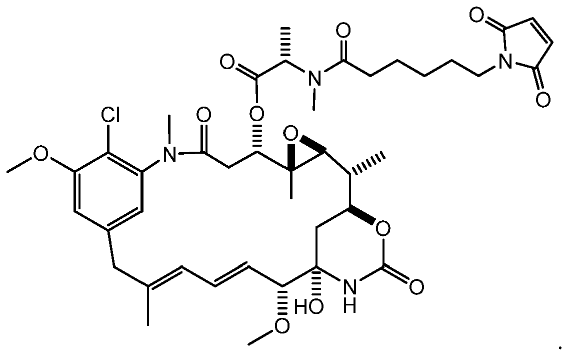 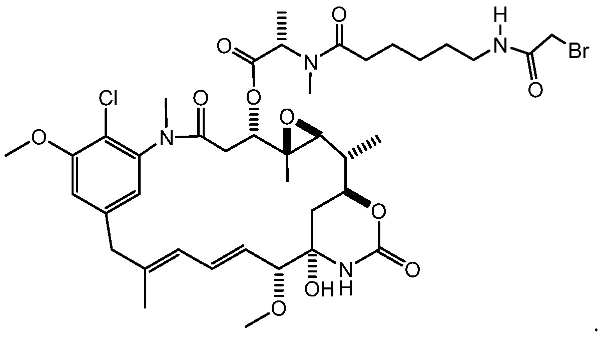 or 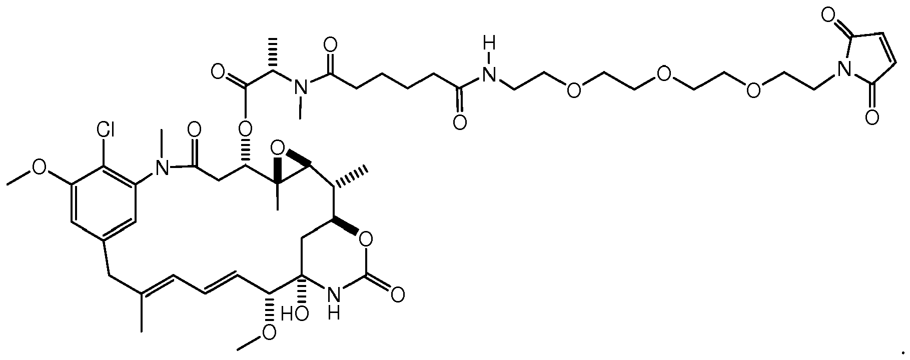 or

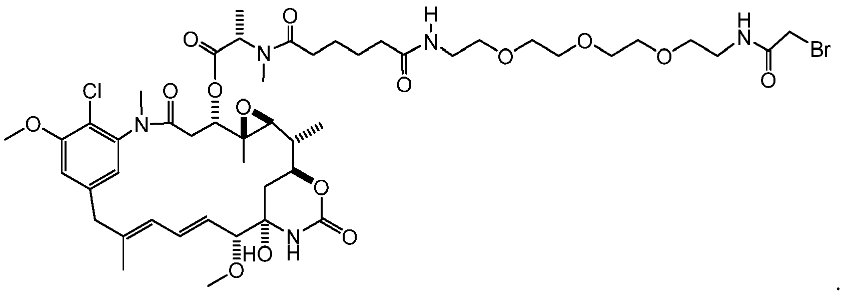

7. An antibody-drug conjugate selected from Formula Ia or Ib: 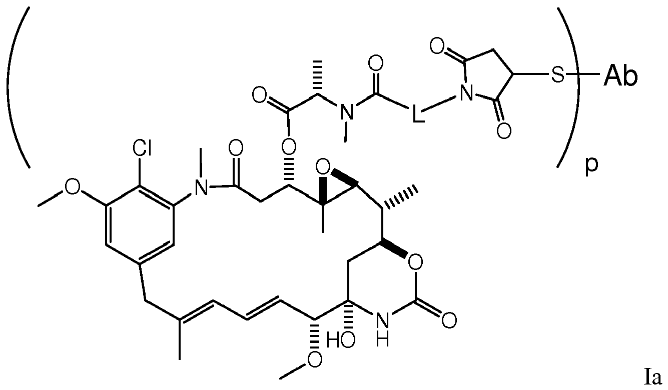 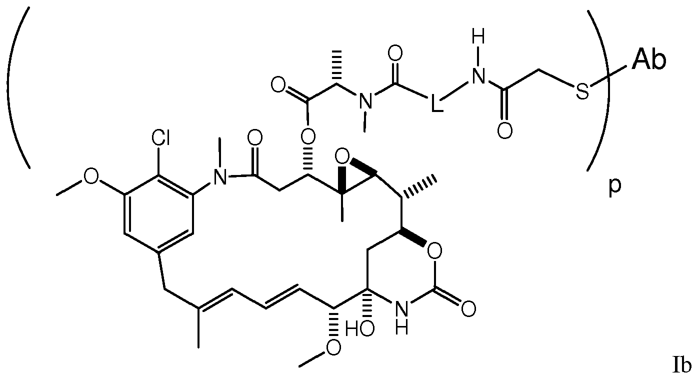 wherein:
L is 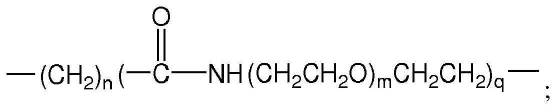
n is 2, 3, 4, 5, or 6;
m is 2, 3 or 4;
q is 0 or 1;
p is 1 to 4; and
Ab is an antibody.

8. The antibody-drug conjugate of claim 7 selected from the structures:

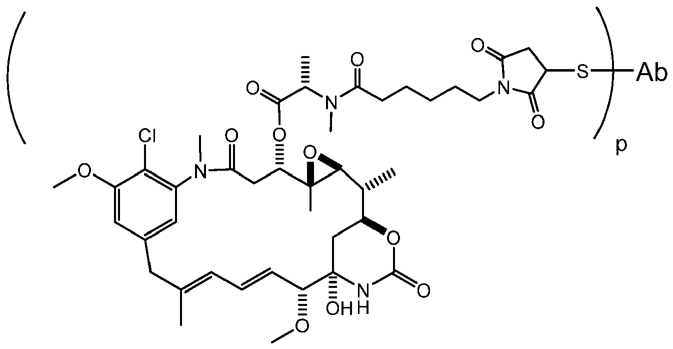

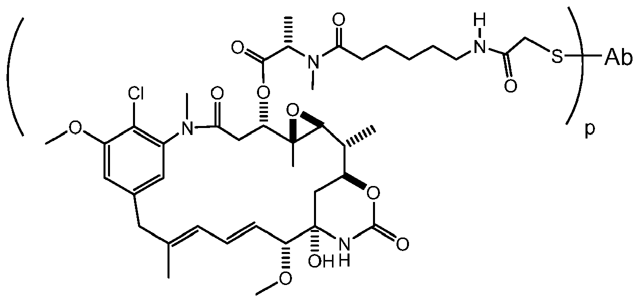

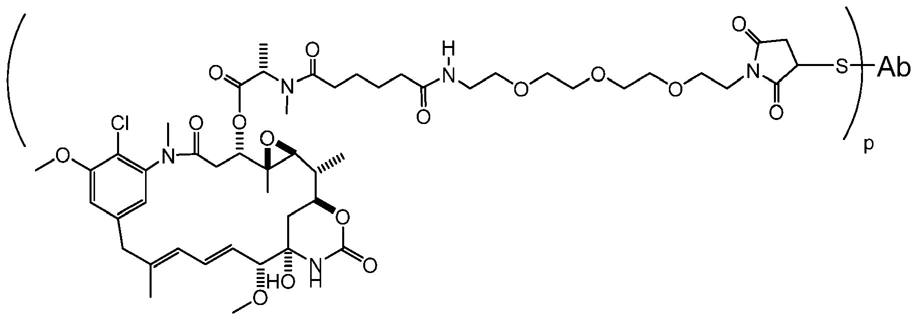

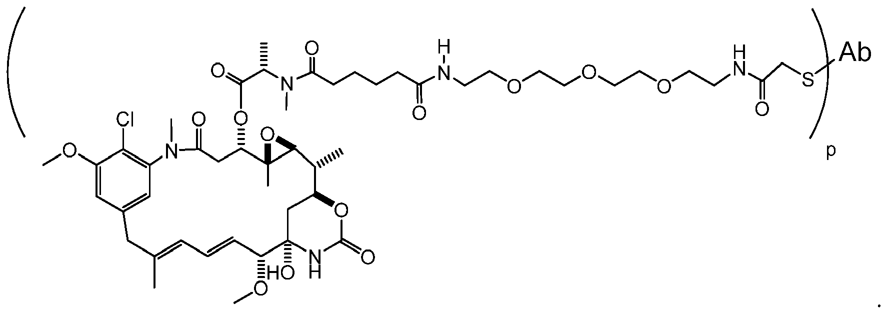

9. The antibody-drug conjugate of claim 8 wherein the antibody is a cysteine engineered antibody (Ab) conjugated through a free cysteine amino acid to a linker (L).

10. The antibody-drug conjugate of claim 9 wherein:
the free cysteine amino acid of the cysteine engineered antibody is A118C (EU numbering) of the heavy chain; or
the free cysteine amino acid of the cysteine engineered antibody is V205C (Kabat numbering) of the light chain; or the cysteine engineered antibody comprises a free cysteine amino acid and a sequence in the heavy chain selected from SEQ ID NOS 1-49 or a sequence in the light chain selected from SEQ ID NOS 50-98 wherein a cysteine in the sequence is the free cysteine amino acid.

11. The antibody-drug conjugate of claim 9 or claims 10 wherein the cysteine engineered antibody is prepared by a process comprising:
(i) mutagenizing a nucleic acid sequence encoding the cysteine engineered antibody;
(ii) expressing the cysteine engineered antibody; and
(iii) isolating and purifying the cysteine engineered antibody.

12. The antibody-drug conjugate of any one of claims 9 to 11, wherein the cysteine engineered antibody is selected from a monoclonal antibody, a bispecific antibody, a chimeric antibody, a human antibody, a humanized antibody, and a Fab fragment.

13. The antibody-drug conjugate of any one of claims 9 to 13, wherein the cysteine engineered antibody is prepared by a process comprising replacing one or more amino acid residues of a parent antibody with the one or more free cysteine amino acids, where the parent antibody selectively binds to an antigen and the cysteine engineered antibody selectively binds to the same antigen as the parent antibody.

14. The antibody-drug conjugate of any one of claims 7 to 13, wherein the antibody binds to one or more of receptors (1)-(51):
(1) BMPR1B (bone morphogenetic protein receptor-type IB);
(2) E16 (LAT1, SLC7A5);
(3) STEAP1 (six transmembrane epithelial antigen of prostate);
(4) 0772P (CA125, MUC16);
(5) MPF (MPF, MSLN, SMR, megakaryocyte potentiating factor, mesothelin);
(6) Napi3b (NAPI-3B, NPTIIb, SLC34A2, solute carrier family 34 (sodium phosphate), member 2, type II sodium-dependent phosphate transporter 3b);
(7) Sema 5b (FLJ10372, KIAA1445, Mm.42015, SEMA5B, SEMAG, Semaphorin 5b Hlog, sema domain, seven thrombospondin repeats (type 1 and type 1-like), transmembrane domain (TM) and short cytoplasmic domain, (semaphorin) 5B);
(8) PSCA hlg (2700050C12Rik, C530008O16Rik, RIKEN cDNA 2700050C12, RIKEN cDNA 2700050C12 gene);
(9) ETBR (Endothelin type B receptor);
(10) MSG783 (RNF124, hypothetical protein FLJ20315);
(11) STEAP2 (HGNC_8639, IPCA-1, PCANAP1, STAMP1, STEAP2, STMP, prostate cancer associated gene 1, prostate cancer associated protein 1, six transmembrane epithelial antigen of prostate 2, six transmembrane prostate protein);
(12) TrpM4 (BR22450, FLJ20041, TRPM4, TRPM4B, transient receptor potential cation channel, subfamily M, member 4);
(13) CRIPTO (CR, CR1, CRGF, CRIPTO, TDGF1, teratocarcinoma-derived growth factor);
(14) CD21 (CR2 (Complement receptor 2) or C3DR (C3d/Epstein Barr virus receptor) or Hs.73792);
(15) CD79b (CD79B, CD79β, IGb (immunoglobulin-associated beta), B29);
(16) FcRH2 (IFGP4, IRTA4, SPAP1A (SH2 domain containing phosphatase anchor protein 1a), SPAP1B, SPAP1C);
(17) HER2;
(18) NCA;
(19) MDP;
(20) IL20Rα;
(21) Brevican;
(22) EphB2R;
(23) ASLG659;
(24) PSCA;
(25) GEDA;
(26) BAFF-R (B cell -activating factor receptor, BLyS receptor 3, BR3);
(27) CD22 (B-cell receptor CD22-B isoform);
(28) CD79a (CD79A, CD79α, immunoglobulin-associated alpha;
(29) CXCR5 (Burkitt's lymphoma receptor 1;
(30) HLA-DOB (Beta subunit of MHC class II molecule);
(31) P2X5 (Purinergic receptor P2X ligand-gated ion channel 5;
(32) CD72 (B-cell differentiation antigen CD72, Lyb-2);
(33) LY64 (Lymphocyte antigen 64 (RP105), type I membrane protein of the leucine rich repeat (LRR) family);
(34) FcRH1 (Fc receptor-like protein 1);
(35) IRTA2 (Immunoglobulin superfamily receptor translocation associated 2);
(36) TENB2 (putative transmembrane proteoglycan);
(37) PMEL17 (silver homolog; SILV; D12S53E; PMEL17; (SI); (SIL); ME20; gp100);
(38) TMEFF1 (transmembrane protein with EGF-like and two follistatin-like domains 1; Tomoregulin-1; H7365; C9orf2; C9ORF2; U19878; X83961;
(39) GDNF-Ra1 (GDNF family receptor alpha 1; GFRA1; GDNFR; GDNFRA; RETL1; TRNR1; RET1L; GDNFR-alpha1; GFR-ALPHA-1; U95847; BC014962);
(40) Ly6E (lymphocyte antigen 6 complex, locus E; Ly67,RIG-E,SCA-2,TSA-1);
(41) TMEM46 (shisa homolog 2 (Xenopus laevis); SHISA2);
(42) Ly6G6D (lymphocyte antigen 6 complex, locus G6D; Ly6-D, MEGT1);
(43) LGR5 (leucine-rich repeat-containing G protein-coupled receptor 5; GPR49, GPR67);
(44) RET (ret proto-oncogene; MEN2A; HSCR1; MEN2B; MTC1; (PTC); CDHF12; Hs.168114; RET51; RET-ELE1);
(45) LY6K (lymphocyte antigen 6 complex, locus K; LY6K; HSJ001348; FLJ35226);
(46) GPR19 (G protein-coupled receptor 19; Mm 4787);
(47) GPR54 (KISS1 receptor; KISS1R; GPR54; HOT7T175; AXOR12);
(48) ASPHD1 (aspartate beta-hydroxylase domain containing 1; LOC253982);
(49) Tyrosinase (TYR; OCAIA; OCA1A; tyrosinase; SHEP3);
(50) TMEM118 (ring finger protein, transmembrane 2; RNFT2; FLJ14627); and
(51) GPR172A (G protein-coupled receptor 172A; GPCR41; FLJ11856; D15Ertd747e).

15. A pharmaceutical composition comprising the antibody-drug conjugate compound of any one of claims 7 to 14 and a pharmaceutically acceptable diluent, carrier or excipient.

16. The pharmaceutical composition of claim 15 further comprising a therapeutically effective amount of a chemotherapeutic agent.

17. An antibody-drug conjugate compound of any one of claims 7 to 14 for use in therapy.

18. An antibody-drug conjugate compound of any one of claims 7 to 14 for use in a method of treating cancer comprising administering to a patient the antibody-drug conjugate compound.

19. The antibody-drug conjugate compound for use in a method of treatment of claim 18, wherein the patient is administered a chemotherapeutic agent, in combination with the antibody-drug conjugate compound.

20. The use of an antibody-drug conjugate compound of claim 10 in the manufacture of a medicament for the treatment of cancer in a mammal.

21. An article of manufacture comprising
an antibody-drug conjugate compound of any one of claims 7 to14;
a container; and
a package insert or label indicating that the compound can be used to treat cancer.

## Patentansprüche

1. Verbindung ausgewählt aus Formel I: 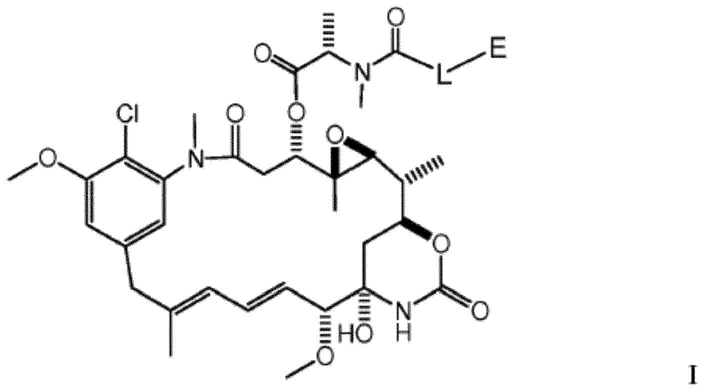 worin:
L 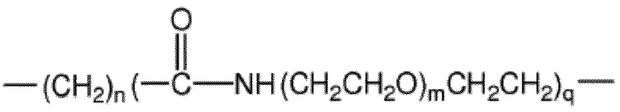 ist;
E 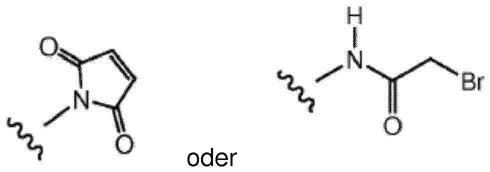 ist;
n = 2, 3, 4, 5 oder 6 ist;
m = 2, 3 oder 4 ist; und
q = 0 oder 1 ist.

2. Verbindung nach Anspruch 1, worin L - (CH₂)ₙ - ist.

3. Verbindung nach Anspruch 2, worin n = 5 ist.

4. Verbindung nach Anspruch 1, worin L 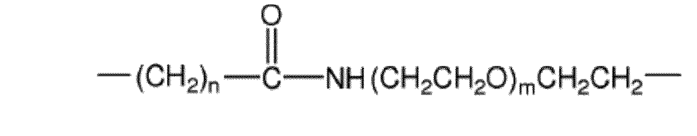 ist.

5. Verbindung nach Anspruch 4, worin n = 5 ist und m = 3 ist.

6. Verbindung nach Anspruch 1 mit der Struktur: 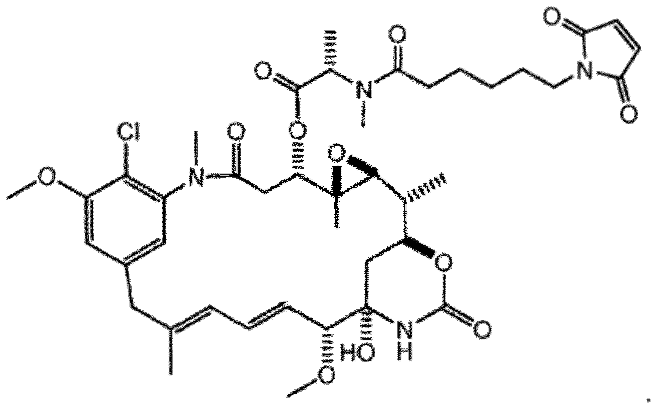 oder 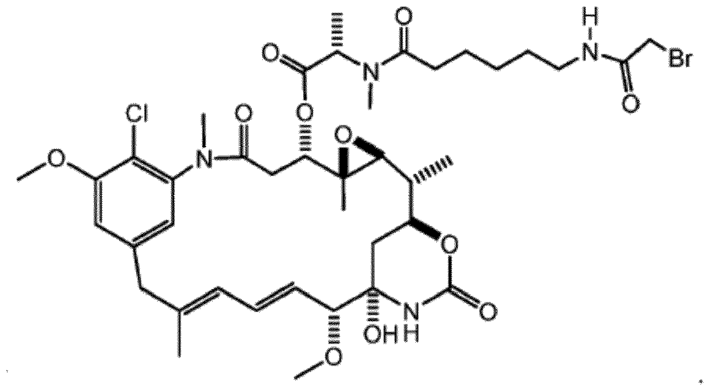 oder 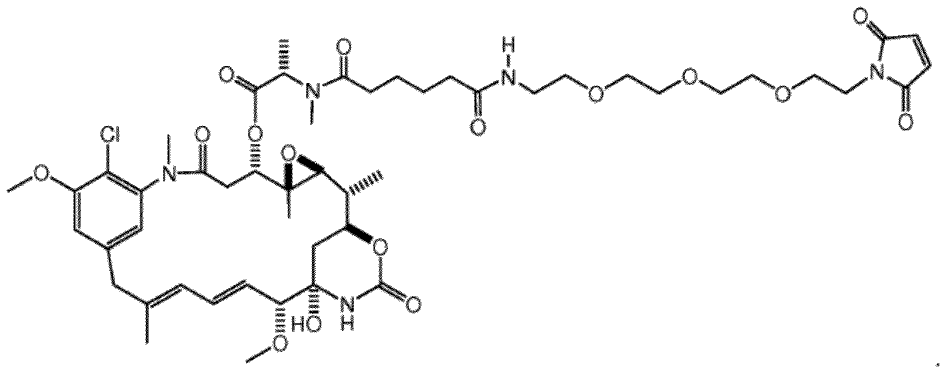 oder

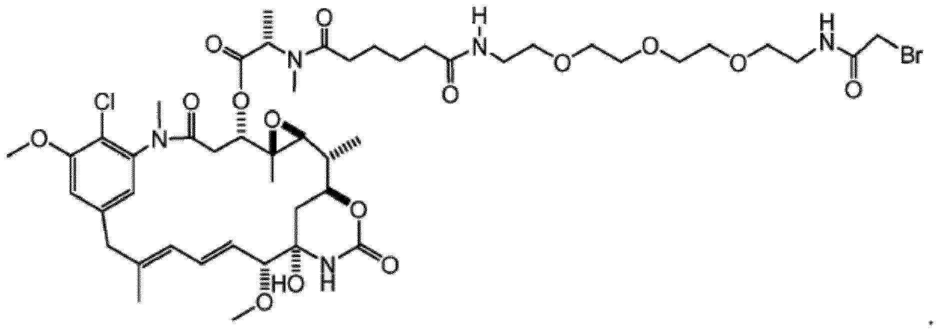

7. Antikörper-Arzneimittel-Konjugat ausgewählt aus Formel la oder Ib: 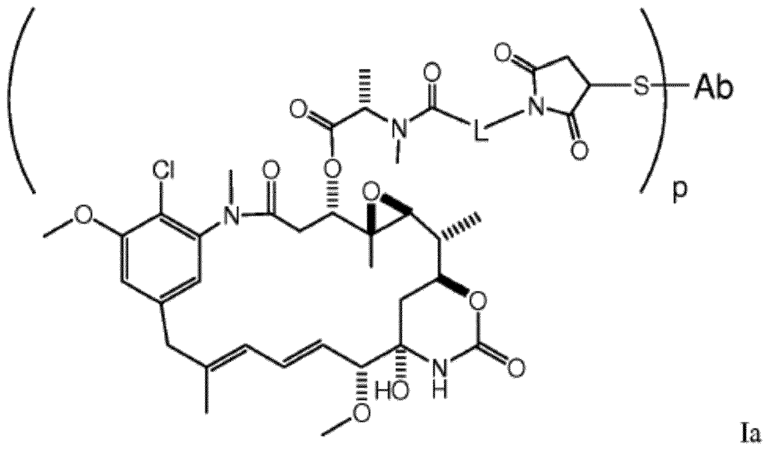 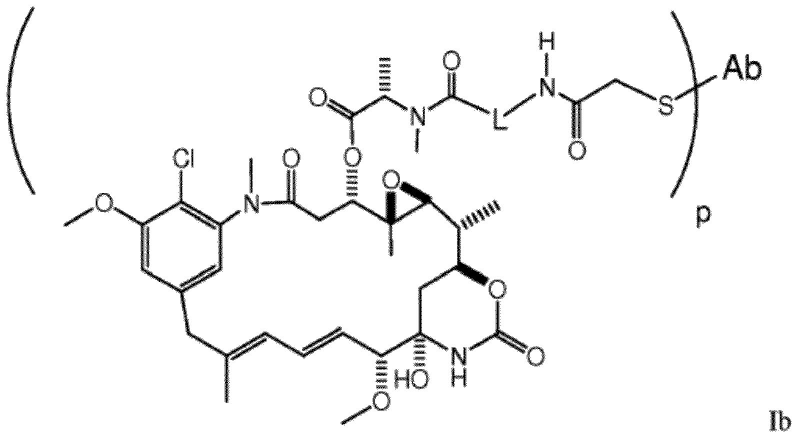 worin:
L 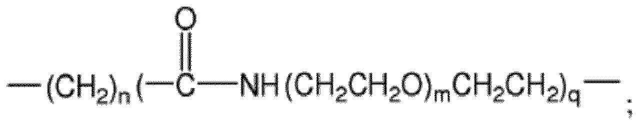 ist;
n = 2, 3, 4, 5 oder 6 ist;
m = 2, 3 oder 4 ist;
q = 0 oder 1 ist;
p = 1 bis 4 ist; und
Ab ein Antikörper ist.

8. Antikörper-Arzneimittel-Konjugat nach Anspruch 7, ausgewählt aus den folgenden Strukturen:

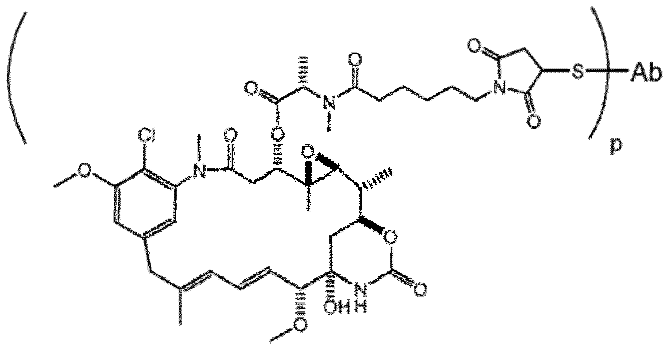

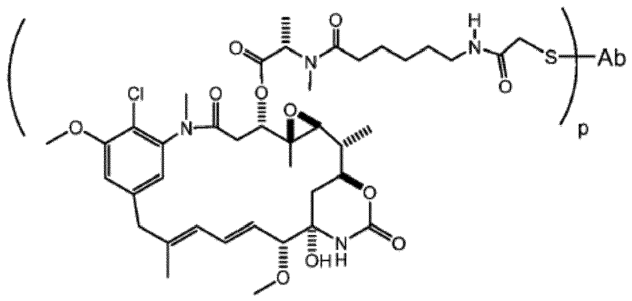

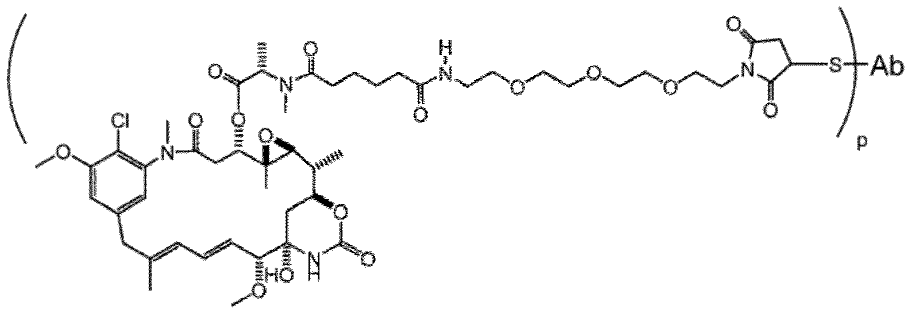

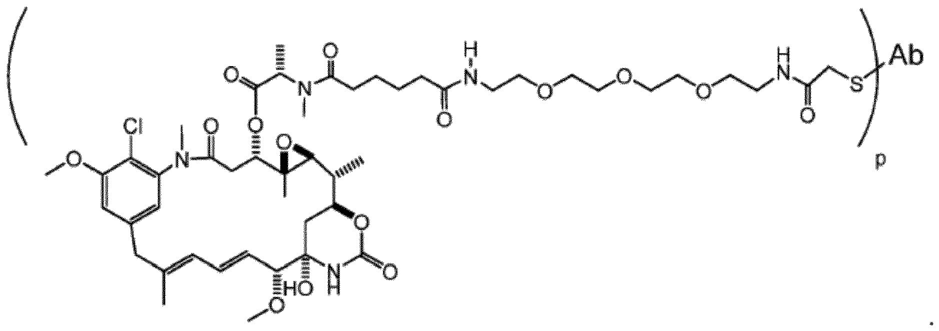

9. Antikörper-Arzneimittel-Konjugat nach Anspruch 8, worin der Antikörper ein Cystein-modifizierter Antikörper (Ab) ist, der über eine freie Cystein-Aminosäure an einen Linker (L) konjugiert ist.

10. Antikörper-Arzneimittel-Konjugat nach Anspruch 9, worin:
die freie Cystein-Aminosäure des Cystein-modifizierten Antikörpers A118C (EU-Nummerierung) der schweren Kette ist; oder
die freie Cystein-Aminosäure des Cystein-modifizierten Antikörpers V205C (Kabat-Nummerierung) der leichten Kette ist; oder der Cystein-modifizierte Antikörper eine freie Cystein-Aminosäure und eine aus den Seq.-ID Nr. 1-49 ausgewählte Sequenz in der schweren Kette oder eine aus Seq.-ID Nr. 50-98 ausgewählte Sequenz in der leichten Kette umfasst, wobei ein Cystein in der Sequenz die freie Cystein-Aminosäure ist.

11. Antikörper-Arzneimittel-Konjugat nach Anspruch 9 oder 10, worin der Cystein-modifizierte Antikörper durch ein Verfahren hergestellt wird, das Folgendes umfasst:
(i) das Mutagenisieren einer Nucleinsäuresequenz, die für den Cystein-modifizierten Antikörper kodiert;
(ii) das Exprimieren des Cystein-modifizierten Antikörpers; und
(iii) das Isolieren und Reinigen des Cystein-modifizierten Antikörpers.

12. Antikörper-Arzneimittel-Konjugat nach einem der Ansprüche 9 bis 11, worin der Cystein-modifizierte Antikörper aus einem monoklonalen Antikörper, einem bispezifischen Antikörper, einem chimären Antikörper, einem menschlichen Antikörper, einem humanisierten Antikörper und einem Fab-Fragment ausgewählt ist.

13. Antikörper-Arzneimittel-Konjugat nach einem der Ansprüche 9 bis 13, worin der Cystein-modifizierte Antikörper durch ein Verfahren hergestellt wird, welches das Ersetzen eines oder mehrerer Aminosäurereste eines Ausgangsantikörpers durch eine oder mehrere freie Cystein-Aminosäuren umfasst, wobei der Ausgangsantikörper selektiv an ein Antigen bindet und der Cystein-modifizierte Antikörper selektiv an dasselbe Antigen wie der Ausgangsantikörper bindet.

14. Antikörper-Arzneimittel-Konjugat nach einem der Ansprüche 7 bis 13, worin der Antikörper an einen oder mehrere der Rezeptoren (1)-(51) bindet:
(1) BMPR1 B (Knochenmorphogener Proteinrezeptor-Typ IB);
(2) E15 (LAT1, SLC7A5);
(3) STEAP1 (Sechs-Transmembran-Epithel-Antigen der Prostata);
(4) 0772P (CA 125, MUC16);
(5) MPF (MPF, MSLN, SMR, Megakaryozyten potenzierender Faktor, Mesothelin);
(6) Napi3b (NAPI-3B, NPTIIb, SLC34A2, Gelöststoffträgerfamilie 34 (Natriumphosphat), Mitglied 2, natriumabhängiger Typ-II-Phosphattransporter 3b);
(7) Sema 5b (FLJ10372, KIAA 1445, Mm.42015, SEMA5B, SEMAG, Semaphorin 5b Hlog, Semadomäne, sieben Thrombospondin-Wiederholungen (vom Typ 1 und typ-1-artig), Transmembrandomäne (TM) und kurze zytoplasmatische Domäne (Semaphorin) 5B);
(8) PSCA hlg (2700050C12Rik, C530008016Rik, RIKEN cDNA 2700050C12, RIKEN cDNA 2700050C12 Gen);
(9) ETBR (Endothelin-Typ-B-Rezeptor);
(10) MSG783 (RNF124, hypothetisches Protein FLJ20315);
(11) STEAP2 (HGNC_8639, IPCA-1, PCANAP1, STAMP1, STEAP2, STMP, Prostatakrebs-assoziiertes Gen 1, Prostatakrebs-assoziiertes Protein 1, Sechs-Transmembran-Epithel-Antigen der Prostata 2, Sechs-Transmembran-Prostataprotein);
(12) TrpM4 (BR22450, FLJ20041, TRPM4, TRPM4B, transientes Rezeptorpotentialkationen-5-Kanal, Unterfamilie M, Mitglied 4);
(13) CRIPTO (CR, CR1, CRGF, CRIPTO, TDGF1, von Teratokarzinom abgeleiteter Wachstumsfaktor);
(14) CD21 (CR2 (Komplementrezeptor 2) oder C3DR (C3d/Epstein-Barr-Virus-Rezeptor) oder Hs.73792);
(15) CD79b (CD79B, OΩ79β, IGb (Immunoglobulin-assoziiertes Beta), B29);
(16) FcRH2 (IFGP4, IRTA4, SPAP1A (SH2-Domänen enthaltendes Phosphatase-Aankerprotein 51 a), SPAP1 B, SPAP1 C);
(17) HER2 (ErbB2);
(18) NCA
(19) MDP
(20) IL20R-alpha
(21) Brevican
(22) EphB2R
(23) ASLG659
(24) PSCA
(25) GEDA
(26) BAFF-R (B-Zell-Aktivierungsfaktor-Rezeptor, BLyS-Rezeptor 3, BR3);
(27) CD22 (B-Zell-Rezeptor CD22-B Isoform)
(28) CD79a (CD79A, CD79alpha, Immunoglobulin-assoziiertes α);
(29) CXCR5 (Burkitt's Lymphomrezeptor 1);
(30) HLA-DOB (β-Untereinheit des MHC-Klasse-II-Moleküls);
(31) P2X5 (purinergischer Rezeptor-P2X-Liganden-beschränkter lonenkanal 5);
(32) CD72 (B-Zell-Differenzierungsantigen CD72, Lyb-2);
(33) LY64 (Lymphozytenantigen 64 (RP105), Typ-I-Membranprotein aus der Familie der leucinreichen Repeats (LRR));
(34) FcRH1 (Fc-rezeptorartiges Protein 1);
(35) IRTA2 (Immunglobulin-Überfamilien-Rezeptortranslokation assoziiert 2);
(36) TENB2 (mögliches Transmembran-Proteoglykan);
(37) PMEL 17 (Silberhomolog; SILV; D12S53E; PMEL17; (SI); (SIL); ME20; gp100);
(38) TMEFF1 (Transmembranprotein mit EGF-artiger und zwei Follistatin-artigen Domänen; Tomoregulin-1; H7365; C9orf2; C9ORF2; U19878; X83961);
(39) GDNF-Ra1 (GNDF-Rezeptorfamilie α1; GFRA1;GDNFR; GDNFRA; RETL1; TRNR1; RET1 L; GDNFR-α1; GDR-ALPHA-1; U95847;BC014962);
(40) Ly6E (Lymphozytenantigen-6-Komplex, Locus E; Ly67, RIG-E, SCA-2, TSA-1);
(41) TMEM46 (Shisha-Homolog-2 (Xenopus laevis); SHISA2);
(42) Ly6G6D (Lymphozytenantigen-6-Komplex, Locus G6D; Ly6-D, MEGT1);
(43) LGR5 (leucinreiche Repeats enthaltender G-Protein-gekoppelter Rezeptor 5; GPR49, GPR67);
(44) RET (Ret-Proto-Onkogen; MEN2A; HSCR1; MEN2B; METC1; (PTC); CDHF12; Hs.168114; RET51; RET-ELE1);
(45) LY6K (Lymphozytenantigen-6-Komplex, Locus K; LY6K; HSJ001348; FLJ35226);
(46) GPR19 (G-Protein-gekoppelter Rezeptor 19; Mm 4787);
(47) GPR54 (KISS1-Rezeptor; KISS1R; GPR54; HOT7T175); AXOR12);
(48) ASPHD1 (Aspartat-β-Hydroxylase-Domäne, enthaltend 1; LOC253982);
(49) Tyrosinase (TYR; OCAIA; OCA1A; Tyrosinase; SHEP3);
(50) TMEM118 (Ringfingerprotein, Transmembran 2; RNFT2; FLJ14627); und
(51) GPR172A (G-Protein-gekoppelter Rezeptor 172A; GPCR41; FLJ11856; D15Ertd747e).

15. Pharmazeutische Zusammensetzung, die eine Antikörper-Arzneimittel-Konjugatverbindung nach einem der Ansprüche 7 bis 14 und einen pharmazeutisch annehmbaren Verdünner, Träger oder Exzipienten umfasst.

16. Pharmazeutische Zusammensetzung nach Anspruch 15, die zudem eine therapeutisch wirksame Menge eines chemotherapeutischen Mittels umfasst.

17. Antikörper-Arzneimittel-Konjugatverbindung nach einem der Ansprüche 7 bis 14 zur Verwendung in der Therapie.

18. Antikörper-Arzneimittel-Konjugatverbindung nach einem der Ansprüche 7 bis 14 für die Verwendung in einem Verfahren zum Behandeln von Krebs, das die Verabreichung der Antikörper-Arzneimittel-Konjugatverbindung an einen Patienten umfasst.

19. Antikörper-Arzneimittel-Konjugatverbindung zur Verwendung in einem Verfahren zur Behandlung nach Anspruch 18, wobei dem Patienten ein chemotherapeutisches Mittel in Kombination mit der Antikörper-Arzneimittel-Konjugatverbindung verabreicht wird.

20. Verwendung einer Antikörper-Arzneimittel-Konjugatverbindung nach Anspruch 10 zur Herstellung eines Medikaments zur Behandlung von Krebs bei einem Säugetier.

21. Fertigartikel, der umfasst:
eine Antikörper-Arzneimittel-Konjugatverbindung nach einem der Ansprüche 7 bis 14;
einen Behälter; und
eine Packungsbeilage oder ein Etikett, die/das angibt, dass die Verbindung zur Behandlung von Krebs einsetzbar ist.

## Revendications

1. Composé choisi parmi les composés de formule I : 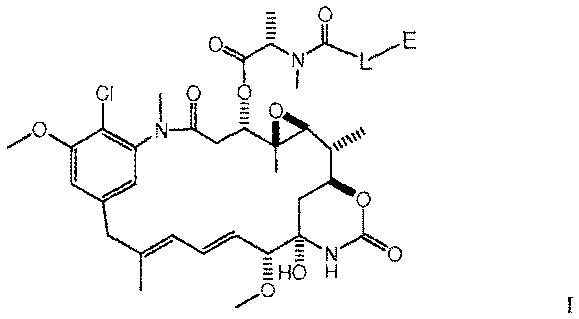 dans laquelle :
L représente un groupe 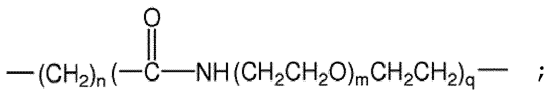
E représente un groupe 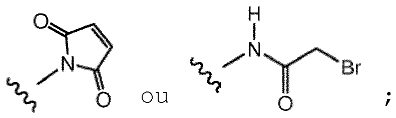
n est égal à 2, 3, 4, 5 ou 6 ;
m est égal à 2, 3 ou 4 ; et
q est égal à 0 ou 1.

2. Composé suivant la revendication 1, dans lequel L représente un groupe
-(CH₂)ₙ-.

3. Composé suivant la revendication 2, dans lequel n est égal à 5.

4. Composé suivant la revendication 1, dans lequel L représente un groupe

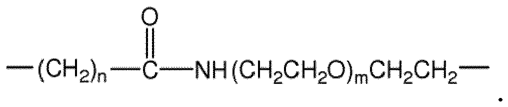

5. Composé suivant la revendication 4, dans lequel n est égal à 4 et m est égal à 3.

6. Composé suivant la revendication 1, ayant la structure : 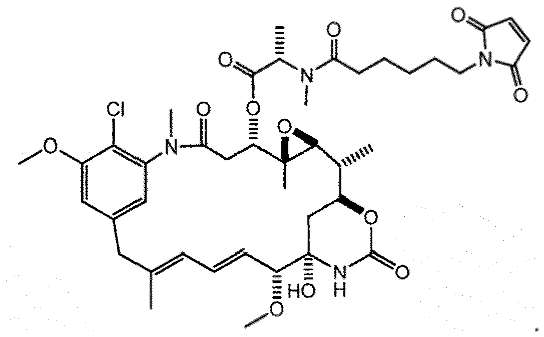 ou 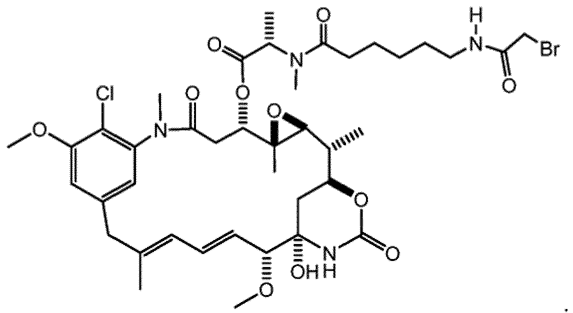 ou 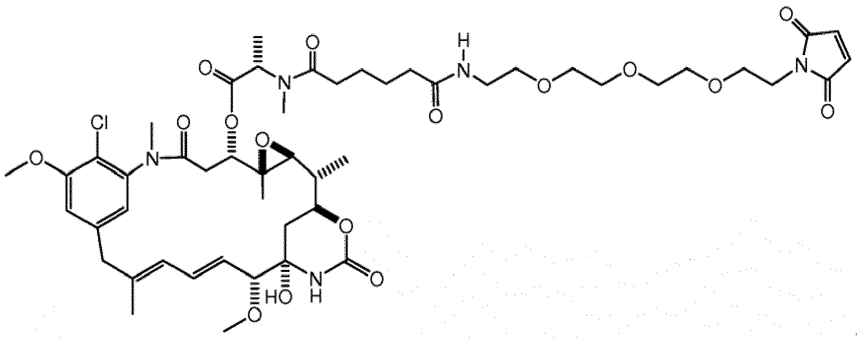 ou

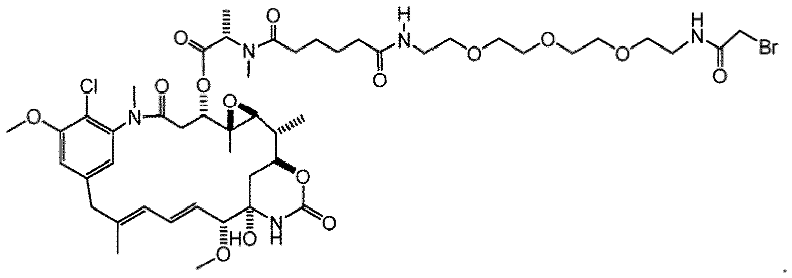

7. Conjugué anticorps-médicament choisi entre la formule la et la formule Ib : 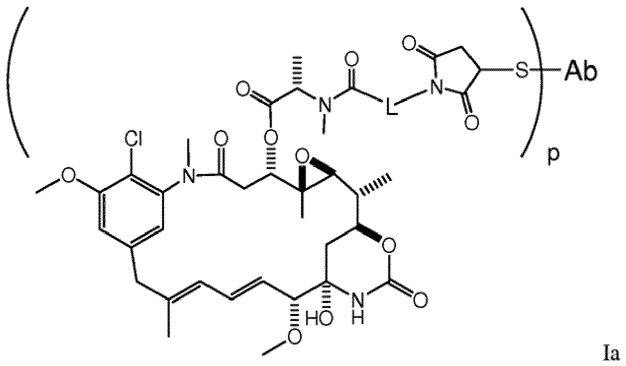 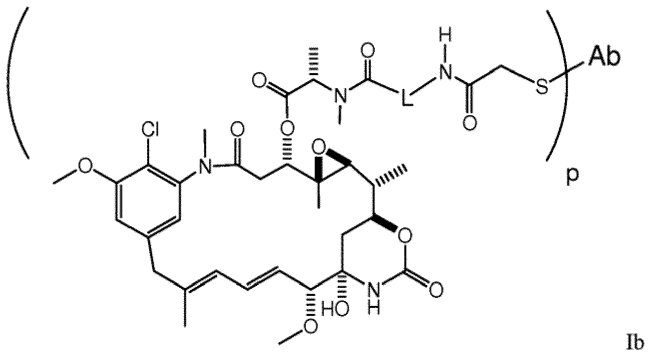 dans lesquelles :
L représente un groupe 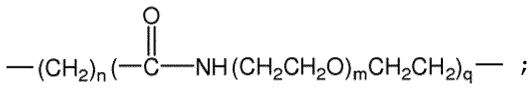
n est égal à 2, 3, 4, 5 ou 6 ;
m est égal à 2, 3 ou 4 ;
q est égal à 0 ou 1 ;
p a une valeur de 1 à 4 ; et
Ab représente un anticorps.

8. Conjugué anticorps-médicament suivant la revendication 7, choisi entre les structures :

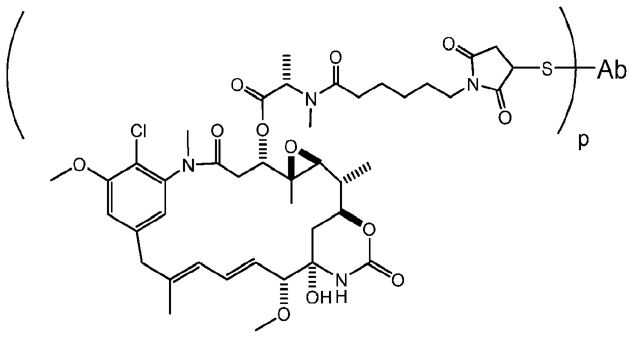

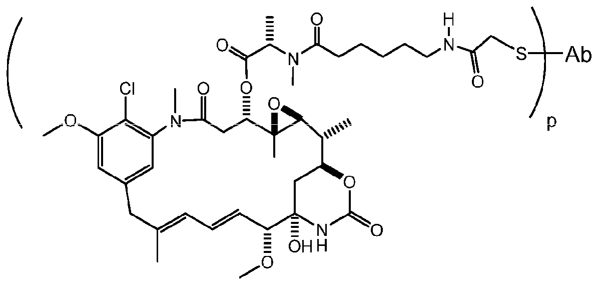

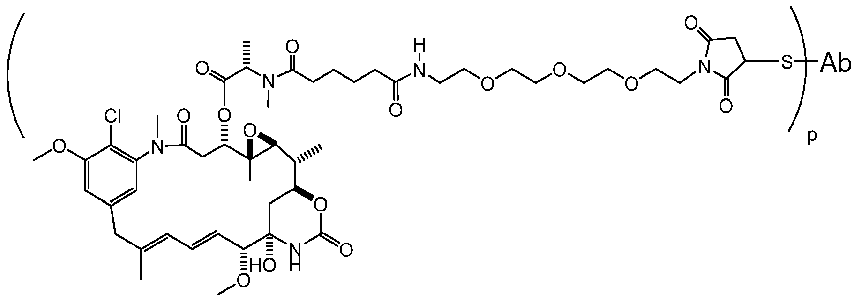

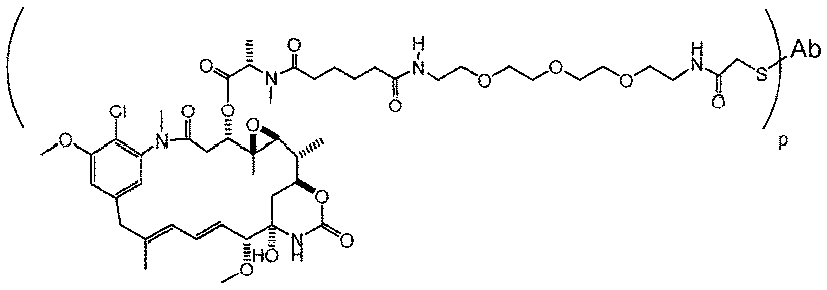

9. Conjugué anticorps-médicament suivant la revendication 8, dans lequel l'anticorps est un anticorps modifié génétiquement avec une cystéine (Ab) conjugué par un amino-acide cystéine libre à un groupe de liaison (L).

10. Conjugué anticorps-médicament suivant la revendication 9, dans lequel :
l'aminoacide cystéine libre de l'anticorps modifié génétiquement avec une cystéine est A118C (numérotation EU) de la chaîne lourde ; ou
l'aminoacide cystéine libre de l'anticorps modifié génétiquement avec une cystéine est V205C (numérotation de Kabat) de la chaîne légère ; ou l'anticorps modifié génétiquement avec une cystéine comprend un aminoacide cystéine libre et une séquence dans la chaîne lourde choisie parmi les SEQ ID N° 1-49 ou une séquence dans la chaîne légère choisie parmi les SEQ ID N° 50-98, une cystéine dans la séquence étant l'aminoacide cystéine libre.

11. Conjugué anticorps-médicament suivant la revendication 9 ou la revendication 10, dans lequel l'anticorps modifié génétiquement avec une cystéine est préparé par un procédé comprenant :
(i) la mutagenèse d'une séquence d'acide nucléique codant pour l'anticorps modifié génétiquement avec une cystéine ;
(ii) l'expression de l'anticorps modifié génétiquement avec une cystéine ; et
(iii) l'isolement et la purification de l'anticorps modifié génétiquement avec une cystéine.

12. Conjugué anticorps-médicament suivant l'une quelconque des revendications 9 à 11, dans lequel l'anticorps modifié génétiquement avec une cystéine est choisi entre un anticorps monoclonal, un anticorps bispécifique, un anticorps chimère, un anticorps humain, un anticorps humanisé et un fragment Fab.

13. Conjugué anticorps-médicament suivant l'une quelconque des revendications 9 à 13, dans lequel l'anticorps modifié génétiquement avec une cystéine est préparé par un procédé comprenant le remplacement d'un ou plusieurs résidus d'aminoacides d'un anticorps parental par le ou les amino-acides cystéine libres, l'anticorps parental se liant sélectivement à un antigène et l'anticorps modifié génétiquement avec une cystéine se liant sélectivement au même antigène que l'anticorps parental.

14. Conjugué anticorps-médicament suivant l'une quelconque des revendications 7 à 13, dans lequel l'anticorps se lie à un ou plusieurs des récepteurs (1)-(51) :
(1) BMPR1B (récepteur de protéine morphogénétique osseuse de type 1B) ;
(2) E16 (LAT1, SLC7A5) ;
(3) STEAP1 (antigène épithélial à six domaines transmembranaire de la prostate) ;
(4) 0772P (CA125, MUC16) ;
(5) MPF (MPF, MSLN, SMR, facteur de potentialisation des mégacaryocytes, mésothéline) ;
(6) Napi3b (NAPIµ-3B, NPTIIb, SLC34A2, famille des supports de soluté 34 (phosphate de sodium), membre 2, transporteur de phosphate dépendant du sodium de type II 3b) ;
(7) Sema 5b (FLJ10372, KIAA1445, Mm.42015, SEMA5B, SEMAG, Sémaphorine 5b Hlog, domaine sema, sept segments répétés de thrombospondine (type 1 et analogue type 1), domaine transmembranaire (TM) et domaine cytoplasmique court (sémaphorine) 5B) ;
(8) PSCA hlg (2700050C12Rik, C530008O16Rik, ADNc RIKEN 2700050C12, gène à ADNc RIKEN 2700050C12) ;
(9) ETBR (récepteur d'endothéline de type B) ;
(10) MSG783 (RNF124, protéine hypothétique FLJ20315) ;
(11) STEAP2 (HGNC_8639, IPCA-1, PCANAP1, STAMP1, STEAP2, STMP, gène 1 associé au cancer de la prostate, protéine 1 associée au cancer de la prostate, antigène épithélial à six domaines transmembranaire de prostate 2, protéine à six domaines transmembranaire de prostate) ;
(12) TrpM4 (BR22450, FLJ20041, TRPM4, TRPM4B, canal cationique potentiel de récepteur transitoire, sous-famille M, membre 4) ;
(13) CRIPTO (CR, CR1, CRGF, CRIPTO, TDGF1, facteur de croissance dérivé d'un tératocarcinome ;
(14) CD21 (CR2 (récepteur du complément 2) ou C3DR (C3d/récepteur du virus d'Epstein Barr) ou Hs.73792) ;
(15) CD79b (CD79B, CD79β, IGb (associé aux immunoglobulines bêta), B29) ;
(16) FcRH2 (IFGP4, IRTA4, SPAP1A (protéine d'ancrage de phosphatase contenant le domaine SH2 la), SPAP1B, SPAP1C) ;
(17) HER2 ;
(18) NCA ;
(19) MDP ;
(20) IL20Rα ;
(21) Brevican ;
(22) FphB2R ;
(23) ASLG659 ;
(24) PSCA ;
(25) GEDA ;
(26) BAFF-R (récepteur de facteur d'activation des lymphocytes B, récepteur BLyS 3, BR3) ;
(27) CD22 (isoforme CD22-B des récepteurs de lymphocytes B) ;
(28) CD79a (CD79A, CD79α, associé aux immunoglobulines alpha) ;
(29) CXCR5 (récepteur de lymphome de Burkitt 1) ;
(30) HLA-DOB (sous-unité bêta de molécule du CMH de classe II) ;
(31) P2X5 (canal ionique déclenché par ligand P2X de récepteur purinergique 5) ;
(32) CD72 (antigène de différenciation des lymphocytes B CD72, Lyb-2) ;
(33) LY64 (antigène lymphocytaire 64 (RP105), protéine membranaire de type 1 de la famille des segments répétés riches en leucine (LRR)) ;
(34) FcRH1 (protéine du type récepteur Fc 1) ;
(35) IRTA2 (translocation associée aux récepteurs de la superfamille des immunoglobulines 2) ;
(36) TENB2 (protéoglycane transmembranaire putatif) ;
(37) PMEL17 (homologue d'argent ; SILV ; D12S53E ; PMEL17 ; (SI) ; (SIL) ; ME20 ; gp100) ;
(38) TMEFF1 (protéine transmembranaire avec un domaine du type EGF et deux domaines de type follistatine 1 ; tomoréguline-+1 ; H7365 ; C9orf2 ; C90RF2 ; U19878 ; X83961 ;
(39) GDNF-Ra1 (récepteur alpha 1 de la famille du GDNF ; GFRA1 ; GDNFR ; GDNFRA ; RET11 ; TRNR1 ; RET1L ; GDNFR-alpha 1 ; GFA-ALPHA-1 ; U95847 ; BC014962) ;
(40) Ly6E (complexe d'antigène lymphocytaire 6, locus E, Ly67, RIG-E.SCA-2, TSA-1) ;
(41) TMEM46 (homologue shisa 2 (Xenopus laevis) ; SHISA2) ;
(42) Ly6G6D (complexe d'antigène lymphocytaire 6, locus G6D, Ly6-D, MEGT1) ;
(43) LGR5 (récepteur couplé à la protéine G contenant un segment répété riche en leucine 5 ; GPR49, GPR67) ;
(44) RET (proto-oncogène ret ; MEN2A ; HSCR1 ; MEN2B ; MTC1 ; (PTC) ; CDHF12 ; Hs.168114 ; RET51. RET-ELE1).
(45) LY6K (complexe d'antigène lymphocytaire 6, locus K ; LY6K ; HSJ001348 ; FLJ35226) ;
(46) GPR19 (récepteur 19 couplé à la protéine G ; Mm 4787) ;
(47) GPR54 (récepteur KISS1 ; GPR54 ; HOT7T175 ; AXOR12) ;
(48) ASPHD1 (domaine d'aspartate-bêta-hydroxylase contenant 1 ; LOC253982) ;
(49) Tyrosinase (TYR ; OCALA ; OCA1A ; tyrosinase ; SHEP3) ;
(50) TMEM118 (protéine digitiforme en anneau, transmembranaire 2 ; RNFT2 ; FLJ14627) ; et
(51) GPR172A (récepteur couplé à la protéine G 172A ; GPRC41 ; FLJ11856 ; D15Ertd747e).

15. Composition pharmaceutique comprenant le composé conjugué anticorps-médicament de l'une quelconque des revendications 7 à 14 et un diluant, support ou excipient pharmaceutiquement acceptable.

16. Composition pharmaceutique suivant la revendication 15, comprenant en outre une quantité thérapeutiquement efficace d'un agent chimiothérapeutique.

17. Composé conjugué anticorps-médicament de l'une quelconque des revendications 7 à 14, pour une utilisation en thérapie.

18. Composé conjugué anticorps-médicament de l'une quelconque des revendications 7 à 14, pour une utilisation dans un procédé de traitement du cancer, comprenant l'administration à un patient du composé conjugué anticorps-médicament.

19. Composé conjugué anticorps-médicament pour une utilisation dans un procédé de traitement suivant la revendication 18, le patient étant soumis à l'administration d'un agent chimiothérapeutique, en association avec le composé conjugué anticorps-médicament.

20. Utilisation d'un composé conjugué anticorps-médicament de la revendication 10, dans la production d'un médicament pour le traitement d'un cancer chez un mammifère.

21. Article manufacturé, comprenant
un composé conjugué anticorps-médicament de l'une quelconque des revendications 7 à 14 ;
un récipient ; et
un encart d'emballage ou une étiquette indiquant que le composé peut être utilisé pour traiter le cancer.


### Sequence Listing

<110> GENENTECH, INC.
<120> ALANINYL MAYTANSINOL ANTIBODY CONJUGATES
<130> P4497R1-WO
<141> 2011-11-16
<150> US 61/414,535
   <151> 2010-11-17
<160> 98
<210> 1
   <211> 9
   <212> PRT
   <213> Artificial sequence
<220>
   <223> sequence is synthesized
<400> 1
   Glu Val Gln Cys Val Glu Ser Gly Gly
    1               5
<210> 2
   <211> 11
   <212> PRT
   <213> Artificial sequence
<220>
   <223> sequence is synthesized
<400> 2
   Gln Leu Val Glu Ser Cys Gly Gly Leu Val Gln
    1               5                  10
<210> 3
   <211> 11
   <212> PRT
   <213> Artificial sequence
<220>
   <223> sequence is synthesized
<400> 3
   Val Glu Ser Gly Gly Cys Leu Val Gln Pro Gly
   1 5 10
<210> 4
   <211> 11
   <212> PRT
   <213> Artificial sequence
<220>
   <223> sequence is synthesized
<400> 4
   Gly Gly Ser Leu Arg Cys Ser Cys Ala Ala Ser
   1 5 10
<210> 5
   <211> 11
   <212> PRT
   <213> Artificial sequence
<220>
   <223> sequence is synthesized
<400> 5
   Leu Arg Leu Ser Cys Cys Ala Ser Gly Phe Asn
   1 5 10
<210> 6
   <211> 11
   <212> PRT
   <213> Artificial sequence
<220>
   <223> sequence is synthesized
<400> 6
   Ser Cys Ala Ala Ser Cys Phe Asn Ile Lys Asp
   1 5 10
<210> 7
   <211> 11
   <212> PRT
   <213> Artificial sequence
<220>
   <223> sequence is synthesized
<400> 7
   Cys Ala Ala Ser Gly Cys Asn Ile Lys Asp Thr
   1 5 10
<210> 8
   <211> 11
   <212> PRT
   <213> Artificial sequence
<220>
   <223> sequence is synthesized
<400> 8
   Phe Asn Ile Lys Asp Cys Tyr Ile His Trp Val
    1               5                  10
<210> 9
   <211> 11
   <212> PRT
   <213> Artificial sequence
<220>
   <223> sequence is synthesized
<400> 9
   Ile His Trp Val Arg Cys Ala Pro Gly Lys Gly
    1               5                  10
<210> 10
   <211> 11
   <212> PRT
   <213> Artificial sequence
<220>
   <223> sequence is synthesized
<400> 10
   Trp Val Arg Gln Ala Cys Gly Lys Gly Leu Glu
    1               5                  10
<210> 11
   <211> 11
   <212> PRT
   <213> Artificial sequence
<220>
   <223> sequence is synthesized
<400> 11
   Arg Gln Ala Pro Gly Cys Gly Leu Glu Trp Val
    1               5                  10
<210> 12
   <211> 11
   <212> PRT
   <213> Artificial sequence
<220>
   <223> sequence is synthesized
<400> 12
   Gln Ala Pro Gly Lys Cys Leu Glu Trp Val Ala
   1 5 10
<210> 13
   <211> 11
   <212> PRT
   <213> Artificial sequence
<220>
   <223> sequence is synthesized
<400> 13
   Gly Lys Gly Leu Glu Cys Val Ala Arg Ile Tyr
   1 5 10
<210> 14
   <211> 11
   <212> PRT
   <213> Artificial sequence
<220>
   <223> sequence is synthesized
<400> 14
   Thr Arg Tyr Ala Asp Cys Val Lys Gly Arg Phe
   1 5 10
<210> 15
   <211> 11
   <212> PRT
   <213> Artificial sequence
<220>
   <223> sequence is synthesized
<400> 15
   Ser Val Lys Gly Arg Cys Thr Ile Ser Ala Asp
   1 5 10
<210> 16
   <211> 11
   <212> PRT
   <213> Artificial sequence
<220>
   <223> sequence is synthesized
<400> 16
   Phe Thr Ile Ser Ala Cys Thr Ser Lys Asn Thr
   1 5 10
<210> 17
   <211> 11
   <212> PRT
   <213> Artificial sequence
<220>
   <223> sequence is synthesized
<400> 17
   Ser Ala Asp Thr Ser Cys Asn Thr Ala Tyr Leu
    1               5                  10
<210> 18
   <211> 11
   <212> PRT
   <213> Artificial sequence
<220>
   <223> sequence is synthesized
<400> 18
   Asp Thr Ser Lys Asn Cys Ala Tyr Leu Gln Met
    1               5                  10
<210> 19
   <211> 11
   <212> PRT
   <213> Artificial sequence
<220>
   <223> sequence is synthesized
<400> 19
   Ser Lys Asn Thr Ala Cys Leu Gln Met Asn Ser
    1               5                  10
<210> 20
   <211> 11
   <212> PRT
   <213> Artificial sequence
<220>
   <223> sequence is synthesized
<400> 20
   Lys Asn Thr Ala Tyr Cys Gln Met Asn Ser Leu
    1               5                  10
<210> 21
   <211> 11
   <212> PRT
   <213> Artificial sequence
<220>
   <223> sequence is synthesized
<400> 21
   Asn Thr Ala Tyr Leu Cys Met Asn Ser Leu Arg
    1               5                  10
<210> 22
   <211> 11
   <212> PRT
   <213> Artificial sequence
<220>
   <223> sequence is synthesized
<400> 22
   Leu Gln Met Asn Ser Cys Arg Ala Glu Asp Thr
    1               5                  10
<210> 23
   <211> 11
   <212> PRT
   <213> Artificial sequence
<220>
   <223> sequence is synthesized
<400> 23
   Met Asn Ser Leu Arg Cys Glu Asp Thr Ala Val
    1               5                  10
<210> 24
   <211> 11
   <212> PRT
   <213> Artificial sequence
<220>
   <223> sequence is synthesized
<400> 24
   Ser Leu Arg Ala Glu Cys Thr Ala Val Tyr Tyr
    1               5                  10
<210> 25
   <211> 11
   <212> PRT
   <213> Artificial sequence
<220>
   <223> sequence is synthesized
<400> 25
   Ala Glu Asp Thr Ala Cys Tyr Tyr Cys Ser Arg
    1               5                  10
<210> 26
   <211> 11
   <212> PRT
   <213> Artificial sequence
<220>
   <223> sequence is synthesized
<400> 26
   Glu Asp Thr Ala Val Cys Tyr Cys Ser Arg Trp
   1               5                   10
<210> 27
   <211> 11
   <212> PRT
   <213> Artificial sequence
<220>
   <223> sequence is synthesized
<400> 27
   Val Tyr Tyr Cys Ser Cys Trp Gly Gly Asp Gly
   1               5                   10
<210> 28
   <211> 11
   <212> PRT
   <213> Artificial sequence
<220>
   <223> sequence is synthesized
<400> 28
   Tyr Cys Ser Arg Trp Cys Gly Asp Gly Phe Tyr
   1               5                   10
<210> 29
   <211> 11
   <212> PRT
   <213> Artificial sequence
<220>
   <223> sequence is synthesized
<400> 29
   Gly Phe Tyr Ala Met Cys Tyr Trp Gly Gln Gly
   1               5                   10
<210> 30
   <211> 11
   <212> PRT
   <213> Artificial sequence
<220>
   <223> sequence is synthesized
<400> 30
   Asp Tyr Trp Gly Gln Cys Thr Leu Val Thr Val
   1 5 10
<210> 31
   <211> 11
   <212> PRT
   <213> Artificial sequence
<220>
   <223> sequence is synthesized
<400> 31
   Gln Gly Thr Leu Val Cys Val Ser Ser Ala Ser
   1 5 10
<210> 32
   <211> 11
   <212> PRT
   <213> Artificial sequence
<220>
   <223> sequence is synthesized
<400> 32
   Val Thr Val Ser Ser Cys Ser Thr Lys Gly Pro
   1 5 10
<210> 33
   <211> 11
   <212> PRT
   <213> Artificial sequence
<220>
   <223> sequence is synthesized
<400> 33
   Ser Ala Ser Thr Lys Cys Pro Ser Val Phe Pro
   1 5 10
<210> 34
   <211> 11
   <212> PRT
   <213> Artificial sequence
<220>
   <223> sequence is synthesized
<400> 34
   Lys Ser Thr Ser Gly Cys Thr Ala Ala Leu Gly
   1 5 10
<210> 35
   <211> 11
   <212> PRT
   <213> Artificial sequence
<220>
   <223> sequence is synthesized
<400> 35
   Val Lys Asp Tyr Phe Cys Glu Pro Val Thr Val
    1               5                  10
<210> 36
   <211> 11
   <212> PRT
   <213> Artificial sequence
<220>
   <223> sequence is synthesized
<400> 36
   Val Thr Val Ser Trp Cys Ser Gly Ala Leu Thr
    1               5                  10
<210> 37
   <211> 11
   <212> PRT
   <213> Artificial sequence
<220>
   <223> sequence is synthesized
<400> 37
   Thr Val Ser Trp Asn Cys Gly Ala Leu Thr Ser
    1               5                  10
<210> 38
   <211> 11
   <212> PRT
   <213> Artificial sequence
<220>
   <223> sequence is synthesized
<400> 38
   Val Ser Trp Asn Ser Cys Ala Leu Thr Ser Gly
    1               5                  10
<210> 39
   <211> 11
   <212> PRT
   <213> Artificial sequence
<220>
   <223> sequence is synthesized
<400> 39
   Ser Gly Ala Leu Thr Cys Gly Val His Thr Phe
   1 5 10
<210> 40
   <211> 11
   <212> PRT
   <213> Artificial sequence
<220>
   <223> sequence is synthesized
<400> 40
   Ser Gly Val His Thr Cys Pro Ala Val Leu Gln
   1 5 10
<210> 41
   <211> 11
   <212> PRT
   <213> Artificial sequence
<220>
   <223> sequence is synthesized
<400> 41
   Leu Ser Ser Val Val Cys Val Pro Ser Ser Ser
   1 5 10
<210> 42
   <211> 11
   <212> PRT
   <213> Artificial sequence
<220>
   <223> sequence is synthesized
<400> 42
   Val Thr Val Pro Ser Cys Ser Leu Gly Thr Gln
   1 5 10
<210> 43
   <211> 11
   <212> PRT
   <213> Artificial sequence
<220>
   <223> sequence is synthesized
<400> 43
   Ser Leu Gly Thr Gln Cys Tyr Ile Cys Asn Val
   1 5 10
<210> 44
   <211> 11
   <212> PRT
   <213> Artificial sequence
<220>
   <223> sequence is synthesized
<400> 44
   Thr Tyr Ile Cys Asn Cys Asn His Lys Pro Ser
    1               5                   10
<210> 45
   <211> 11
   <212> PRT
   <213> Artificial sequence
<220>
   <223> sequence is synthesized
<400> 45
   Asn His Lys Pro Ser Cys Thr Lys Val Asp Lys
    1               5                   10
<210> 46
   <211> 11
   <212> PRT
   <213> Artificial sequence
<220>
   <223> sequence is synthesized
<400> 46
   His Lys Pro Ser Asn Cys Lys Val Asp Lys Lys
    1               5                   10
<210> 47
   <211> 11
   <212> PRT
   <213> Artificial sequence
<220>
   <223> sequence is synthesized
<400> 47
   Pro Ser Asn Thr Lys Cys Asp Lys Lys Val Glu
    1               5                   10
<210> 48
   <211> 11
   <212> PRT
   <213> Artificial sequence
<220>
   <223> sequence is synthesized
<400> 48
   Thr Lys Val Asp Lys Cys Val Glu Pro Lys Ser
    1               5                   10
<210> 49
   <211> 7
   <212> PRT
   <213> Artificial sequence
<220>
   <223> sequence is synthesized
<400> 49
   Lys Ser Cys Asp Lys Cys His
    1               5
<210> 50
   <211> 11
   <212> PRT
   <213> Artificial sequence
<220>
   <223> sequence is synthesized
<400> 50
   Met Thr Gln Ser Pro Cys Ser Leu Ser Ala Ser
    1               5                   10
<210> 51
   <211> 11
   <212> PRT
   <213> Artificial sequence
<220>
   <223> sequence is synthesized
<400> 51
   Gly Lys Ala Pro Lys Cys Leu Ile Tyr Ser Ala
    1               5                   10
<210> 52
   <211> 11
   <212> PRT
   <213> Artificial sequence
<220>
   <223> sequence is synthesized
<400> 52
   Pro Lys Leu Leu Ile Cys Ser Ala Ser Phe Leu
    1               5                   10
<210> 53
   <211> 11
   <212> PRT
   <213> Artificial sequence
<220>
   <223> sequence is synthesized
<400> 53
   Ile Tyr Ser Ala Ser Cys Leu Tyr Ser Gly Val
   1 5 10
<210> 54
   <211> 11
   <212> PRT
   <213> Artificial sequence
<220>
   <223> sequence is synthesized
<400> 54
   Ser Gly Thr Asp Phe Cys Leu Thr Ile Ser Ser
   1 5 10
<210> 55
   <211> 11
   <212> PRT
   <213> Artificial sequence
<220>
   <223> sequence is synthesized
<400> 55
   Gly Thr Asp Phe Thr Cys Thr Ile Ser Ser Leu
   1 5 10
<210> 56
   <211> 11
   <212> PRT
   <213> Artificial sequence
<220>
   <223> sequence is synthesized
<400> 56
   Thr Asp Phe Thr Leu Cys Ile Ser Ser Leu Gln
   1 5 10
<210> 57
   <211> 11
   <212> PRT
   <213> Artificial sequence
<220>
   <223> sequence is synthesized
<400> 57
   Asp Phe Thr Leu Thr Cys Ser Ser Leu Gln Pro
   1               5                   10
<210> 58
   <211> 11
   <212> PRT
   <213> Artificial sequence
<220>
   <223> sequence is synthesized
<400> 58
   Thr Leu Thr Ile Ser Cys Leu Gln Pro Glu Asp
   1               5                   10
<210> 59
   <211> 11
   <212> PRT
   <213> Artificial sequence
<220>
   <223> sequence is synthesized
<400> 59
   Thr Ile Ser Ser Leu Cys Pro Glu Asp Phe Ala
   1               5                   10
<210> 60
   <211> 11
   <212> PRT
   <213> Artificial sequence
<220>
   <223> sequence is synthesized
<400> 60
   Ile Ser Ser Leu Gln Cys Glu Asp Phe Ala Thr
   1               5                   10
<210> 61
   <211> 11
   <212> PRT
   <213> Artificial sequence
<220>
   <223> sequence is synthesized
<400> 61
   Tyr Cys Gln Gln His Cys Thr Thr Pro Pro Thr
   1               5                   10
<210> 62
   <211> 11
   <212> PRT
   <213> Artificial sequence
<220>
   <223> sequence is synthesized
<400> 62
   Gln His Tyr Thr Thr Cys Pro Thr Phe Gly Gln
   1               5                   10
<210> 63
   <211> 11
   <212> PRT
   <213> Artificial sequence
<220>
   <223> sequence is synthesized
<400> 63
   Thr Pro Pro Thr Phe Cys Gln Gly Thr Lys Val
   1               5                   10
<210> 64
   <211> 11
   <212> PRT
   <213> Artificial sequence
<220>
   <223> sequence is synthesized
<400> 64
   Pro Thr Phe Gly Gln Cys Thr Lys Val Glu Ile
   1               5                   10
<210> 65
   <211> 11
   <212> PRT
   <213> Artificial sequence
<220>
   <223> sequence is synthesized
<400> 65
   Phe Gly Gln Gly Thr Cys Val Glu Ile Lys Arg
   1               5                   10
<210> 66
   <211> 11
   <212> PRT
   <213> Artificial sequence
<220>
   <223> sequence is synthesized
<400> 66
   Gln Gly Thr Lys Val Cys Ile Lys Arg Thr Val
   1 5 10
<210> 67
   <211> 11
   <212> PRT
   <213> Artificial sequence
<220>
   <223> sequence is synthesized
<400> 67
   Glu Ile Lys Arg Thr Cys Ala Ala Pro Ser Val
   1 5 10
<210> 68
   <211> 11
   <212> PRT
   <213> Artificial sequence
<220>
   <223> sequence is synthesized
<400> 68
   Lys Arg Thr Val Ala Cys Pro Ser Val Phe Ile
   1 5 10
<210> 69
   <211> 11
   <212> PRT
   <213> Artificial sequence
<220>
   <223> sequence is synthesized
<400> 69
   Thr Val Ala Ala Pro Cys Val Phe Ile Phe Pro
   1 5 10
<210> 70
   <211> 11
   <212> PRT
   <213> Artificial sequence
<220>
   <223> sequence is synthesized
<400> 70
   Ala Ala Pro Ser Val Cys Ile Phe Pro Pro Ser
   1 5 10
<210> 71
   <211> 11
   <212> PRT
   <213> Artificial sequence
<220>
   <223> sequence is synthesized
<400> 71
   Pro Ser Val Phe Ile Cys Pro Pro Ser Asp Glu
    1               5                   10
<210> 72
   <211> 11
   <212> PRT
   <213> Artificial sequence
<220>
   <223> sequence is synthesized
<400> 72
   Phe Ile Phe Pro Pro Cys Asp Glu Gln Leu Lys
    1               5                   10
<210> 73
   <211> 11
   <212> PRT
   <213> Artificial sequence
<220>
   <223> sequence is synthesized
<400> 73
   Pro Ser Asp Glu Gln Cys Lys Ser Gly Thr Ala
    1               5                   10
<210> 74
   <211> 11
   <212> PRT
   <213> Artificial sequence
<220>
   <223> sequence is synthesized
<400> 74
   Asp Glu Gln Leu Lys Cys Gly Thr Ala Ser Val
    1               5                   10
<210> 75
   <211> 11
   <212> PRT
   <213> Artificial sequence
<220>
   <223> sequence is synthesized
<400> 75
   Gln Leu Lys Ser Gly Cys Ala Ser Val Val Cys
   1               5                   10
<210> 76
   <211> 11
   <212> PRT
   <213> Artificial sequence
<220>
   <223> sequence is synthesized
<400> 76
   Leu Lys Ser Gly Thr Cys Ser Val Val Cys Leu
   1               5                   10
<210> 77
   <211> 11
   <212> PRT
   <213> Artificial sequence
<220>
   <223> sequence is synthesized
<400> 77
   Lys Ser Gly Thr Ala Cys Val Val Cys Leu Leu
   1               5                   10
<210> 78
   <211> 11
   <212> PRT
   <213> Artificial sequence
<220>
   <223> sequence is synthesized
<400> 78
   Val Val Cys Leu Leu Cys Asn Phe Tyr Pro Arg
   1               5                   10
<210> 79
   <211> 11
   <212> PRT
   <213> Artificial sequence
<220>
   <223> sequence is synthesized
<400> 79
   Val Cys Leu Leu Asn Cys Phe Tyr Pro Arg Glu
   1               5                   10
<210> 80
   <211> 11
   <212> PRT
   <213> Artificial sequence
<220>
   <223> sequence is synthesized
<400> 80
   Leu Leu Asn Asn Phe Cys Pro Arg Glu Ala Lys
   1               5                   10
<210> 81
   <211> 11
   <212> PRT
   <213> Artificial sequence
<220>
   <223> sequence is synthesized
<400> 81
   Asn Asn Phe Tyr Pro Cys Glu Ala Lys Val Gln
   1               5                   10
<210> 82
   <211> 11
   <212> PRT
   <213> Artificial sequence
<220>
   <223> sequence is synthesized
<400> 82
   Phe Tyr Pro Arg Glu Cys Lys Val Gln Trp Lys
   1               5                   10
<210> 83
   <211> 11
   <212> PRT
   <213> Artificial sequence
<220>
   <223> sequence is synthesized
<400> 83
   Arg Glu Ala Lys Val Cys Trp Lys Val Asp Asn
   1               5                   10
<210> 84
   <211> 11
   <212> PRT
   <213> Artificial sequence
<220>
   <223> sequence is synthesized
<400> 84
   Ala Lys Val Gln Trp Cys Val Asp Asn Ala Leu
   1 5 10
<210> 85
   <211> 11
   <212> PRT
   <213> Artificial sequence
<220>
   <223> sequence is synthesized
<400> 85
   Val Gln Trp Lys Val Cys Asn Ala Leu Gln Ser
   1 5 10
<210> 86
   <211> 11
   <212> PRT
   <213> Artificial sequence
<220>
   <223> sequence is synthesized
<400> 86
   Val Asp Asn Ala Leu Cys Ser Gly Asn Ser Gln
   1 5 10
<210> 87
   <211> 11
   <212> PRT
   <213> Artificial sequence
<220>
   <223> sequence is synthesized
<400> 87
   Gln Ser Gly Asn Ser Cys Glu Ser Val Thr Glu
   1 5 10
<210> 88
   <211> 11
   <212> PRT
   <213> Artificial sequence
<220>
   <223> sequence is synthesized
<400> 88
   Leu Thr Leu Ser Lys Cys Asp Tyr Glu Lys His
   1 5 10
<210> 89
   <211> 11
   <212> PRT
   <213> Artificial sequence
<220>
   <223> sequence is synthesized
<400> 89
   Thr Leu Ser Lys Ala Cys Tyr Glu Lys His Lys
   1               5                   10
<210> 90
   <211> 11
   <212> PRT
   <213> Artificial sequence
<220>
   <223> sequence is synthesized
<400> 90
   Lys Ala Asp Tyr Glu Cys His Lys Val Tyr Ala
   1               5                   10
<210> 91
   <211> 11
   <212> PRT
   <213> Artificial sequence
<220>
   <223> sequence is synthesized
<400> 91
   Tyr Ala Cys Glu Val Cys His Gln Gly Leu Ser
   1               5                   10
<210> 92
   <211> 11
   <212> PRT
   <213> Artificial sequence
<220>
   <223> sequence is synthesized
<400> 92
   Glu Val Thr His Gln Cys Leu Ser Ser Pro Val
   1               5                   10
<210> 93
   <211> 11
   <212> PRT
   <213> Artificial sequence
<220>
   <223> sequence is synthesized
<400> 93
   Val Thr His Gln Gly Cys Ser Ser Pro Val Thr
   1 5 10
<210> 94
   <211> 11
   <212> PRT
   <213> Artificial sequence
<220>
   <223> sequence is synthesized
<400> 94
   His Gln Gly Leu Ser Cys Pro Val Thr Lys Ser
   1 5 10
<210> 95
   <211> 11
   <212> PRT
   <213> Artificial sequence
<220>
   <223> sequence is synthesized
<400> 95
   Gln Gly Leu Ser Ser Cys Val Thr Lys Ser Phe
   1 5 10
<210> 96
   <211> 11
   <212> PRT
   <213> Artificial sequence
<220>
   <223> sequence is synthesized
<400> 96
   Gly Leu Ser Ser Pro Cys Thr Lys Ser Phe Asn
   1 5 10
<210> 97
   <211> 11
   <212> PRT
   <213> Artificial sequence
<220>
   <223> sequence is synthesized
<400> 97
   Leu Ser Ser Pro Val Cys Lys Ser Phe Asn Arg
   1 5 10
<210> 98
   <211> 11
   <212> PRT
   <213> Artificial sequence
<220>
   <223> sequence is synthesized
<400> 98
   Ser Ser Pro Val Thr Cys Ser Phe Asn Arg Gly
    1               5                   10